# EUROPEAN PATENT SPECIFICATION

(11) **EP 1 735 276 B1**
(45) Date of publication and mention of the grant of the patent: **16.02.2011**
(21) Application number: 05715124.3
(22) Date of filing: 23.03.2005
(51) Int. Cl.: C07C 401/00

(54) **NOVEL METHOD FOR THE PREPARATION OF INTERMEDIATES USEFUL FOR THE SYNTHESIS OF VITAMIN D ANALOGUES**
NEUES VERFAHREN ZUR HERSTELLUNG VON FÜR DIE SYNTHESE VON VITAMIN-D-ANALOGA GEEIGNETEN ZWISCHENPRODUKTEN
NOUVEAU PROCEDE DE PREPARATION D'INTERMEDIAIRES UTILISES DANS LA SYNTHESE D'ANALOGUES DE VITAMINE D

(30) Priority: 02.04.2004 US 558546 P
(43) Date of publication of application: 27.12.2006
(73) Proprietor: LEO PHARMA A/S, 2750 Ballerup (DK)
(72) Inventor: HANSEN, Erik, Torngaard, DK-3390 Hundested (DK); SABROE, Thomas, Peter, 2970 Horsholm (DK); CALVERLEY, Martin, John, DK-2730 Herlev (DK); PEDERSEN, Henrik, DK-4340 Tølløse (DK); DEUSSEN, Heinz-Josef, Wilhelm, DK-2860 Søborg (DK)
(86) International application number: PCT/DK2005/000203
(87) International publication number: WO 2005/095336

(56) References cited:
- WO-A-87/00834
- WO-A-2004/037781
- WO-A-2005/051903
- US-A- 5 247 104
- CALVERLEY M J: "SYNTHESIS OF MC 903, A BIOLOGICALLY ACTIVE VITAMIN D METABOLITE ANALOGUE" TETRAHEDRON, ELSEVIER SCIENCE PUBLISHERS, AMSTERDAM, NL, vol. 43, no. 20, 1987, pages 4609-4619, XP001147723 ISSN: 0040-4020
- STEINMEYER ANDREAS ET AL: "Synthesis and biological activities of a new series of secosteroids: Vitamin D phosphonate hybrids" STEROIDS, vol. 66, no. 3-5, March 2001 (2001-03), pages 257-266, XP002328551 ISSN: 0039-128X
- DAUBEN W G ET AL: "THE SYNTHESIS OF 25 OXO-25-PHOSPHAVITAMIN D-3" TETRAHEDRON LETTERS, vol. 30, no. 6, 1989, pages 677-680, XP002328552 ISSN: 0040-4039
- M. A. BLANCHETTE ET AL: "HORNER-WADSWORTH-EMMONS REACTIONS: USE OF LITHIUM CHLORIDE AND AN AMINE FOR BASE SENSITIVE COMPOUNDS" TETRAHEDRON LETTERS., vol. 25, no. 21, 1984, pages 2183-2186, XP002328553 NLELSEVIER SCIENCE PUBLISHERS, AMSTERDAM.
- RESUL B ET AL: "PHENYL-SUBSTITUTED PROSTAGLANDINS: POTENT AND SELECTIVE ANTIGLAUCOMA AGENTS" JOURNAL OF MEDICINAL CHEMISTRY, AMERICAN CHEMICAL SOCIETY. WASHINGTON, US, vol. 36, no. 2, 1993, pages 243-248, XP000673914 ISSN: 0022-2623
- SOERENSEN, HANNE ET AL: "In vitro metabolism of Calcipotriol (MC 930), a vitamin D analog" BIOCHEMICAL PHARMACOLOGY , 39(2), 391-3 CODEN: BCPCA6; ISSN: 0006-2952, 1990, XP002343619
- DATABASE CA [Online] CHEMICAL ABSTRACTS SERVICE, COLUMBUS, OHIO, US; OKAMOTO, MASATO ET AL: "Preparation of 1.alpha.,24-dihydroxy-22(E)-dehydrovitamin D3 and its derivatives" XP002343720 retrieved from STN Database accession no. 1997:139652 -& JP 08 325226 A (TEIJIN LTD, JAPAN) 10 December 1996 (1996-12-10)

## Description

### FIELD OF THE INVENTION

The present invention relates to novel intermediates which are useful in the synthesis of calcipotriol {(5Z, 7E, 22E, 24S)-24-cyclopropyl-9,10-secochola-5,7,10(19),22-tetraene-1α-3β-24-triol} and methods for the preparation thereof. The present invention relates further to the use of intermediates produced with said methods for making calcipotriol or calcipotriol monohydrate.

### BACKGROUND OF THE INVENTION

Calcipotriol or calcipotriene (structure I) [CAS 112965-21-6] shows a strong activity in inhibiting undesirable proliferation of epidermal keratinocytes [F.A.C.M. Castelijins, M.J. Gerritsen, I.M.J.J. van Vlijmen-Willems, P.J. van Erp, P.C.M. van de Kerkhof; Acta Derm. Venereol. 79, 11, 1999]. The efficacy of calcipotriol (Ia) and calcipotriol monohydrate (Ib) in the treatment of psoriasis was shown in a number of clinical trials [D.M. Ashcroft et al.; Brit. Med. J. 320, 963-67, 2000] and calcipotriol is currently used in several commercial drug formulations.

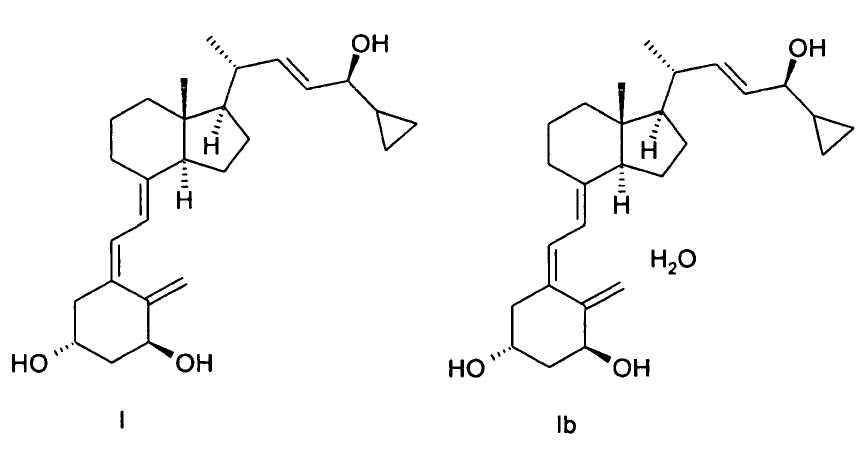

A key step in the synthesis of calcipotriol or intermediates useful for the synthesis of calcipotriol is the attachment of the cyclopropyl-enone side chain to the CD-ring of suitable precursors, which has been described with a Wittig reagent IV.

For example, in an industrial synthesis of calcipotriol, the cyclopropyl containing phosphorane side chain IV is reacted with the aldehyde IIIa in a Wittig reaction to give the enone Va, wherein R₁ and R₂ are tert-butyldimethylsilyl (see e.g. WO 87/00834 or M.J. Calverley; Tetrahedron, 43 (20), 4609-19, 1987). Calcipotriol is then obtained from the key intermediate Va by reduction to the C-24 alcohol followed by photoisomerisation and the removal of the silyl protecting groups.

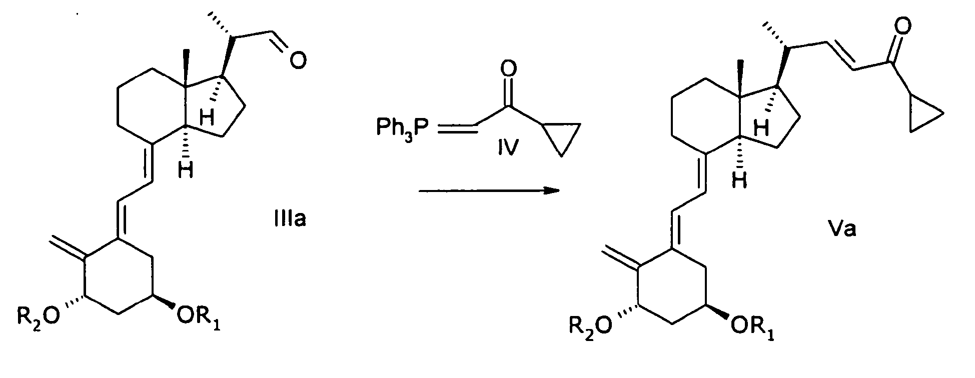

The Wittig processes using the phosphorane IV have a number of disadvantages, especially on a large scale: a) During the C=C-bond forming reaction triphenylphosphine oxide is formed as a side product which is difficult to remove from the reaction mixture. The formation of triphenylphosphine oxide currently adds an additional chromatographic step to the process outlined above. b) The Wittig reaction furthermore necessitates reaction temperatures above 95°C due to the low reactivity of the phosphorane IV. Lower reaction temperatures would be advantageous in an industrial process.

It is an object of this invention to provide an alternative process which may overcome one or more of the various problems and disadvantages described above. The present invention thus provides a novel process which can be run at lower temperature and which avoids the tedious chromatographic removal of triphenylphosphine oxide to produce intermediates useful for the synthesis of calcipotriol, such as the enone of general structure Va.

### SUMMARY OF THE INVENTION

It was surprisingly found that a compound of general structure IIa, 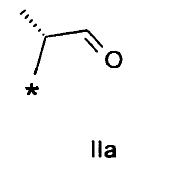 wherein the carbon marked with an asterisk is either connected by a single bond to a carbon atom of a vitamin D analogue fragment at C-17, or to a fragment of a precursor for the synthesis of a vitamin D analogue at a C-17 analogous position forming compounds of formulae IIIa, IIIb, VIa, VIb, XIIIa, XIIIb, XVa, XVb or IXX (see structures below), can be reacted with a phosphonate of general structure VII, 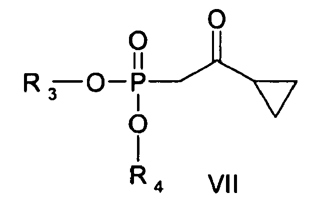 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy, in the presence of a base,
to give a compound of general structure of general structure II, 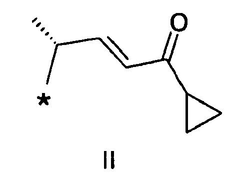 wherein the carbon marked with an asterisk is either connected by a single bond to a carbon atom of a vitamin D analogue fragment at C-17, or to a fragment of a precursor for the synthesis of a vitamin D analogue at a C-17 analogous position forming compounds of formulae Va, Vb, VIIIa, VIIIb, XIVa, XIVb, XVIa, XVIb or XX (see structures below).

Accordingly, a compound of general structure IIIa, IIIb, VIa, VIb, XIIIa, XIIIb, XVa, or XVb, or IXX, 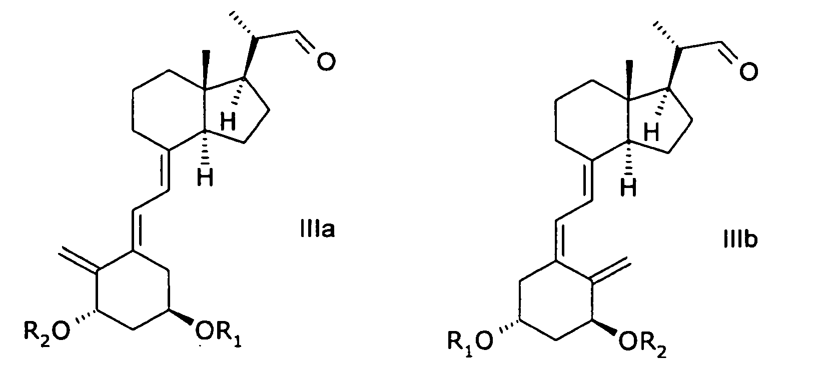 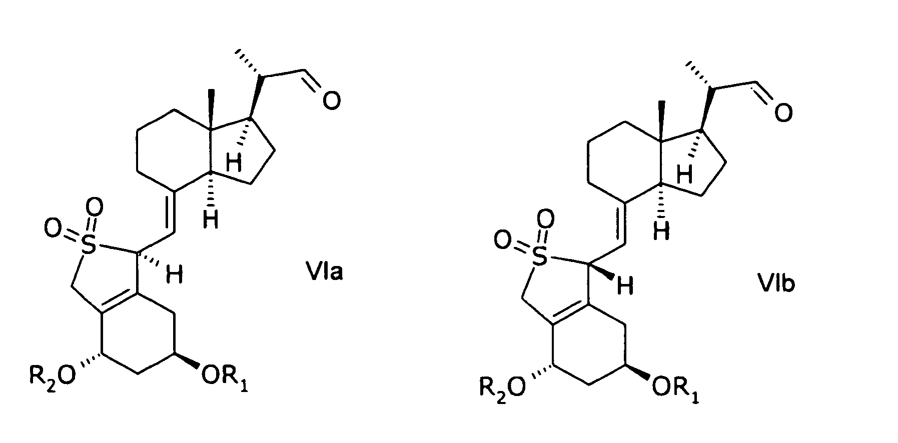 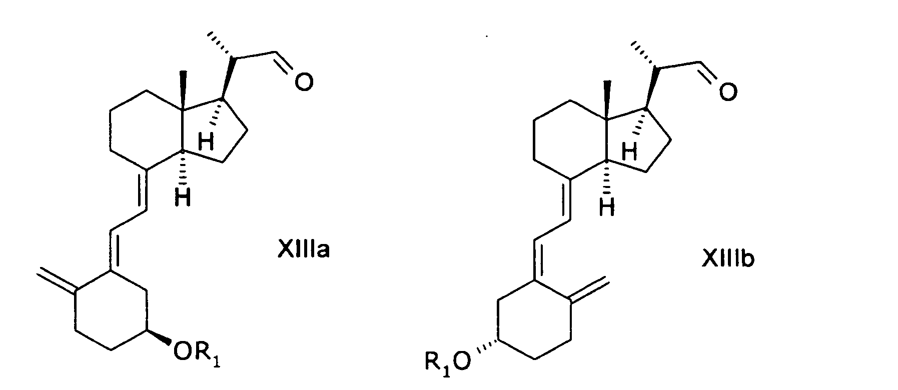 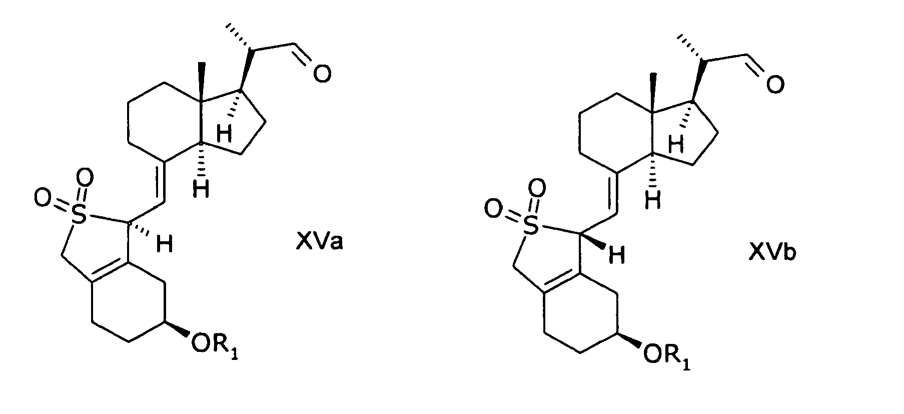 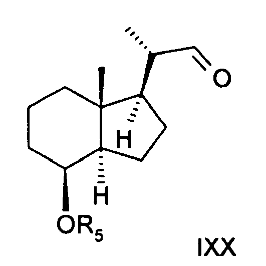 wherein R₁ and R₂ are the same or different and represent hydrogen or a hydroxy protecting group,
and wherein R₅ represents hydrogen or a hydroxy protecting group,
can be reacted with a phosphonate of general structure VII, 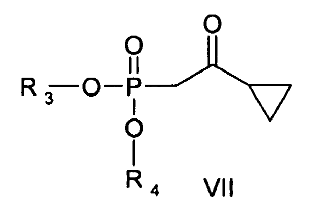 wherein R₃ and R₄ are the same or different and represent alkyl, haloalkyl, hydroxyalkyl, alkenyl, alkynyl, aralkyl, aralkenyl, aralkynyl, or aryl, each being each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy, in the presence of a base, to give a compound of general structure Va, Vb, VIIIa, VIIIb, XIVa, XIVb, XVIa, XVIb, or XX respectively, 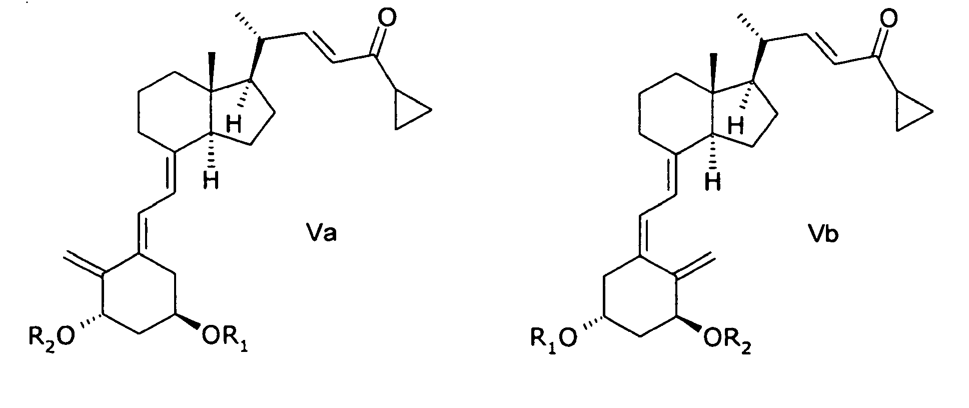 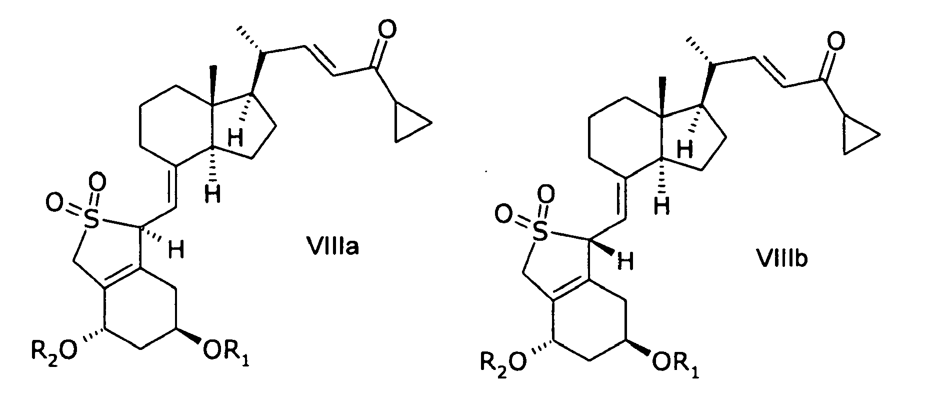 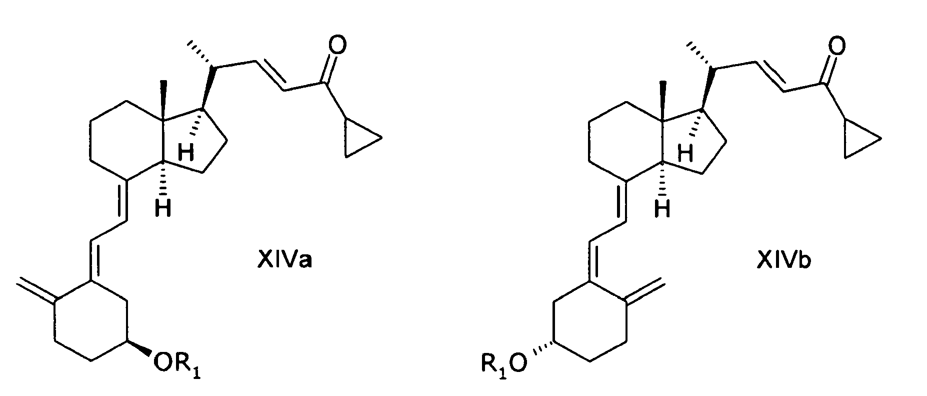 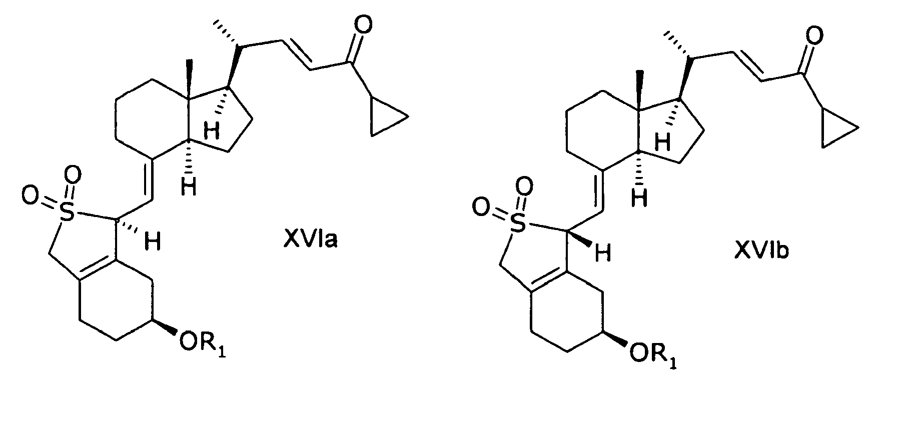 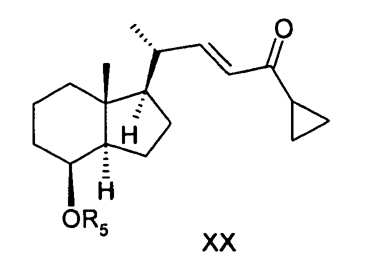 wherein R₁, R₂, and R₅ are as defined above.

This process, also called Wadsworth-Emmons, Wittig-Horner, or Horner-Emmons-Wadsworth reaction, has several advantages over the use of the phosphorane reagent IV: a) The reagent of general structure VII is more reactive than the corresponding phosphorane allowing the usage of mild reaction conditions such as low temperature, typically below 35°C. b) The phosphorus product of the reaction is a phosphate ester, and hence soluble in water, unlike triphenylphosphine oxide, which makes it easy to separate it from the enones Va, Vb, VIIIa, VIIIb, XIVa, XIVb, XVIa, XVIb, or XX. c) The Wittig-Horner reaction is more trans-selective resulting in a better yield and in improved purity of the desired products Va, Vb, VIIIa, VIIIb, XIVa, XIVb, XVIa, XVIb, or XX.

In a first aspect, this invention relates to a method of reacting a compound of general structure IIIa, IIIb, VIa, VIb, XIIIa, XIIIb, XVa, XVb, or IXX as above with a phosphonate of general structure VII to give a compound of general structure Va, Vb, VIIIa, VIIIb, XIVa, XIVb, XVIa, XVIb, or XX as above.

In yet another aspect, this invention relates to a compound of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy, provided that that the compound is not (2-cyclopropyl-2-oxoethyl)-phosphonic acid diethyl ester.

In yet another aspect, this invention relates to the use of a compound of general structure VII as defined above or to the use of (2-cyclopropyl-2-oxoethyl)phosphonic acid diethyl ester in the manufacture of calcipotriol or calcipotriol monohydrate in the processes described herein.

In a still further aspect, this invention relates to the use of a compound, such as a compound of general formula Vb, XIVa, XIVb, VII, IIIa, IIIb, VIa, VIb, XIIIa, XIIIb, XVa, XVb, XX, XXIa, XXII, XXIIIB, or Va as defined above, as an intermediate in the manufacture of calcipotriol or calcipotriol monohydrate according to processes described herein.

In a further aspect, this invention relates to a method for producing calcipotriol or calcipotriol monohydrate, the method comprising the steps of:
(i) reacting a compound of general structure IIIa,
   wherein R₁ and R₂ are the same or different and represent hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure Va, wherein R₁ and R₂ are as defined above;
(ii) reducing the compound of general structure Va with a suitable reducing agent to give a compound of general structure IXa or a mixture of compounds of general structure IXa and IXb, 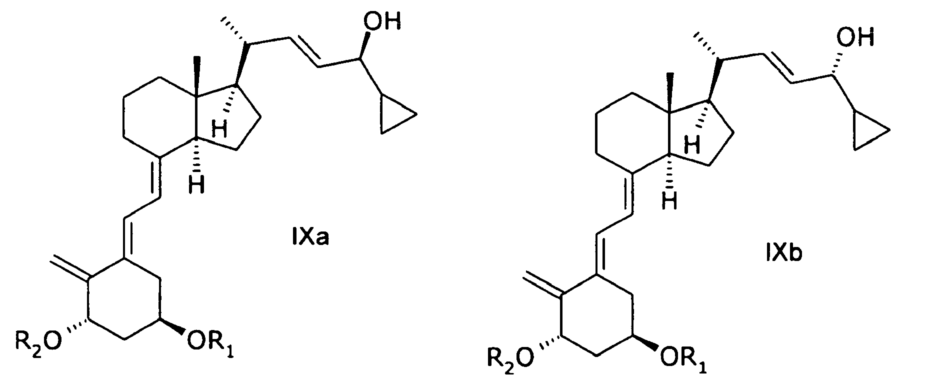 wherein R₁ and R₂ are as defined above;
(iii) optionally separating the compound of general structure IXa from the mixture of compounds of general structure IXa and IXb;
(iv) photoisomerising the compound of general structure IXa to the compound of general structure Xa, 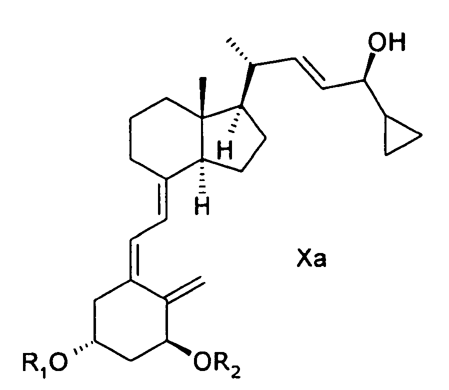 wherein R₁ and R₂ are as defined above;
(v) when R₁ and/or R₂ are not hydrogen, removing the hydroxy protecting group(s) R₁ and/or R₂ of the compound of general structure Xa to generate calcipotriol; and
(vi) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure IIIb,
   wherein R₁ and R₂ are the same or different and represent hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure Vb, wherein R₁ and R₂ are as defined above;
(ii) reducing the compound of general structure Vb with a suitable reducing agent to give a compound of general structure Xa or a mixture of compounds of general structure Xa and Xb, 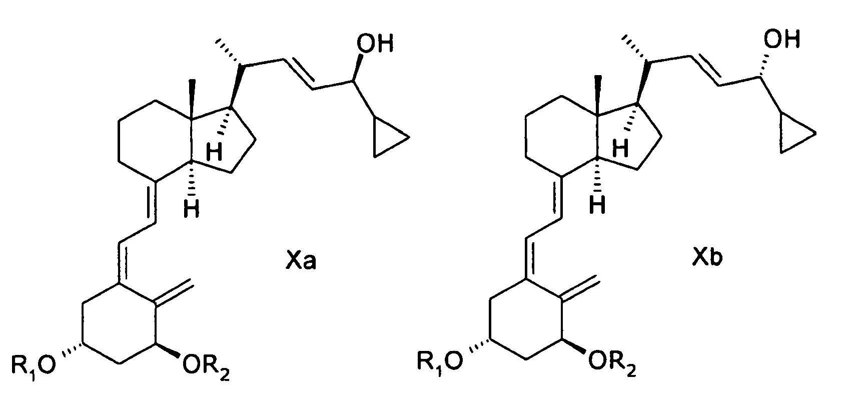 wherein R₁ and R₂ are as defined above;
(iii) optionally separating the compound of general structure Xa from the mixture of compounds of general structure Xa and Xb;
(iv) when R₁ and/or R₂ are not hydrogen, removing the hydroxy protecting group(s) R₁ and/or R₂ of the compound of general structure Xa to generate calcipotriol; and
(v) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure VIa and/or VIb,
   wherein R₁ and R₂ are the same or different and represent hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure VIIIa and/or VIIIb, wherein R₁ and R₂ are as defined above;
(ii) heating the compounds of general structure VIIIa and/or VIIIb above 60°C in the presence of a base to give a compound of general structure Va,
   wherein R₁ and R₂ are as defined above;
(iii) reducing the compound of general structure Va with a suitable reducing agent to give a compound of general structure IXa or a mixture of compounds of general structure IXa and IXb,
   wherein R₁ and R₂ are as defined above;
(iv) optionally separating the compound of general structure IXa from the mixture of compounds of general structure IXa and IXb;
(v) photoisomerising the compound of general structure IXa to the compound of general structure Xa,
   wherein R₁ and R₂ are as defined above;
(vi) when R₁ and/or R₂ are not hydrogen, removing the hydroxy protecting group(s) R₁ and/or R₂ of the compound of general structure Xa to generate calcipotriol; and
(vii) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure VIa and/or VIb,
   wherein R₁ and R₂ are the same or different and represent hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure VIIIa and/or VIIIb, wherein R₁ and R₂ are as defined above;
(ii) reducing the compounds of general structure VIIIa and/or VIIIb, with a suitable reducing agent in an inert solvent, to give compounds of general structure XIaa and/or XIba, or a mixture of compounds of general structure XIaa and/or XIba and XIab and/or XIbb, 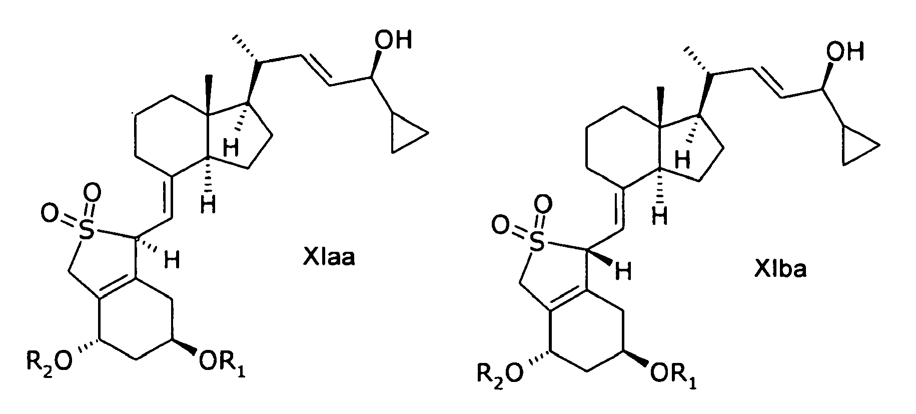 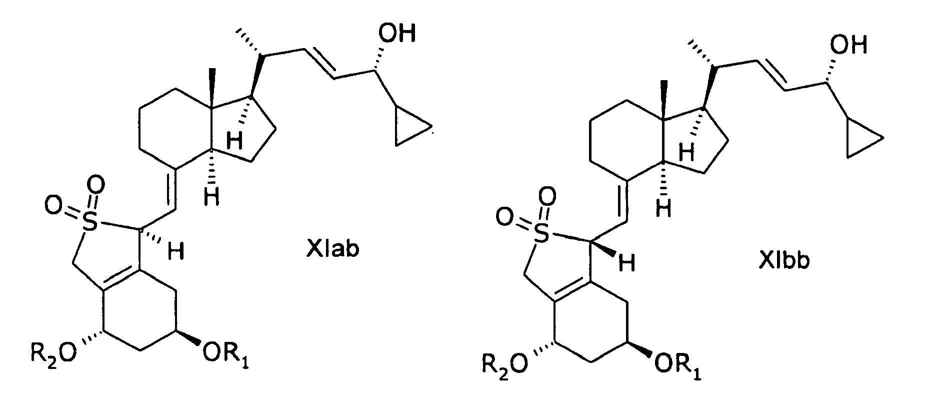 wherein R₁ and R₂ are as defined above;
(iii) optionally separating the compounds of general structure XIaa and/or XIba from the reaction mixture;
(iv) heating the compounds of general structure XIaa and/or XIba above 60°C in the presence of a base to give a compound of general structure IXa,
   wherein R₁ and R₂ are as defined above;
(v) optionally separating the compound of general structure IXa from the reaction mixture;
(vi) photoisomerising the compound of general structure IXa to the compound of general structure Xa,
   wherein R₁ and R₂ are as defined above;
(vii) when R₁ and/or R₂ are not hydrogen, removing the hydroxy protecting group(s) R₁ and/or R₂ of the compound of general structure Xa to generate calcipotriol; and
(viii) optionally crystallising the calcipotriol from a mixture of an organic solvent and water to give calcipotriol monohydrate;
   wherein steps (vi) and (vii) may be in reversed order.

In a still further aspect, this invention relates to a method for producing calcipotriol or calcipotriol monohydrate, the method comprising the steps of:
(i) reacting a compound of general structure XIIIa,
   wherein R₁ represents hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure XIVa, wherein R₁ is as defined above;
(ii) hydroxylating the compound of general structure XIVa with suitable hydroxylating agent to give a compound of general structure Va,
   wherein R₁ represents hydrogen or a hydroxy protecting group and R₂ is hydrogen;
(iii) optionally reacting the compound of general structure Va, wherein R₁ represents hydrogen or a hydroxy protecting group and R₂ is hydrogen with a suitable protecting agent to give a compound of general structure Va, wherein R₁ and R₂ are the same or different and represent a hydroxy protecting group;
(iv) reducing the compound of general structure Va with a suitable reducing agent to give a compound of general structure IXa or a mixture of compounds of general structure IXa and IXb,
   wherein R₁ and R₂ are as defined above;
(v) optionally separating the compound of general structure IXa from the mixture of compounds of general structure IXa and IXb;
(vi) photoisomerising the compound of general structure IXa to a compound of general structure Xa,
   wherein R₁ and R₂ are as defined above;
(vii) when R₁ and/or R₂ are not hydrogen, removing the hydroxy protecting group(s) R₁ and/or R₂ of the compound of general structure Xa to generate calcipotriol; and
(viii) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure XIIIb,
   wherein R₁ represents hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure XIVb, wherein R₁ is as defined above;
(ii) photoisomerising the compound of general structure XIVb to a compound of general structure XIVa,
   wherein R₁ is as defined above;
(iii) hydroxylating the compound of general structure XIVa with suitable hydroxylating agent to give a compound of general structure Va,
   wherein R₁ represents hydrogen or a hydroxy protecting group and R₂ is hydrogen;
(iv) optionally reacting the compound of general structure Va, wherein R₁ represents hydrogen or a hydroxy protecting group and R₂ is hydrogen with a suitable protecting agent to give a compound of general structure Va, wherein R₁ and R₂ are the same or different and represent a hydroxy protecting group;
(v) reducing the compound of general structure Va with a suitable reducing agent to give a compound of general structure IXa or a mixture of compounds of general structure IXa and IXb,
   wherein R₁ and R₂ are as defined above;
(vi) optionally separating the compound of general structure IXa from the mixture of compounds of general structure IXa and IXb;
(vii) photoisomerising the compound of general structure IXa to the compound of general structure Xa,
   wherein R₁ and R₂ are as defined above;
(viii) when R₁ and/or R₂ are not hydrogen, removing the hydroxy protecting group(s) R₁ and/or R₂ of the compound of general structure Xa to generate calcipotriol; and
(ix) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure XVa and/or XVb,
   wherein R₁ represents a hydrogen or a hydroxy protecting group,
   with a phosphonate of general structure VII, wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
   in the presence of a base, to give a compound of general structure XVIa and/or XVIb, wherein R₁ is as defined above;
(ii) heating the compounds of general structure XVIa and/or XVIb above 60°C in the presence of a base to give a compound of general structure XIVa,
   wherein R₁ is as defined above;
(iii) hydroxylating the compound of general structure XIVa with suitable hydroxylating agent to give a compound of general structure Va,
   wherein R₁ represents hydrogen or a hydroxy protecting group and R₂ is hydrogen;
(iv) optionally reacting the compound of general structure Va, wherein R₁ represents hydrogen or a hydroxy protecting group and R₂ is hydrogen with a suitable protecting agent to give a compound of general structure Va, wherein R₁ and R₂ are the same or different and represent a hydroxy protecting group;
(v) reducing the compound of general structure Va with a suitable reducing agent to give a compound of general structure IXa or a mixture of compounds of general structure IXa and IXb,
   wherein R₁ and R₂ are as defined above;
(vi) optionally separating the compound of general structure IXa from the mixture of compounds of general structure IXa and IXb;
(vii) photoisomerising the compound of general structure IXa to the compound of general structure Xa,
   wherein R₁ and R₂ are as defined above;
(viii) when R₁ and/or R₂ are not hydrogen, removing the hydroxy protecting group(s) R₁ and/or R₂ of the compound of general structure Xa to generate calcipotriol; and
(ix) optionally crystallising the calcipotriol from a mixture of an organic solvent and water to give calcipotriol monohydrate.

In a still further aspect, this invention relates to a method for producing calcipotriol or calcipotriol monohydrate, the method comprising the steps of:
(i) reacting a compound of general structure IXX,
   wherein R₅ represents hydrogen or a hydroxy protecting group,
   with a phosphonate of general structure VII,
   wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy, in the presence of a base,
   to give a compound of general structure XX, wherein R₅ is as defined above;
(ii) reducing the compound of general structure XX with a suitable reducing agent to give a compound of general structure XXIa or a mixture of compounds of general structure XXIa and XXIb, wherein R₅ is as defined above and R₆ is hydrogen; 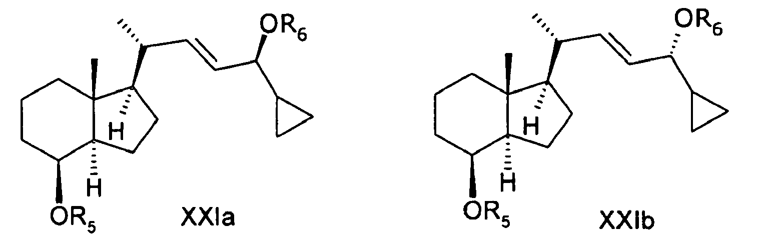
(iii) optionally separating the compound of general structure XXIa from the mixture of compounds of general structure XXIa and XXIb;
(iv) protecting the allylic hydroxy group of the compound of general structure XXIa with a suitable hydroxy protecting reagent to give a compound of general structure XXIa, wherein R₆ is a hydroxy protecting group and R₅ is as defined above;
(v) when R₅ is not hydrogen, removing the hydroxy protecting group R₅ of the compound of general structure XXIa to give a compound of general structure XXIa, wherein R₅ is hydrogen;
(vi) oxidising the hydroxy group of the compound of general structure XXIa with a suitable oxidising agent to give a compound of general structure XXII, wherein R₆ is as defined above;
(vii) coupling of the compound of general structure XXII with a Wittig reagent XXIIIa or a Wittig Horner reagent XXIIIb, wherein R₁ and R₂ represent a hydrogen or a hydroxy protecting group, and wherein R₃ and R₄ are as defined above; 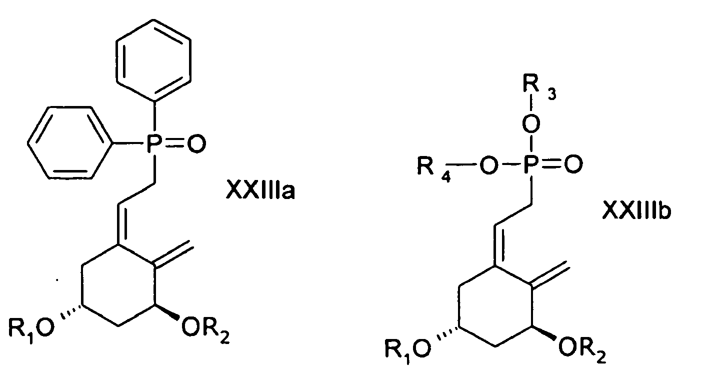 in the presence of a base to give a compound of general structure XXIVa, 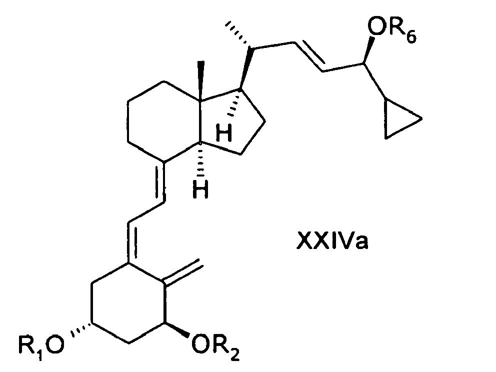 wherein R₁ and R₂ are the same or different and represent hydrogen or a hydroxy protecting group, and wherein R₆ is as defined above;
(viii) when R₆ is not hydrogen, removing the hydroxy protecting group R₆ of the compound of general structure XXIVa;
(ix) optionally separating the compound of general structure XXIVa;
(x) when R₁ and R₂ are not hydrogen, removing the hydroxy protecting group(s) R₁ and R₂ of the compound of general structure XXIVa to generate calcipotriol;
   and
(xi) optionally crystallising the calcipotriol from a mixture of an organic solvent and water to give calcipotriol monohydrate.

### DETAILED DESRIPTION OF THE INVENTION

### Definitions

As used herein, "vitamin D-analogue" means any derivative of vitamin D₂ or D₃, such as 1α,25-dihydroxyvitamin D₂ or 1α,25-dihydroxyvitamin D₃, including derivatives wherein one or more of the A, C, or D ring are modified or/and where the side chain attached to C-17 is different from natural vitamin D₂ or D₃. Examples of vitamin D-analogues can for example be found in ["Vitamin D", D. Feldman, Ed., Academic Press, San Diego, USA, 1997] and [G.-D. Zhu et al., Chem. Rev. 1995, 95, 1877-1952] and references cited therein, and include hydroxy protected or unprotected calcipotriol, and isomers and derivatives of calcipotriol.

As used herein, "vitamin D-analogue fragment" means a C-17 radical of a vitamin D-analogue as defined above without the side chain usually attached at C-17. Examples of vitamin D-analogue fragments are represented by structures A, B, C, D, E, F, G, H; wherein the C-17 analogous positions in the sense of the present invention are indicated below; and wherein R₁ and R₂ are the same or different and represent hydrogen or a hydroxy protecting group.

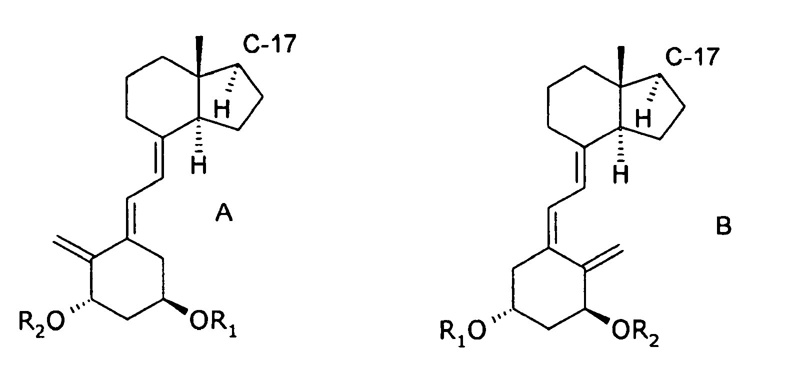

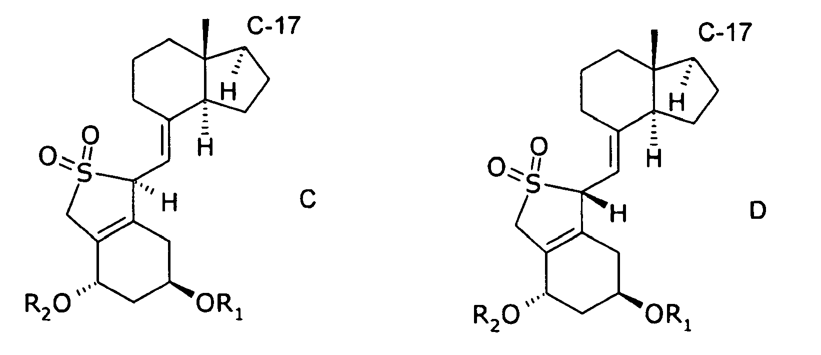

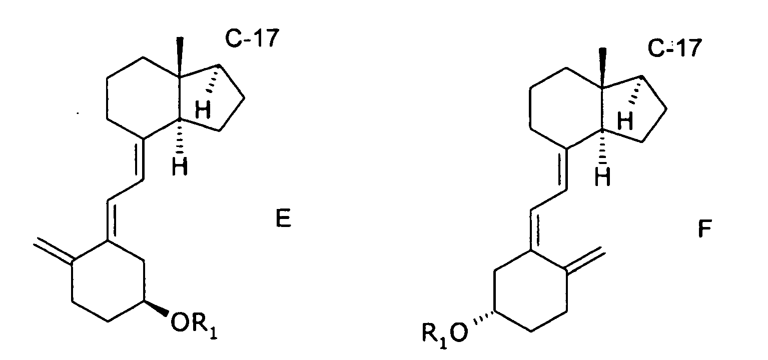

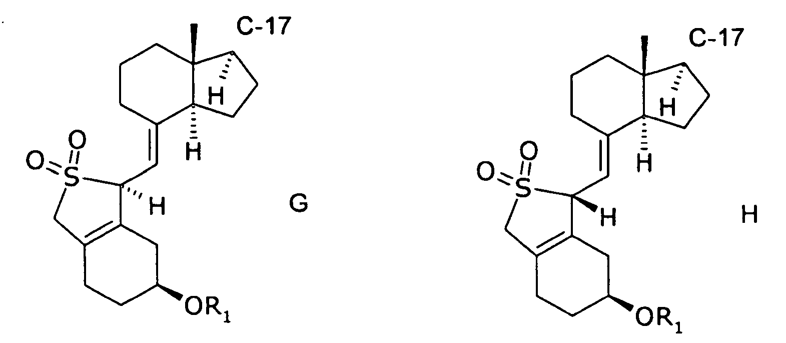

As used herein, "a precursor for the synthesis of a vitamin D-analogue" means any molecule useful in the synthesis of a vitamin D derivative as defined above, such as a starting material or intermediate, wherein part of the precursor molecule becomes incorporated into the final vitamin D-analogue. Examples include, but are not limited to steroid ring systems, such as ergosterol, cholesterol, or 7-dehydrocholesterol, or derivatives of the CD-rings of steroids, such as Grundmann's ketone or derivatives of Grundmann's ketone. Examples of precursors for the synthesis of a vitamin D-analogue can for example be found in [G.-D. Zhu et al., Chem. Rev. 1995, 95, 1877-1952] and references cited therein. Examples of specific derivatives of CD-rings of steroids, which are in particular useful are the ring structures M and N illustrated below, wherein PG is hydrogen or a hydrogen protecting group as defined below.

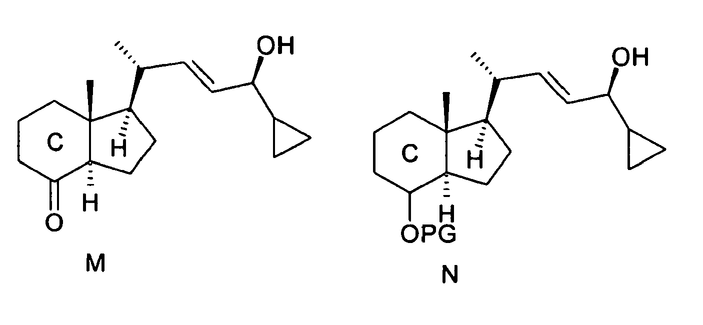

A C-17 analogous position of such a precursor is intended to mean the carbon atom of said precursor, which will correspond to the C-17 carbon atom in the final vitamin D-analogue or calcipotriol.

As used herein, "a fragment of a precursor for the synthesis of a vitamin D-analogue" means a radical of a precursor for the synthesis of a vitamin D-analogue as defined above. For example a fragment of a precursor for the synthesis of a vitamin D-analogue may be a steroid ring system fragment, which may be represented by structure Q or R, wherein the C-17 analogous positions in the sense of the present invention are indicated below.

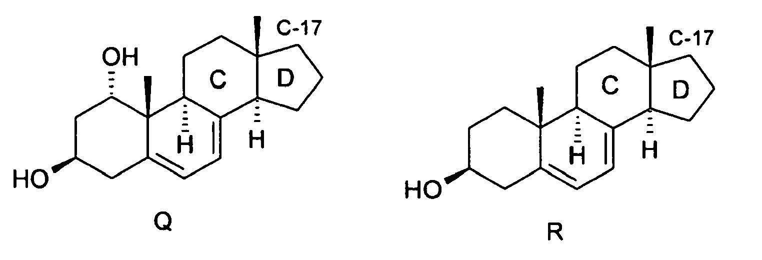

Other examples of fragments of a precursor for the synthesis of a vitamin D-analogue are fragments of derivatives of the CD-rings of steroids, which may for example be represented by structure O or P, wherein the C-17 analogous positions in the sense of the present invention are indicated and wherein PG is as defined above.

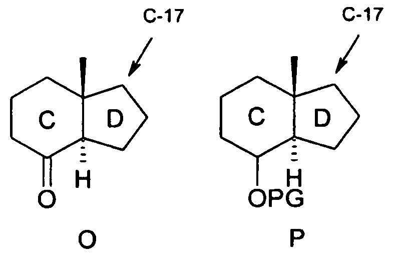

As used herein a "hydroxy protecting group" is any group which forms a derivative that is stable to the projected reactions wherein said hydroxy protecting group can be selectively removed by reagents that do not attack the regenerated hydroxy group. Said derivative can be obtained by selective reaction of a hydroxy protecting agent with a hydroxy group. Silyl derivatives, e.g. trialkylsilyl, such as *tert*-butyldimethylsilyl, trimethylsilyl, triethylsilyl, diphenylmethylsilyl, triisopropylsilyl, *tert*-butyldiphenylsilyl, forming silyl ethers are examples of hydroxy protecting groups. Silyl chlorides such as *tert*-butyldimethylsilyl chloride (TBSCI), trimethylsilylchloride, triethylsilylchloride, diphenylmethylsilylchloride, triisopropylsilylchloride, and *tert*-butyldiphenylsilylchloride are examples of hydroxy protecting agents. Silyl chlorides are for example reacted with the hydroxy group(s) in the presence of a base, such as imidazole. Hydrogen fluoride, such as aqueous HF in acetonitrile, or tetra n-butylammonium fluoride are examples of reagents which can remove silyl groups. Other hydroxy protecting groups include ethers, such as tetrahydropyranyl (THP) ether, benzyl ether, *tert*-butyl ether, including alkoxyalkyl ethers (acetals), such as methoxymethyl (MOM) ether, or esters, such as chloroacetate ester, trimethylacetate, acetate or benzoate ester. Non-limiting examples of hydroxy protecting groups and methods of protection and removal, all included in the scope of this application, can for example be found in "Protective Groups in Organic Synthesis", 3rd ed., T. W. Greene & P. G. M. Wuts eds., John Wiley 1999 and in "Protecting Groups", 1st ed., P.J. Kocienski, G. Thieme 2000, Jarowicki, K., Kocienski, P., J. Chem. Soc., Perkin Trans. 1, 2000, 2495-2527.

As used herein, "alkyl" is intended to mean a linear or branched alkyl group, which may be cyclic or acyclic, having one to twenty carbon atoms, such as 1-12, such as 1-7, such as 1-4 carbon atoms. The term includes the subclasses normal alkyl (*n-*alkyl), secondary and tertiary alkyl, such as methyl, ethyl, *n-*propyl, isopropyl, *n-*butyl, isobutyl, *sec*.-butyl, *tert*.-butyl, pentyl, isopentyl, hexyl, isohexyl, and the *tert*-butyldimethyl group.

The term "halogen" is intended to indicate a substituent from the 7^{th} main group of the periodic table, preferably fluoro, chloro and bromo.

The term "alkenyl" is intended to indicate a mono-, di-, tri-, tetra- or pentaunsaturated hydrocarbon radical comprising 2-10 carbon atoms, in particular 2-6 carbon atoms, such as 2-4 carbon atoms, e.g. ethenyl, propenyl, butenyl, pentenyl or hexenyl.

The term "alkynyl" is intended to indicate an hydrocarbon radical comprising 1-5 triple C-C bonds and 2-20 carbon atoms, the alkane chain typically comprising 2-10 carbon atoms, in particular 2-6 carbon atoms, such as 2-4 carbon atoms, e.g. ethynyl, propynyl, butynyl, pentynyl or hexynyl.

The term "haloalkyl" is intended to indicate an alkyl group as defined above substituted with one or more halogen atoms as defined above.

The term "hydroxyalkyl" is intended to indicate an alkyl group as defined above substituted with one or more hydroxy groups.

The term "alkoxy" is intended to indicate a radical of the formula -OR', wherein R' is alkyl as indicated above, e.g. methoxy, ethoxy, n-propoxy, isopropoxy, butoxy, etc.

The term "alkoxycarbonyl" is intended to indicate a radical of the formula -C(O)-O-R', wherein R' is alkyl as indicated above, e.g. methoxycarbonyl, ethoxycarbonyl, n-propoxycarbonyl, isopropoxycarbonyl, etc.

The term "alkylcarbonyloxy" is intended to indicate a radical of the formula -O-C(O)-R', wherein R' is alkyl as indicated above.

The term "cycloalkyl" is intended to indicate a saturated cycloalkane radical comprising 3-20 carbon atoms, preferably 3-10 carbon atoms, in particular 3-8 carbon atoms, such as 3-6 carbon atoms, e.g. cyclopropyl, cyclobutyl, cyclopentyl or cyclohexyl.

The term "cycloalkenyl" is intended to indicate mono-, di- tri- or tetraunsaturated non-aromatic cyclic hydrocarbon radicals, comprising 3-20 carbon atoms, typically comprising 3-10 carbon atoms, such as 3-6 carbon atoms, e.g. cyclopropenyl, cyclobutenyl, cyclopentenyl or cyclohexenyl.

The term "aryl" is intended to indicate a radical of aromatic carbocyclic rings comprising 6-20 carbon atoms, such as 6-14 carbon atoms, preferably 6-10 carbon atoms, in particular 5- or 6-membered rings, optionally fused carbocyclic rings with at least one aromatic ring, such as phenyl, naphthyl, indenyl and indanyl.

The term "aralkyl" is intended to indicate an alkyl group as defined above substituted with one or more aryl radicals as defined above.

The term "aralkenyl" is intended to indicate an alkenyl group as defined above substituted with one or more aryl radicals as defined above.

The term "aralkynyl" is intended to indicate an alkynyl group as defined above substituted with one or more aryl radicals as defined above.

As used herein "suitable reducing agent" is intended to mean any agent capable of reducing, preferably enantioselectively or diastereoselectively reducing, the C-24 keto group of a compound of general structure XX, Va, Vb, VIIIa, or VIIIb to give preferably a compound of general structure XXIa (R₆=hydrogen), IXa, Xa, XIaa, or XIba respectively. Examples of reducing agents include, but are not limited to borane reducing agents, metallic hydrides, such as lithium aluminium hydride, sodium borohydride, or AlH₃, optionally in the presence of lanthanide salts (e.g. LaCl₃, CeBr₃, CeCl₃), or NaBH₃(OAc), Zn(BH₄)₂, and Et₃SiH. Borane reducing agents include borane, borohydrides, and borane complexes with amines or ethers. Non-limiting examples of borane reducing agents e.g. include *N*,*N-*diethylaniline-borane, borane-tetrahydrofuran, 9-borabicyclononane (9-BBN), or borane dimethylsulfide. Other reducing agents include, but are not limited to, hydrogen in the presence of a catalyst, such as platinum or ruthenium, sodium in ethanol, isopropyl alcohol and aluminium isopropoxide, and zinc powder in water or alcohol.

When reducing the C-24 keto group of a compound of general structure XX, XVIa, XVIb, VIIIa, or VIIIb, the term "suitable reducing agent" includes chiral reducing agents or chiral ligand-reducing agent complexes, such as the complex of LiAlH₄ and 2,2'-dihydroxy-1,1'binaphthyl . Other examples are hydrogen in the presence of binaphthyl derivatives, such as 2,2'-dihydroxy-1,1'binaphthyl derivatives, e.g. (*R*)-2,2'-bis(diphenylphosphino)-1,1'-binaphthyl-ruthenium acetate.

Chiral reducing agents or chiral ligand-reducing agents include reducing agents where a chiral auxiliary is reacted with the reducing agent prior to the reduction *in situ* to form a chiral reducing agent or the where the chiral auxiliary may for example serve as a chiral ligand in a complex with the reducing agent, i.e. for example to give a chiral reducing agent. The present invention includes the use of such chiral reducing agents or chiral ligand-reducing agent complexes, which were prepared and isolated separately before being used for the reduction.

For example, the chiral auxiliary may react with a borane reducing agent prior to the reduction *in situ* to form a chiral borane reducing agent or the chiral auxiliary may serve as a chiral ligand in a borane complex. Examples of such chiral borane reducing agents are chiral oxaborolidines or oxazaborolidines, such as chiral oxazaborolidine reagents derived from (1R,2S)-*cis*-1-amino-2-indanol, (1S,2R)-*cis*-1-amino-2-indanol, (S)-prolinol, (R)-prolinol or B-(3-pinanyl)-9-borabicyclo[3.3.2]nonane (alpine-borane), or e.g. 5,5-diphenyl-2-methyl-3,4-propano-1,3,2-oxazaborolidine, (S)-2-methyl-CBS-oxazaborolidine, (R)-2-methyl-CBS-oxazaborolidine. The present invention therefore includes the use of such chiral reducing agents, such as chiral borane reducing agents, or chiral ligand-reducing agent complexes, such as chiral ligand-borane complexes, which were prepared and isolated before being used for the reduction.

Another example of a chiral ligand in a complex with the reducing agent is the complex of LiAlH₄ and 2,2'-dihydroxy-1,1'binaphthyl.

The reduction of a compound of general structure XX, XVIa, XVIb, VIIIa, or VIIIb may be carried out in the presence of a chiral auxiliary, such as in an inert solvent. Non-limiting examples of chiral auxiliaries include chiral 1,2-amino-alcohols, such as chiral *cis*-1-amino-2-indanol derivatives, such as (15,2*R*)-(-)-*cis*-1-amino-2-indanol, or *cis*-1-amino-1,2,3,4-tetrahydronaphthalen-2-ol, such as (1*S*,2*R*)-*cis*-1-amino-1,2,3,4-tetrahydronaphthalen-2-ol. Other examples are binaphthyl derivatives, such as (*R*)-2,2'-bis(diphenylphosphino)-1,1'-binaphthyl-ruthenium acetate 2,2'-dihydroxy-1,1'binaphthyl derivatives. Further examples include but are not limited to (R)-(+)-α,α-diphenyl-2-pyrrolidinmethanol, (R)-(+)-2-amino-4-methyl-1,1-diphenyl-1-pentanol, (R)-(-)-2-amino-3-methyl-1,1-diphenyl-1-butanol, (R)-(+)-2-amino-1,1,3-triphenyl-1-propanol, and (1R,2S)-(-)-2-amino-1,2-diphenyl ethanol.

As used herein, "separating a compound" includes the purification and/or isolation of a compound, e.g. to at least 90% purity, such as to at least 95% purity, such as 97% purity, 98% purity, or 99% purity. The term "separating a compound" also includes the meaning of enhancing the concentration of the compound in a mixture of such compounds, optionally comprising solvents, such that the mixture is further enriched with a desired or preferred compound or isomer, such as an epimer, after said separation. Most preferably R₁ and/or R₂ represent alkylsilyl, such as *tert-*butyldimethylsilyl, and most preferably R₁ and R₂ are the same, and R₆ is hydrogen when compounds of the present invention are separated by chromatography.

As used herein, "inert solvent" means any organic solvent compatible with said suitable reducing agent under the reaction conditions employed, or mixtures of such solvents. The choice of such solvent will depend on the specific reducing agent used. Non-limiting examples of inert solvents include hydrocarbons, such as toluene, and ethers, such as *tert*-butyl methyl ether or tetrahydrofuran.

### Preferred embodiments

In another aspect, this invention relates to 20(R),1(S),3(R)-bis(*tert-*butyldimethylsilyloxy)-20-(3'-cyclopropyl-3'-oxoprop-1'(*E*)-enyl)-9,10-secopregna-5(*E*),7(*E*),10(19)-triene obtained by a process comprising the method of reacting a compound of general structure IIIa with a phosphonate of general structure VII.

In a further aspect, this invention relates to 20(R),1(S),3(R)-bis(*tert-*butyldimethylsilyloxy)-20-(3'-cyclopropyl-3'-oxoprop-1'(*E*)-enyl)-9,10-secopregna-5(*Z*),7(*E*),10(19)-triene obtained by a process comprising the method of reacting a compound of general structure IIIb with a phosphonate of general structure VII.

In a still further aspect, this invention relates to the SO₂ adducts of 20(R),1(S),3(R)-bis(*tert*-butyldimethylsilyloxy)-20-(3'-cyclopropyl-3'-oxoprop-1'(*E*)-enyl)-9,10-secopregna-5(*E*),7(*E*),10(19)-triene obtained by a process comprising the method of reacting a compound of general structure VIa or VIb with a phosphonate of general structure VII.

In a currently preferred embodiment of the present invention R₁ and/or R₂ represent alkylsilyl, such as *tert*-butyldimethylsilyl, most preferably R₁ and R₂ are the same.

In another embodiment of the present invention R₁ and/or R₂ represent hydrogen, most preferably R₁ and R₂ are the same.

In a currently preferred embodiment of the present invention R₃ and/or R₄ represent alkyl, such as (C₁-C₆)alkyl, such as methyl, ethyl, or 1-propyl, most preferably R₃ and R₄ are the same.

In one embodiment of the present invention the hydroxy protecting group R₅ is alkylsilyl, such as triethylsilyl, and the hydroxy protecting group R₆ is alkylsilyl, such as *tert-*butyldimethylsilyl.

Compounds and intermediates of the present invention may comprise asymmetrically substituted (chiral) carbon atoms and carbon-carbon double bonds which may give rise to the existence of isomeric forms, e.g. enantiomers, diastereomers and geometric isomers. Epimers are known as diastereomers that have opposite configuration (R or S) at only one of multiple tetrahedral stereogenic centres in molecules having multiple stereogenic centres, such as the vitamin D analogues to which the present invention is directed. Designation of, for example, C-24 as the epimeric centre of a pair of enantiomers therefore implies that the configuration at the other stereogenic centres of the pair are identical. The present invention relates to all isomeric forms, such as epimers, either in pure form or as mixtures thereof. Pure stereoisomeric forms of the compounds and the intermediates of this invention may be obtained by the application of procedures known in the art, such as by chromatography or crystallisation, or by stereoselective synthesis.

The indication of a specific conformation or configuration either in the formulas or the names of compounds or intermediates of the present invention shall indicate that this specific conformation or configuration is a preferred embodiment of the invention. The indication of a specific conformation or configuration either in the formulas or the names of compounds or intermediates of the present invention shall include any other isomer than specifically indicated, either in pure form or as mixtures thereof, as a further embodiment of the present invention.

### Methods of preparation

Compounds of general structure IIIa can for example be synthesised according to methods disclosed for example by M. J. Calverley, Tetrahedron, Vol. 43, No. 20, pp. 4609-4619, 1987 or in WO 87/00834. For example compound IIIa, wherein both R₁ and R₂ are *tert*-butyldimethylsilyl which preparation is described in these references can be deprotected with aqueous hydrofluoric acid in acetonitrile or with tetrabutylammonium fluoride to give a mixture of compounds wherein R₁ or R₂ are hydrogen, or to give a compound wherein R₁ and R₂ are hydrogen. This mixture of compounds can for example be separated by chromatography or crystallised as generally described herein. By reaction of said compounds of general structure IIIa, wherein R₁ and/or R₂ are hydrogen with a suitable protecting agent, new groups R₁ and/or R₂ can be introduced. Depending on the stoichiometry of the protecting agent used and the reaction conditions, mixtures of unprotected, monoprotected, and deprotected compounds can be obtained. Any intermediate of a mixture wherein one of R₁ or R₂ is hydrogen can then be isolated by chromatography and reacted with suitable protecting agent different from the first one used, to give compounds of general structure IIIa, wherein R₁ is different from R₂.

Compounds of general structure IIIb can be obtained from compounds of general structure IIIa by photo isomerisation, such as with UV-light in the presence of a triplet sensitizer, such as anthracene or 9-acetylanthracene. Such processes are well known to a person skilled in the art of vitamin D-derivatives and are for example described by M. J. Calverley, Tetrahedron, Vol. 43, No. 20, pp. 4609-4619, 1987 or in WO 87/00834 .

Compounds of general structure VIa and/or VIb can be obtained from compounds of general structure IIIa or IIIb by treatment of a compound of general structure IIIa or IIIb with sulphur dioxide. The sulphur dioxide used can be liquid, gaseous or being dissolved in a suitable solvent. Suitable solvents for this Diels-Alder type reaction are all solvents, which are compatible with the reaction conditions, such as alkanes, such as hexane or heptane, hydrocarbons, such as xylenes, toluene, ethers, such as diethyl ether or methyl-*tert*-butyl ether (MTBE), acetates, such as ethyl acetate or 2-propyl acetate, halogenated solvents such as dichloromethane, or mixtures of said solvents, such as a mixture of a water immiscible solvent and water, e.g. toluene and water. The reaction can also be carried out in neat sulphur dioxide without a solvent. A suitable reaction temperature of the process is -50°C to 60 °C, such as -30°C to 50°C, such as - 15°C to 40°C, such as -5°C to 30°C, such as 0°C to 35°C, such as 5°C to 30°C most such as 10°C to 25°C, such as 15°C to 20°C. Preferably the sulphur dioxide is used in excess (mol/mol), such as 5-100 molar excess, such as 7-30 molar excess, such as 10-15 molar excess. Any excess of unreacted sulphur dioxide can be removed from the reaction mixture by e.g. washing with aqueous base, such as aqueous sodium hydroxide or by distilling the sulphur dioxide off, optionally together with a solvent, optionally under reduced pressure. Reacting compounds of general structure IIIa with sulphur dioxide usually leads to mixtures of the two epimers VIa and VIb. The molar ratio VIa/VIb of the mixture of the epimers obtained in the Diels-Alder reaction will depend on the groups R₁ and R₂ and the reaction conditions used.

Compounds of general structure XVa and XVb can for example be synthesised as previously described in EP 0078704 for R₁ = *tert*-butyldimethylsilyloxy (Example 11 (c). Compounds XVa and XVb, wherein R₁ is *tert*-butyldimethylsilyl can for example be deprotected with a suitable deprotecting reagent, such as aqueous hydrofluoric acid in acetonitrile or with tetrabutylammonium fluoride to give compounds, wherein R₁ is hydrogen, which then can be reacted with a suitable protecting agent, to give compounds of general structure XVa and XVb with a group R₁ different from the starting compound. Furthermore compounds of general structure XVa and XVb can be synthesised by ozonolysis of compounds 6a, 6b, 7a, or 7b disclosed in Tetrahedron, Vol. 43, No. 20, pp. 4609-4619, 1987.

Compounds of general structure XIIIa can for example be synthesised starting from the sulphur dioxide adducts XVa and XVb by base assisted retro Diels-Alder reaction, such as described below. Different groups R₁ may be introduced, before or after the retro Diels-Alder reaction, by methods well known to a person skilled in the art of organic chemistry and as for example described above for compounds of general structure IIIa.

Compounds of general structure XIIIb can be obtained from compounds of general structure XIIIa, and vice versa, by photo isomerisation as described above.

The C,D-ring building blocks of general structure IXX can for example be prepared from vitamin D₂ (ergocalciferol) by methods disclosed in Eur. J. Org. Chem, 2003, 3889-3895; J. Med. Chem. 2000, 43, 3581-3586; J. Med. Chem. 1995, 38, 4529-4537, Chemical Reviews, 1995, Vol. 95, No.6, and J. Org. Chem. 1992, 57, 3173-3178. Different groups R₅ can be introduced by using standard protection group chemistry such as described herein.

The sulphur dioxide adducts of the present invention are preferably converted to the unprotected triene derivatives in the presence of a base in a retro Diels-Alder reaction. The reaction may be carried out in all solvents, which are compatible with the reaction conditions, such as alkanes, such as hexane or heptane, hydrocarbons, such as xylenes, toluene, ethers, such as diethyl ether or methyl-*tert*-butyl ether (MTBE), acetates, such as ethyl acetate or 2-propyl acetate, halogenated solvents such as dichloromethane, water or mixtures of said solvents. Methods of this retro Diels Alder type reaction are well known to a person skilled in the art of vitamin D synthesis (see e.g. M. J. Calverley, Tetrahedron, Vol. 43, No. 20, pp. 4609-4619, 1987 or in WO 87/00834). Preferred solvents are toluene, *tert*-butyl methyl ether, water, or mixtures thereof. Suitable bases to be used in the retro Diels-Alder reaction include, but are not limited to NaHCO₃, KHCO₃, Na₂CO₃, or K₂CO₃. In a preferred embodiment of the present invention, the base is aqueous NaHCO₃ and/or the retro Diels-Alder reaction is run above 60°C, such as between 60°C and 120 °C, most preferably above 70°C, such as between 74°C and 79 °C, typically for about one-two hours.

Compounds of general structure VIa and/or VIb can be further obtained by ozonolysis of the SO₂ adducts of 1(S),3(R)-bis(tert-butyldimethylsilyloxy)-9,10-seco-ergosta-5,7(E),10(19),22(E)-tetraene as for example described in Tetrahedron, Vol. 43, No. 20, pp. 4609-4619, 1987, optionally followed by deprotection and protection of the hydroxy groups as described above for compounds of general structure IIIa and/or IIIb.

The synthetic methods used in the present invention are well known to a person skilled in the art of vitamin D synthesis or organic chmistry. Suitable reaction conditions can e.g. be found in Tetrahedron, Vol. 43, No. 20, pp. 4609-4619, 1987, in WO 87/00834, in WO 94/15912, in US 69,553,962, and in Chemical Reviews, 1995, Vol. 95, No.6; and the references cited therein.

The reduction of the compounds of general structure VIIIa and/or VIIIb, or XVIa and/or XVIb respectively, or XX is preferably carried out by reacting with a chiral borane reducing agent, such as a chiral oxaborolidines or oxazaborolidines, such as chiral oxazaborolidine reagents derived from *N*,*N-*diethylaniline-borane and (1S,2R)-*cis*-1-amino-2-indanol, (1R,2S)-*cis*-1-amino-2-indanol, (1S,2R)-*cis*-1-amino-2-indanol, (S)-prolinol, (R)-prolinol or B-(3-pinanyl)-9-borabicyclo[3.3.2]nonane (alpine-borane), or e.g. 5,5-diphenyl-2-methyl-3,4-propano-1,3,2-oxazaborolidine, (S)-2-methyl-CBS-oxazaborolidine, (R)-2-methyl-CBS-oxazaborolidine. These reduction methods and methods for the preparation of the compounds of general structure VIIIa and/or VIIIb have been described in detail in US Appl. No. 60/553,962. The molar ratio of chiral auxiliary/reducing agent is preferably in the range of 2.3-2.7. The reduction reaction is usually carried out in a temperature interval between 5°C to 35°C, preferably 10°C to 30°C, preferably 15°C to 25°C, most preferably 15°C to 20°C. The reducing agent is preferably used in an equimolar amount or in molar excess to a compound of general structure VIIIa and/or VIIIb, or XVIa and/or XVIb respectively, or XX, such as in 2.5-3.0 molar excess.

The process results in the enantioselective/diastereoselective reduction of the prochiral ketone of general structure VIIIa and/or VIIIb, or XVIa and/or XVIb respectively, or XX, such that the C-24 epimers XIa and/or XIb, or XVIa and/or XVIb respectively, or XXIa (R₆=hydrogen) are formed in preference. Such borane-catalysed reactions were for example reviewed by Deloux and Srebnik [Chem. Rev. 93, 763, 1993]. Examples of efficient catalysts based on chiral modified borane can for example be found in [A. Hirao, J. Chem. Soc. Chem. Commun. 315, 1981; E.J. Corey, J. Am. Chem. Soc. 109, 7925, 1987]. Examples of the synthesis and/or use of e.g. 1,2- and 1,3-amino alcohols in stereoselective reduction with borane can e.g. be found in [E. Didier et al.; Tetrahedron 47, 4941-4958, 1991; C.H. Senanayake et al., Tetrahedron Letters, 36(42), 7615-18, 1995, EP 0698028, EP 0640089, EP 0305180, WO 93/23408, WO 94/26751]. The synthesis and/or use of chiral *cis*-1-amino-2-indanol derivatives in borane reductions can e.g. be found in [C.H. Senanayake, Aldrichimica Acta, 31 (1), 1-15, 1998; A.K. Ghosh et. al., Synthesis, 937-961, 1998; Y. Hong et. al., Tetrahedron Letters, 35(36), 6631-34, 1994; B. Di Simone, Tetrahedron Asymmetry, 6(1) 301-06, 1995; Y. Hong et al., Tetrahedron Letters, 36(36), 6631-34, 1994; R. Hett et al., Org. Process Res. & Dev., 2, 96-99, 1998; or EP 0763005], and references cited therein.

The method for producing calcipotriol as described herein may be modified with regard to the order of the reaction steps, by omitting one or more reaction steps, or by introducing additional purification or reaction steps at any stage of the reaction sequence. The present invention includes all such modifications. A person skilled in the art of vitamin D chemistry or organic chemistry will know where such modifications can be made.

The method for producing calcipotriol as described herein includes further all variants, where the hydroxy protecting groups R₁ and/or R₂ for compounds or intermediates, where R₁ and/or R₂ are not hydrogen, are removed at any stage of the reaction sequence. Compounds or intermediates, where R₁ and/or R₂ are hydrogen may be protected with protecting agents at any stage of the reaction sequence, including protecting agents which yield other protecting groups than those removed earlier in the reaction sequence.

The reduction of a compounds of general formula XIVa, XIVb, XVIa, XVIb, XX, Va, Vb, VIIIa, and/or VIIIb with a suitable reducing agent in an inert solvent will, depending on the reducing agent and the reaction conditions used, give a mixture of the C-24 epimers of the corresponding alcohols formed, such as the compounds of general structures IXa and IXb, or such as the compounds of general structure Xa and Xb, or such as the compounds of general structure XIaa and XIab or XIba and XIbb, or such as XXIa and XXIb. Depending of the composition of the mixture, the desired epimers XXIa, IXa, Xa, XIaa, or XIba are advantageously separated by common purification methods known to the skilled person in the art before proceeding in the reaction sequence.

The separation, isolation, and purification methods of the present invention include, but are not limited to chromatography, such as adsorption chromatography (including column chromatography and simulated moving bed (SMB)), crystallisation, or distillation. The separation, isolation, and purification methods may be used subsequently and in combination. Column chromatography, useful for the separation of vitamin D analogues of the present invention is well known to those skilled in the art of pharmaceutical chemistry. The technique employs a column packed with a stationary phase, for example silica, such as pretreated silica onto which sample to be separated is loaded. The sample is then eluted with a suitable eluent. Elution can be isocratic or so-called solvent programmed (gradient), wherein the composition of the eluent is varied regularly (e.g. linearly) or irregularly (e.g. stepwise over time. Pretreated silica gel, well known to a person skilled in the art of chromatography, is a suitable stationary phase. Elution with 5% (v:v) ethyl acetate in hexane or heptane followed by neat ethyl acetate is but one example of an elution program that produces the desired separation. Other suitable eluents will be deduced by the skilled person through routine methods of development, e.g. by using mixtures of heptane and ethylacetate of suitable polarity. For the chromatography steps, any combination of stationary phase (packing) and eluent that is capable of resolving the mixtures, e.g. if C-24 epimers, can be used. Such combinations can be readily determined by the skilled person by routine experimentation.

The Horner-Emmons reagents of general structure VII can be synthesized by various synthetic approaches, ranging from the direct Arbuzov reaction of trisubstituted phosphites, e.g. trialkylphosphites, such as triethylphosphite or trimethylphosphate, with 2-halo-1-cyclopropylethanone, such as 2-chloro-1-cyclopropylethanone or 2-bromo-1-cyclopropylethanone [B.A. Arbuzov, Pure Appl. Chem. 1964, 9, 307] to methods using organometallic reagents (see for example references 5 (a)-(k) in [B. Corbel et al., Synth. Communications, 1996, 26(13), 2561-2568]). Other methods of preparation include the Michaelis-Becker process [G. Sturtz, Bull. Soc. Chim. Fr., 1964, 2333] and the use of masked carbonyl compounds (see for example references 8 (a)-(k) in [B. Corbel et al., Synth. Communications, 1996, 26(13), 2561-2568]. A safe and economical procedure for the preparation of (β-keto phosphonates is based on the acylation of magnesium enolate derivative of trialkylphosphonoacetate using magnesium chloride-triethylamine followed by decarboxylation [D.Y. Kim, Synth. Commun. 1996, 26(13), 2487-2496; B. Corbel et al., Synth. Commun., 1996, 26(13), 2561-2568]. Another approach is based on the reactions of α-halophosphonates with esters promoted by a soluble Co(0) complex or by magnesium metal [F. Orsini, Synthesis, 2002, 12, 1683-1688]. Many other procedures are described in the literature and can for example be found in references cited in the above articles, e.g. by D.Y. Kim et al. and by F. Orsini et al..

The Wittig-Horner reaction is usually performed by mixing a compound of general structure IXX, XXII, IIIa, IIIb, VIa and/or VIb, XIIIa, XIIIb, XVa and/or XVb with a phosphonate and a base in an appropriate solvent. The addition of reagents may be in either order, though the addition of the base as the last reagent to the stirred mixture can be advantageously depending on the base used.

Preferably, the phosphonates of the general structure VII include groups R₃ and/or R₄, which render the corresponding phosphate esters XII water soluble, as this will allow the removal of the phosphate esters XII by aqueous extraction from the reaction mixture. 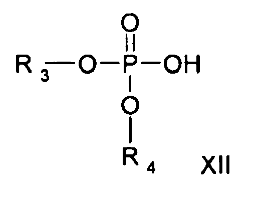 For example those groups of R₃ and/or R₄ of compounds VII or XII are advantageous, which result in a water solubility for compounds of general structure XII of at least 0.1mg/ml at pH 9.5 and 20°C, such as at least 0.5mg/ml at pH 9.5 and 20°C, such as at least 1mg/ml at pH 9.5 and 20°C, such as at least 5mg/ml at pH 9.5 and 20°C, such as at least 10mg/ml at pH 9.5 and 20°C.

In a another embodiment of the invention, phosphonates of general structure VII are preferred, where the water solubility of the corresponding phosphonic acid XII is equal or higher in comparison to the solubility of phosphonic acid XII where R₃ and R₄ are ethyl. Appropriate solvents for the Wittig-Horner reaction include hydrocarbons, such as xylenes, toluene, hexanes, heptanes, cyclohexane, and ethers, such as *tert*-butyl methyl ether, diethyl ether, 1,4-dioxane, diethoxymethane, 1,2-dimethoxyethane, or tetrahydrofuran, and other solvents such as acetonitrile, 2-methyltetrahydrofuran, diglyme, monoglyme, NMP, DMF, DMSO, or acetates, such as ethyl acetate or 2-propyl acetate, or halogenated solvents such as dichloromethane, chlorobenzene, or water, or mixtures of said solvents.

In a preferred embodiment of the invention the reaction is carried out under phase transfer conditions using a mixture of water and a water-immiscible solvent, such as toluene or xylene with a suitable phase transfer catalyst, such as a tetraalkylammonium salt, e.g. a tetrabutylammonium hydroxide, halide, or hydrogensulfate, such as tetrabutylammonium bromide or chloride, or tetrabutylammonium hydrogensulfate.

Suitable bases for the Wittig-Horner reaction include hydroxides, such as tetraalkylammonium hydroxides, e.g. tetrabutylammoniumhydroxide, or alkalimetalhydroxides, such as sodium hydroxide, potassium hydroxide, or group 2 element hydroxides, such as Mg(OH)₂, including aqueous solutions of such hydroxides. Other suitable bases include, depending on the reaction conditions and solvents used, sodium hexamethyldisilazane (NaHMDS) or hydrides, such as sodium or calcium hydride, or alkoxides, such as sodium ethoxide, potassium *tert*-butoxide, or lithium *tert*-butoxide.

The reaction temperature for the Wittig-Horner reactions will depend on the reaction conditions and solvents used. Typically for the reaction of compounds of general structure VIa and/or VIb, or XVa and/or XVb, reaction temperatures above 50°C should be avoided. Suitable reaction temperature for the Wittig-Horner reaction of VIa and/or VIb, or XVa and/or XVb, are in the range of -80°C to 50°C, such as -50°C to 50°C, such as -30°C to 50°C, such as -15°C to 40°C, such as -5°C to 35°C, such as 0°C to 35°C, such as 5°C to 30°C, such as 10°C to 30°C, such as 15°C to 30°C, such as 10°C to 25°C, such as 5°C to 20°C. Suitable reaction temperature for the Wittig-Horner reaction of IXX, XXII, IIIa, IIIb, XIIIa, or XIIIb are in the range of -80°C to 150°C, such as - 50°C to 150°C, -40°C to 120°C, such as -30°C to 100°C, -20°C to 80°C, such as - 15°C to 60°C, such as -10°C to 50°C such as -5°C to 40°C, such as 0°C to 35°C, such as 5°C to 30°C, such as 10°C to 30°C, such as 15°C to 30°C, such as 10°C to 25°C, such as 5°C to 20°C.

The phosphonate VII or XXIIIb is usually used in an equimolar amount or in molar excess with regard to the aldehydes, such as 10% excess, or 30 % excess, or 50 % excess, or 65 % excess, or 70 % excess, or 80 % excess, or 90 % excess, or 100 % excess, or 150 % excess, or 200 % excess, or 300% excess.

The base is usually used equimolar or in molar excess with regard to the phosphonate VII or XXIIIb, such as 10% excess, or 30 % excess, or 50 % excess, or 65 % excess, or 70 % excess, or 80 % excess, or 90 % excess, or 100 % excess, or 150 % excess, or 200 % excess, or 300 % excess, or 350 % excess, or 400 % excess, or 425 % excess, or 450 % excess, or 500 % excess.

The optimal reaction conditions for the Wittig-Horner reaction, such as the solvents, bases, temperature, work-up procedures, stoichiometries, or the reaction times will depend on the starting compounds, e.g. the groups R₁ and/or R₂ in the aldehydes of general structure IIIa, IIIb, VIa, VIb, XIIIa, XIIIb, XVa, or XVb, and the group R₆ of the aldehydes XXII, and the phosphonates VII and XXIIIb, e.g. the groups R₃ and R₄.

The stereoselectivity (trans-selectivity) of the reaction may be controlled by the reaction conditions and the choice of the phosphonate VII and XXIIIb (groups R₃ and R₄).

The oxidation of the compounds of general structure XXIa, wherein R₅ is hydrogen and R₆ is hydrogen or preferably a hydroxy protecting group, such as *tert*-butyldimethylsilyl, to a compound of general structure XXII may for example be performed with pyridinium dichromate (PDC), Dess-Martin reagent, pyridinium chlorochromate (PCC), *N-*methylmorpholine *N-*oxide (NMO), such as *N-*methylmorpholine *N-*oxide on silica, tetrapropylammonium perrhutenate, for example in dichloromethane.

The Wittig reagent XXIIIa can be prepared according to the methods described in Chemical Reviews, 1995, Vol. 95, No.6 and J. Org. Chem. 2002, 67, 1580-1887. The Wittig Horner raegent XXIIIb may for example be prepared from compound 6 disclosed in J. Org. Chem. 2002, 67, 1580-1887, followed by reaction with suitable halogenating agent, such as thionyl chloride, and reaction of the resulting halogenide or chloride with triethyl phosphate in a Michaelis Arbuzov reaction, such as by heating with triethylphosphite.

Coupling conditions of coupling compound XXII with XXIIIa or XXIIIb can also be found in Chemical Reviews, 1995, Vol. 95, No.6, or J. Org. Chem. 2002, 67, 1580-1887, and references cited therein. A suitable base is for example an lithiumalklyl derivative, such as sec-butyl lithium or n-butyllithium.

Hydroxylation, such as hydroxylation of the compound of general structure XIVa can be achieved with a suitable hydroxylating agent, for example by a selenite mediated allylic hydroxylation, such as under the conditions developed by Hesse, e.g. with selene dioxide (SeO₂), such as with SeO₂ and *N-*methylmorpholine *N-*oxide in refluxing methanol and/or dichloromethane) [J. Org. Chem. 1986, 51, 1637] or as described in Tetrahedron Vol. 43. No.20, 4609-4619, 1987 or in WO87/00834. The undesired hydroxy epimer formed during hydroxylation may be removed by the general separation and chromatography methods described herein.

Calicpotriol hydrate can be obtained by crystallisation of calcipotriol from aqueous solvents, such as for example by methods described in WO 94/15912.

### EXAMPLES

### General:

All chemicals, unless otherwise noted were from commercial sources. For ¹H nuclear magnetic resonance (NMR) spectra (300 MHz) and ¹³C NMR (75.6 MHz) chemical shift values (δ) (in ppm) are quoted, unless otherwise specified; for deuteriochloroform solutions relative to internal tetramethylsilane (δ = 0.00) or chloroform (δ = 7.26) or deuteriochloroform (δ = 76.81 for ¹³C NMR) standard. The value of a multiplet, either defined (doublet (d), triplet (t), quartet (q)) or not (m) at the approximate mid point is given unless a range is quoted. All organic solvents used were of technical grade. Chromatography was performed on silica gel optionally using the flash technique. Preferably the silica was from Merck KGaA Germany: LiChroprep® Si60 (15-25µm). Appropriate mixtures of ethyl acetate, dichloromethane, methanol, hexane and petroleum ether (40-60) or heptane were used as eluents unless otherwise noted. Experimental conditions regarding melting points, elemental analysis, UV-VIS absorption, ¹H NMR, and mass spectrometry data were, unless otherwise noted, as described by M. J. Calverley in Tetrahedron, Vol. 43, No. 20, p. 4614-15, 1987.

### Preparation 1:

### (2-cyclopropyl-2-oxoethyl)phosphonic acid diethyl ester

### Compound VII (R_{3,} R₄_= ethyl)

Cyclopropane carbonyl chloride (ALDRICH) (125g) was added slowly to a mixture of anhydrous magnesium chloride (102 g), triethylphosphonoacetate (219 g), and triethyl amine (310 g) in toluene (1600 ml) with stirring keeping the temperature below 25°C. The mixture was stirred for another 30 minutes followed by the cautious addition of first water (950 ml), followed by a mixture of concentrated hydrochloric acid (250 ml) and water (350 ml), keeping the temperature below 25°C. The organic phase was separated, washed with an aqueous sodium chloride (400g NaCl in 1200 ml water) and then washed with water (1600 ml). The organic phase was then concentrated *in vacuo* to the lowest possible volume to give 3-cyclopropyl-2-(diethoxyphosphoryl)-3-oxo-propionic acid ethyl ester as an oil. Water was added (40 ml) to the the oil and this mixture was refluxed for approximately 3 hours. More water (2000 ml) was added to the reaction mixture and the title compound was extracted with methylene chloride. The solvents were removed *in vacuo* to give the title compound as oil. The ³¹P NMR, and mass spectrometry data were found to be in full accordance with structure. ¹H NMR (CDCl₃):
4.16 (m,4H), 3.21 (d,2H), 2.20 (m,1H), 1.34 (t,6H), 1.11 (m,2H), 0.98 (m,2H) ppm.

### Preparation 2:

### (2-cycloproyl-2-oxoethyl)phosphonic acid dimethyl ester

### Compound VII (R₃, R₄ = methyl)

The same procedure as in Preparation 1 may be used, but using trimethylphosphonoacetate instead of triethylphosphonoacetate. The ³¹P NMR, and mass spectrometry data were found to be in full accordance with the structure. ¹H NMR (CDCl₃): 3.80 (d,6H), 3.22 (d,2H), 2.17 (m,1H), 1.11 (m,2H),0.98 (m,2H) ppm.

### Example 1:

### 20(R),1(S),3(R)-bis(tert-butyldimethylsilyloxy)-20-(3'-cyclopropyl-3'-oxoprop-1'(E)-enyl)-9,10-secopregna-5(E),7(E),10(19)-triene

### Compound Va (R₁, R₂ = tert-butyldimethylsilyl)

A mixture of (2-cyclopropyl-2-oxoethyl)phosphonic acid diethyl ester (compound VII /R₃, R₄ = ethyl) (46.0 g, 209mmol), 1(S),3(R)-bis(*tert*-butyldimethylsilyloxy)-20(S)-formyl-9,10-secopregna-5(*E*),7(*E*),10(19)-triene (compound IIIa / R₁, R₂ = *tert-*butyldimethylsilyl) prepared according to M. J. Calverley, Tetrahedron, Vol. 43, No. 20, pp. 4609-4619, 1987 (72.2 g, 126mmol), toluene (1100 ml), water (122 ml), tetrabutyl ammonium bromide (3.13 g), and sodium hydroxide solution 27.7% (128.0 g) was stirred at 30°C for approximately one hour followed by stirring at ambient temperature (15-25°C) overnight. When the reaction was judged to be complete as checked by HPLC [Column LiChrosorb Si 60 5 µm 250x4mm from Merck, 1.5 ml/min flow, detection at 270nm, hexane/ethylacetate 100:2 (v:v)], water was added (500 ml). The pH of the reaction mixture was adjusted to pH 8.5-9.5 by addition of phosphoric acid solution (ca. 20%) keeping the temperature between 20-25°C. The organic phase was separated followed by the addition of hexane (200ml) and methanol (170 ml). The organic phase was once washed with a mixture of water (670 ml), saturated aqueous sodium chloride (120 ml), and saturated aqueous sodium hydrogen carbonate (20 ml). The organic solvents were removed *in vacuo* and the remainder was dissolved in a mixture of methanol (500 ml) and hexane. (580 ml), and the solution was then washed with water (400 ml). The organic solvents were again removed *in vacuo* and the remainder was crystallised from *tert*-butyl methyl ether/methanol. The crystals were filtered off, washed twice with methanol and dried under vacuum to give the title compound 20(R),1(S),3(R)-bis(*tert*-butyldimethylsilyloxy)-20-(3'-cyclopropyl-3'-oxoprop-1'(*E*)-enyl)-9,10-secopregna-5(*E*),7(*E*),10(19)-triene (65.2 g, 102 mmol). The melting point, elemental analysis, UV-VIS absorption, and mass spectrometry data were found to be in full accordance with the structure as described earlier by M. J. Calverley in Tetrahedron, Vol. 43, No. 20, p. 4616, 1987 for compound 17. ¹³C NMR (CDCl₃): 200.4, 153.4, 151.8, 142.5, 135.5, 128.1, 121.4, 116.5, 106.5, 70.0, 67.0, 56.0, 55.3, 46.0, 43.7, 40.2, 40.1, 36.4, 28.7, 27.4, 25.7, 25.6, 23.2, 22.1, 19.3, 18.5, 18.1, 17.9, 12.1, 10.7, 10.7, -5.0, -5.0, -5.1, -5.1 ppm.

### Example 1A:

### 20(R),1(S),3(R)-bis(tert-butyldimethylsilyloxy)-20-(3'-cyclopropyl-3'-oxoprop-1'(E)-enyl)-9,10-secopregna-5(E),7(E),10(19)-triene

### Compound Va (R₁ R₂ = tert-butyldimethylsilyl)

To a solution of (2-cyclopropyl-2-oxoethyl)phosphonic acid diethyl ester (compound VII /R₃, R₄ = ethyl) (1.51 g) and THF (16 ml) was added NaHMDS (sodium hexamethyldisilazane) (3.2ml, 2M in THF) over 10 min below -50 °C and stirred additionally for 3-4 hr followed by addition of a solution of 1(S),3(R)-bis(*tert-*butyldimethylsilyloxy)-20(S)-formyl-9,10-secopregna-5(*E*),7(*E*),10(19)-triene (compound IIIa/ R₁, R₂ = *tert*-butyldimethylsilyl) (2 g) in THF (3 ml) below -50 °C. The reaction was stirred additionally for 2 hr below -50 °C and then 2 hr at -25 °C before the temperature was elevated to room temperature overnight. The reaction was checked for completion by HPLC [Column LiChrosorb Si 60 5 µm 250×4mm from Merck, 1.5 ml/min flow, detection at 270 nm, hexane/ethylacetate 100:2 (v:v)].

### Example 1B:

### 20(R),1(S),3(R)-bis(tert-butyldimethylsilyloxy)-20-(3'-clopropyl-3'-oxoprop-1'(E)-enyl)-9,10-secopregna-5(E),7(E),7(E)10(19)-triene

### Compound Va (R_{1,}R₂ = tert-buyldimethylsilyl)

To a solution of (2-cyclopropyl-2-oxoethyl)phosphonic acid diethyl ester (compound VII /R₃, R₄ = ethyl) (1,51 g) and THF (16 ml) was added NaH (265 mg) over 3 min below -50 °C and stirred additionally for 2-3 hr followed by addition of a solution of 1(S),3(R)-bis(*tert*-butyldimethylsilyloxy)-20(S)-formyl-9,10-secopregna-5(*E*),7(*E*),10(19)-triene (compound IIIa / R₁, R₂ = *tert*-butyldimethylsilyl) (2.1 g) in THF (3 ml) below -50 °C . The reaction was stirred further for 2 hr below -50 °C and then 3.5 hr at -25 °C before the temperature was elevated to room temperature overnight. The reaction was checked for completion by HPLC [Column LiChrosorb Si 60 5 µm 250x4mm from Merck, 1.5 ml/min flow, detection at 270 nm, hexane/ethylacetate 100:2 (v:v)].

### Example 1C:

### 20(R),1(S),3(R)-bis(tert-butyldimethylsilyloxy)-20-(3'-cyclopropyl-3'-oxoprop-1'(E)-enyl)-9,10-secopregna-5(E),7(E),10(19)-triene

### Compound Va (R₁ R₂ = tert-butyldimethylsilyl)

To a solution of (2-cyclopropyl-2-oxoethyl)phosphonic acid dimethyl ester (compound VII / R₃, R₄ = methyl) (1,51 g) and THF (16 ml) was added NaHMDS (3.2ml, 2M in THF) over 10 min below -50 °C and stirred further 4 hr followed by addition of a solution of 1(S),3(R)-bis(*tert*-butyldimethylsilyloxy)-20(S)-formyl-9,10-secopregna-5(*E*),7(*E*),10(19)-triene (compound IIIa / R₁, R₂ = *tert*-butyldimethylsilyl) (2 g) in THF (3 ml). The reaction was stirred additionally for 2 hr below -50 °C and then 2 hr at -25 °C before the temperature was elevated to room temperature overnight. The reaction was checked for completion by HPLC [Column LiChrosorb Si 60 5 µm 250x4mm from Merck, 1.5 ml/min flow, detection at 270 nm, hexane/ethylacetate 100:2 (v:v)].

### Example 1D:

### 20(R),1(S),3(R)-bis(tert-butyldimethylsiyloxy)-20-(3'-cyclopropyl-3'-oxoprop-1'(E)-enyl-9,10-secopregna-5(E),7(E),10(19)-triene

### Compound Va (R_{1,} R₂ = tert-butyldimethylsilyl)

A mixture of (2-cyclopropyl-2-oxoethyl)phosphonic acid dimethyl ester (compound VII /R₃, R₄ = methyl) (1.08 g), 1(S),3(R)-bis(*tert*-butyldimethylsilyloxy)-20(S)-formyl-9,10-secopregna-5(*E*),7(*E*),10(19)-triene (compound IIIa / R₁, R₂ = *tert*-butyldimethylsilyl) (1.28 g), toluene (15 ml), water (1.2 ml), tetrabutyl ammonium bromide (49 mg), and sodium hydroxide solution 27.7% (1.54 ml) was stirred at 33°C overnight. The reaction was checked for completion by HPLC [Column LiChrosorb Si 60 5 µm 250x4mm from Merck, 1.5 ml/min flow, detection at 270 nm, hexane/ethylacetate 100:2 (v:v)].

### Preparation 3:

### 1(S),3(R)-bis(tert-butyldimethylsilyloxy)-20(S)-formyl-9,10-secopregna-5(Z),7(E),10(19)-triene.

Compound IIIb (R₁, R₂ = *tert*-butyldimethylsilyl).

1(S),3(R)-bis(*tert*-butyldimethylsilyloxy)-20(S)-formyl-9,10-secopregna-S(*E*),7(*E*),10(19)-triene (compound IIIa / R₁, R₂ = *tert*-butyldimethylsilyl) may be photoisomerised in toluene using anthracene as triplet sensitizer followed by chromatography of the crude product to give the title compound. ¹³C NMR (CDCl₃): 204.8, 148.1, 139.7, 135.4, 122.7, 118.2, 111.1, 71.9, 67.3, 55.4, 51.3, 49.6, 46.0, 45.9, 44.6, 40.1, 28.6, 26.3, 25.7, 25.6, 23.1, 22.3, 18.0, 18.0, 13.4, 12.2, -4.9, -5.0, - 5.3 ppm.

### Example 2:

### 20(R),1(S),3(R)-bis(tert-butyldimethylsilyloxy)-20-(3'-cyclopropyl-3'-oxoprop-1'(E)-enyl)-9,10-secopregna-5(Z),7(E),10(19)-triene.

### Compound Vb (R₁ R₂ = tert-butyldimethylsilyl).

The same procedure as in Example 1 may be used, using 1(S),3(R)-bis(*tert-*butyldimethylsilyloxy)-20(S)-formyl-9,10-secopregna-5(*Z*),7(*E*),10(19)-triene (compound IIIb / R₁, R₂ = *tert*-butyldimethylsilyl) as the starting material, except that the product may be purified by chromatography instead of crystallisation to give the title compound. ¹H NMR (CDCl₃): 6.78 (dd,1H), 6.24 (d,1H), 6.16 (d,1H), 6.02 (d,1H), 5.19 (d,1H), 4.87 (d,1H), 4.38 (m,1H), 4.20 (m,1H), 2.85 (dd,1H), 2.46 (dd,1H), 2.38 - 1.20 (m,16H), 1.13 (d,3H), 1.08 (m,2H), 0.91 (m,2H), 0.89 (s,18H), 0.59 (s,3H), 0.07 (m,12H) ppm.

### Preparation 4:

### 1(S),3(R)-dihydroxy-20(S)-formyl-9,10-secopregna-5(Z),7(E),10(19)-triene IIIb (R₁, R₂ = hydrogen).

1(S),3(R)-bis(*tert*-butyldimethylsilyloxy)-20(S)-formyl-9,10-secopregna-5(*Z*),7(*E*),10(19)-triene (compound IIIb / R₁, R₂ = *tert*-butyldimethylsilyl) from Preparation 3 may be deprotected with aqueous hydrofluoric acid (40%) to give the title compound IIIb (R₁, R₂ = hydrogen) compound. ¹H NMR (CDCl₃): 9.58 (d,1H), 6.37 (d,1H), 6.04 (d,1H), 5.33 (s,1H), 4.99 (s,1H), 4.43 (m,1H), 4.23 (m,1H), 2.85 (dd,1H), 2.60 (dd,2H), 2.44 - 2.26 (m,2H), 2.10 - 1.30 (m,14H), 1.14 (d,3H), 0.60 (s,3H) ppm.

### Example 4:

### 1(S),3(R)-dihydroxy-20(R)-(3'-cyclopropyl-3'-oxoprop-1'(E)-enyl)-9,10-secopregna-5(Z),7(E),10(19)-triene

### Compound Vb (R₁, R₂ = hydrogen)

The same procedure as in Example 1 may be used, using 1(S),3(R)-dihydroxy-20(S)-formyl-9,10-secopregna-5(*Z*),7(*E*),10(19)-triene (compound IIIb / R₁, R₂ = hydrogen) from Preparation IV as the starting material, except that the product may be purified by chromatography instead of crystallisation to give the title compound. ¹³C NMR (CDCl₃): 200.8, 152.1, 147.7, 142.2, 133.5, 128.3, 124.7, 117.4, 111.8, 70.7, 66.8, 56.1, 55.5, 46.1, 45.2, 42.8, 40.3, 40.2, 29.0, 27.4, 23.5, 22.3, 19.5, 18.7, 12.3, 11.0 ppm.

### Preparation 5:

### 1(S),3(R)-bis(trimethylsilyloxy)-20(S)-formyl-9,10-secopregna-5(Z),7(E),10(19)-triene.

### Compound IIIb (R₁, R₂ = trimethylsilyl).

1(S),3(R)-dihydroxy-20(S)-formyl-9,10-secopregna-5(*Z*),7(*E*),10(19)-triene (compound IIIb / R₁, R₂ = hydrogen) from Preparation 4 may be reacted with trimethyl silyl chloride in the presence of triethylamine in dichloromethane. The obtained raw product may be purified by chromatography to give the pure title compound. ¹³C NMR (CDCl₃): 204.7, 147.8, 140.1, 135.2, 122.9, 118.1, 111.4, 71.4, 67.0, 55.4, 51.3, 49.5, 46.0, 45.7, 44.6, 40.1, 28.7, 26.3, 23.2, 22.3, 13.4, 12.2, 0.0, -0.1 ppm.

### Preparation 6:

### 1(S)-tert-butyldimethylsilyloxy-3(R)-hydroxy-20(S)-formyl-9,10-secopregna-5(E),7(E),10(19)-triene

IIIa (R₁ = hydrogen, R₂ = *tert*-butyldimethylsilyl), and 1(S)-hydroxy-3(R)-*tert*-butyldimethylsilyloxy-20(S)-formyl-9,10-secopregna-5(E),7(*E*),10(19)-triene

IIIa (R₁ = *tert*-butyldimethylsilyl, R₂ = hydrogen).

1(S),3(R)-bis(*tert*-butyldimethylsilyloxy)-20(S)-formyl-9,10-secopregna-5(*E*),7(*E*),10(19)-triene (compound IIIa / R₁, R₂ = *tert*-butyldimethylsilyl) may be partially deprotected with tetrabutylammonium fluoride to give a mixture of the title compounds and the unprotected derivative IIIa (R₁, R₂ = hydrogen). The compounds of the mixture may be separated by column chromatography to give pure fractions of the title compounds IIIa (R₁ = hydrogen, R₂ = *tert*-butyldimethylsilyl), ¹H NMR (CDCl₃): 9.59 (d,1H), 6.50 (d,1H), 5.86 (d,1H), 5.01 (s,1H), 4.94 (s,1H), 4.48 (t,1H), 4.24 (m,1H), 2.88 (dd,1H), 2.62 (dd,1H), 2.50 - 2.30 (m,2H), 2.11 - 1.30 (m,14H), 1.13 (d,3H), 0.88 (s,9H), 0.60 (s,3H), 0.06 (s,3H), 0.04 (s,3H) ppm; and IIIa (R₁ = *tert-*butyldimethylsilyl, R₂ = hydrogen), ¹H NMR (CDCl₃): 9.59 (d,1H), 6.49 (d,1H), 5.86 (d,1H), 5.07 (s,1H), 4.95 (s,1H), 4.49 (m,1H), 4.20 (m,1H), 2.87 (dd,1H), 2.52 (dd,1H), 2.45 - 2.30 (m,2H), 2.12 - 1.31 (m,14H), 1.13 (d,3H), 0.86 (s,9H), 0.59 (s,3H), 0.06 (s,6H) ppm.

### Example 5:

### 1(S)-tert-butyldimethylsilyl-3(R)-hydroxy-20(R)-(3'-cyclopropyl-3'-oxoprop-1'(E)-enyl)-9,10-secopregna-5(E),7(E),10(19)-triene

### Compound Va (R₁ = hydrogen, R₂ = tert-butyldimethylsilyl)

The same procedure as in Example 1 may be used, using 1(S)-*tert*-butyldimethylsilyl-3(R)-hydroxy-20(S)-formyl-9,10-secopregna-5(*E*),7(*E*),10(19)-triene (compound IIIa /R₁ = hydrogen, R₂ = *tert*-butyldimethylsilyl) from Preparation 6 as the starting material, except that the product may be purified by chromatography instead of crystallisation gave the title compound. ¹H NMR (CDCl₃): 6.75 (dd,1H), 6.50 (d,1H), 6.14 (d,1H), 5.84 (d,1H), 5.00 (s,1H), 4.92 (s,1H), 4.47 (t,1H), 4.22 (m,1H), 2.85 (dd,1H), 2.62 (dd,1H), 2.43 (dd,1H), 2.29 (m,1H), 2.15 - 1.15 (m,15H), 1.11 (d,3H), 1.06 (m,2H), 0.87 (s,9H), 0.86 (m,2H), 0.59 (s,3H), 0.06 (s,3H), 0.04 (s,3H) ppm.

### Example 6:

### 1(S)-hydroxy-3(R)-tert-butyldimethylsilyl-20(R)-(3'-cyclopropyl-3'-oxoprop-1'(E)-enyl)-9,10-secopregna-5(E),7(E),10(19)-triene

### Compound Va (R₁ = tert-butyldimethylsilyl, R₂ = hydrogen)

The same procedure as in Example 1 may be used, using 1(S)-hydroxy-3(R)-*tert-*butyldimethylsilyl-20(S)-formyl-9,10-secopregna-5(*E*),7(*E*),10(19)-triene (compound IIIa / R₁ = *tert*-butyldimethylsilyl, R2 = hydrogen) from Preparation 6 as the starting material, except that the product may be purified by chromatography instead of crystallisation gave the title compound. ¹H NMR (CDCl₃): 6.76 (dd,1H), 6.49 (d,1H), 6.14 (d,1H), 5.85 (d,1H), 5.06 (s,1H), 4.95 (s,1H), 4.49 (m,1H), 4.19 (m,1H), 2.86 (dd,1H), 2.52 (dd,1H), 2.45 - 1.20 (m,17H), 1.12 (d,3H), 1.07 (m,2H), 0.88 (m,2H), 0.86 (s,9H), 0.59 (s,3H), 0.06 (s,6H) ppm.

### Example 7:

### 20(R),1(S),3(R)-bis(tert-butyldimethylsilyloxy)-20-(3'-cyclopropyl-3'-oxoprop-1'(E)-enyl)-9,10-secopregna-5(E),7(E),10(19)-triene SO₂-adducts

### Compound VIIIa and VIIIb (R₁,R₂ = tert-butyldimethylsilyl)

A mixture of (2-cyclopropyl-2-oxoethyl)phosphonic acid diethyl ester (Compound VII R₃, R₄ = ethyl) (30 g), 1(S),3(R)-bis(*tert*-butyldimethylsilyloxy)-20(S)-formyl-9,10-secopregna-5(*E*),7(*E*),10(19)-triene SO₂-adducts (compounds VIa and VIb / R₁, R₂ = *tert*-butyldimethylsilyl) (34.8 g) (compounds 14a and 14 b described in M. J. Calverley, Tetrahedron, Vol. 43, No. 20, pp. 4609-4619, 1987), toluene (350 ml), water (35 ml), tetrabutyl ammonium bromide (1.01 g), and sodium hydroxide solution 27.7% (35 ml) was stirred at 33°C for approximately 1.5 hour. When the reaction was judged to be complete as checked by HPLC [Column LiChrosorb Si 60 5 µm 250x4mm from Merck, 1.5 ml/min flow, detection with MS, hexane/ethylacetate 100:2 (v:v)], water was added (160 ml). The pH of the reaction mixture was adjusted to pH 8.5-9.5 by addition of phosphoric acid solution (ca. 20%) keeping the temperature between 20-25°C. The organic phase was separated followed by the addition of MTBE (90ml), water (600 ml), saturated aqueous sodium chloride (60 ml), and saturated aqueous sodium hydrogen carbonate (10 ml). The toluene phase was separated and the solvent removed *in vacuo* without heating (preferably below 30°C) to give the two epimeric SO₂-adducts VIIIa and VIIIb / R₁, R₂ = *tert*-butyldimethylsilyl as a solid mixture predominantly containing VIIIa as checked by TLC. The two epimeric SO₂-adducts VIIIa and VIIIb could be separated by chromatography. Crystalline VIIIa could be furthermore obtained by tituration of the solid mixture with methanol. ¹H NMR (CDCl₃) VIIIa/ R₁, R₂ = *tert*-butyldimethylsilyl = 6.73 (dd,1H), 6.14 (d,1H), 4.69 (d,1H), 4.62 (d,1H), 4.35 (s,1H), 4.17 (m,1H), 3.92 (d,1H), 3.58 (d,1H), 2.61 (m,1H), 2.29 (m,1H), 2.2 - 1.2 (m,16H), 1.11 (d,3H), 1.05 (m,2H), 0.90 (m,2H), 0.87 (s,9H), 0.85 (s,9H), 0.68 (s,3H), 0.06 (s,3H), 0.05 (s,3H), 0.04 (s,3H), 0.02 (s,3H) ppm.

### Example 8:

### 20(R),3(R)-(tert-butyldimethylsilyloxy)-20-(3'-cyclopropyl-3'-oxoprop-1'(E)-enyl)-9.10-secopregna-5(E),7(E),10(19)-triene SO₂-adducts

### Compound XVIa and XVIb (R₁ = tert-butyldimethylsilyl)

The same procedure as in Example 7 using 3(R)-(*tert*-butyldimethylsilyloxy)-20(S)-formyl-9,10-secopregna-5(*E*),7(*E*),10(19)-triene SO₂-adducts (mixture of the two epimeric SO₂-adducts XVa and compound XVb) as the starting material giving the two epimeric SO₂-adducts XVIa and XVIb / R₁ = *tert*-butyldimethylsilyl as a solid mixture predominantly containing XVIa as checked by TLC. The two epimeric SO₂-adducts XVIa and XVIb could be separated by chromatography. Crystalline XVIa could be furthermore obtained by tituration of the solid mixture with methanol. ¹³C-NMR (CDCl₃) (mixture of the two epimeric SO₂-adducts XVIa and XVIb / R₁ = *tert*-butyldimethylsilyl) 200.3, 151.6, 151.4, 149.8, 149.2, 130.5, 130.1, 128.3, 128.1, 126.6, 126.3, 110.5, 110.0, 67.4, 66.7, 66.6, 66.3, 58.0, 57.9, 55.8, 55.6, 55.3, 55.2, 46.3, 45.5, 39.9, 39.7, 34.4, 34.1, 33.9, 31.4, 30.8, 30.5, 29.6, 29.1, 27.3, 27.1, 26.7, 25.6, 25.1, 24.4, 24.1, 23.6, 23.2, 22.4, 21.9, 21.9, 19.4, 19.3, 18.6, 18.4, 17.9, 17.9, 13.9, 12.2, 11.9, 10.8, -5.0 ppm.

### Example 9:

### 20(R),3(R)-(tert-butyidimethylsilyloxyl-20-(3'-cyclopropyl-3'-oxoprop-1'(E)-enyl)-9,10-secopregna-5(E),7(E),10(19)-triene

### Compound XIVa (R₁= tert-butyidimethylsilyl)

A mixture of ETH655 (2-cyclopropyl-2-oxoethyl)phosphonic acid diethyl ester (compound VII / R₃, R₄ = ethyl) (22,4 g), 3(R)-(*tert*-butyldimethylsilyloxy)-20(S)-formyl-9,10-secopregna-5(*E*),7(*E*),10(19)-triene (compounds XIIIa / R₁ = *tert-*butyldimethylsilyl) (27 g) prepared according to M. J. Calverley, Tetrahedron, Vol. 43, No. 20, pp. 4609-4619, 1987, toluene (328 ml), water (35 ml), tetrabutyl ammonium bromide (0.93 g), and sodium hydroxide solution 27.7% (38 g) was stirred at 33°C for approximately 1 hour. When the reaction was judged to be complete as checked by HPLC [Column LiChrosorb Si 60 5 µm 250x4mm from Merck, 1.5 ml/min flow, detection at 270 nm, hexane/ethylacetate 100:2 (v:v)], water was added (150 ml). The pH of the reaction mixture was adjusted to pH 7.8 by addition of phosphoric acid solution (ca. 20%) keeping the temperature between 20-25°C. The organic phase was separated followed by the addition of water (2000 ml), saturated aqueous sodium chloride (36 ml), and saturated aqueous sodium hydrogen carbonate (6 ml). The organic solvents were removed *in vacuo.* ¹³C NMR (CDCl₃) (compound XIVa / R₁ = *tert*-butyldimethylsilyl): 200.3, 151.8, 149.8, 142.8, 136.4, 128.1, 119.7, 116.1, 107.4, 69.2, 56.1, 55.3, 45.9, 40.2, 40.0, 37.3, 35.0, 30.9, 28.7, 27.3, 25.7, 23.2, 22.0, 19.3, 18.5, 18.0, 12.2, 10.7, -4.9 ppm.

### Example 10:

### 1-Cyclopropyl-4-(4-triethylsilanyloxy-7a-methyl-octahydro-inden-1-yl)-pent-2-en-1-one

### Compound XX (R₅= triethylsilyl)

2-(7a-Methyl-4-triethylsilanyloxy-octahydro-inden-1-yl)-propionaldehyde IX (R₅ = triehtylsilyl), which was synthesised as described in Eur.J.Org.Chem. 2003, pp. 3889-3895, (2 g) was added to a mixture of Li-*tert*.-butoxide (0.6 g) and (2-cyclopropyl-2-oxoethyl)phosphonic acid diethyl ester (compound VII / R₃, R₄ = ethyl) (1.62 g) in THF (50ml) at -50 °C. After complete reaction the reaction was quenched with water (50 ml) and extracted with hexane (100 ml). The organic phase was filtered through silica gel and concentrated in vacuo to give compound XX (R₅= triethylsilyl) as an clear oil (2 g). ¹H-NMR (CDCL₃): 6.74 (dd,1H), 6.12 (d,1H), 4.03 (m,1H), 2.40 - 0.80 (m,21H), 1.06 (d,3H), 0.94 (t,9H), 0.54 (q,6H) ppm.

### Preparation 7:

### 1-Cyclopropyl-4-(4-triethylsilanyloxy-7a-methyl-octahydro-inden-1-yl)-pent-2-en-1-(S)-ol

### Compound XXIa (R₅ = triethylsilyl)

(1S,2R)-(-)-cis-1-amino-2-indanol (6.33 g, 0.87 eq.) was mixed with MTBE (100 ml) under a nitrogen atmosphere at 15-25°C followed by the addition of N,N-diethylaniline-borane (16.0 ml, 1.85 eq.) at that temperature. The mixture was stirred until no more evolution of hydrogen could be observed. 1-Cyclopropyl-4-(4-triethylsilanyloxy-7a-methyl-octahydro-inden-1-yl)-pent-2-en-1-one (compound XX / R₅ = triethylsilyl) from Example 10 (19.0 g) was dissolved in MTBE (80ml) at room temperature and then added dropwise to said mixture at 15-25°C over 2 hours. The mixture was stirred for ca. 10 minutes after complete addition and then quenched with saturated aqueous NaHCO₃ (100ml) and extracted with hexane (200ml). The organic phase was separated and washed with 1 M hydrochloric acid (4X120ml) at 0-10°C followed by washing with saturated aqueous NaHCO₃ (100ml) and water (50ml) giving the mixture of compound XXIa and XXIb (R₅ = triethylsilyl) in a molar ratio of 87:13 as checked by HPLC analysis. {Column LiChrosorb Si 60 5 µm 250X4mm from Merck 1ml/min flow, MS-detection, hexane/ethylacetate 90:10 (v:v): RT XXIa= ca. 9.9 min, RT XXIb= ca. 8.4 min}. ¹H-NMR (CDCl₃) XXIa / R₅ = triethylsilyl: 138.0, 128.3, 76.6, 69.1, 56.2, 41.9, 40.5, 39.0, 34.4, 30.1, 27.4, 22.8, 20.0, 17.5, 17.3, 13.5, 6.7, 4.7, ppm; XXIb / R₅ = triethylsilyl: 138.2, 128.4, 77.1, 69.2, 56.1, 53.0, 41.9, 40.5, 39.1, 34.4, 27.5, 22.8, 20.0, 17.5, 17.4, 13.5, 6.7, 4.8 ppm.

## Claims

1. A method of preparing a compound of general structure Va, Vb, VIIIa, VIIIb, XIVa, XIVb, XVIa, XVIb, or XX respectively, 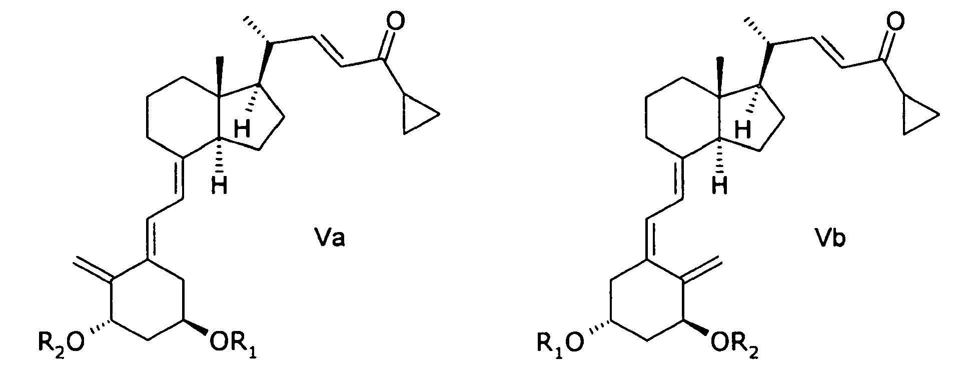 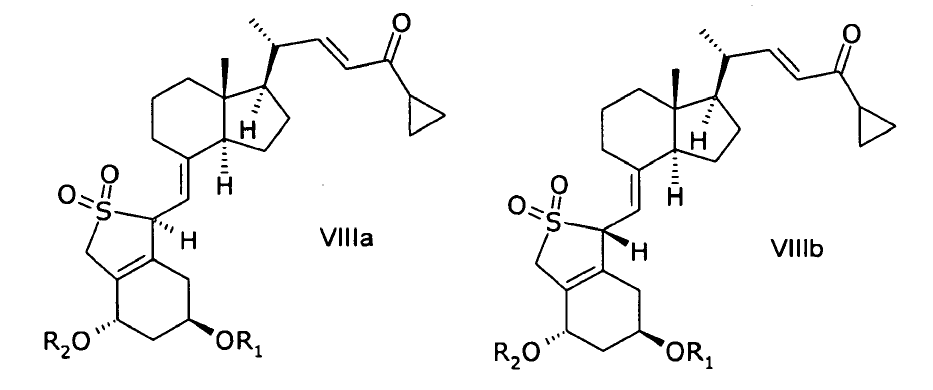 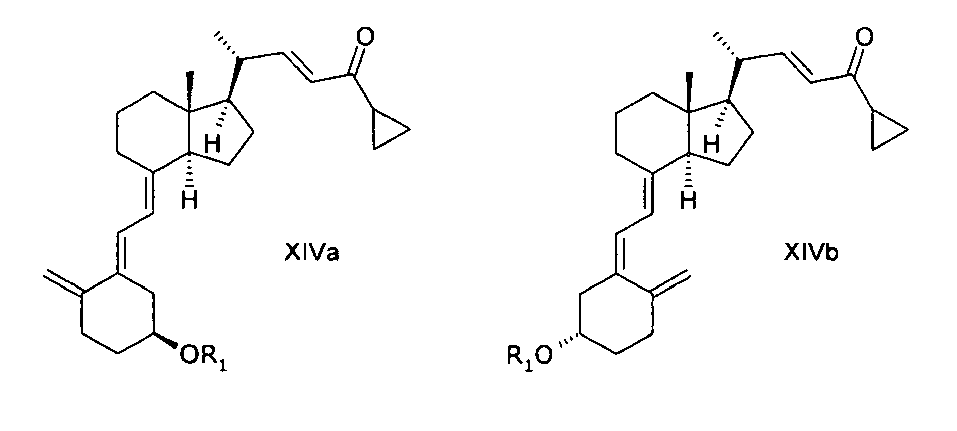 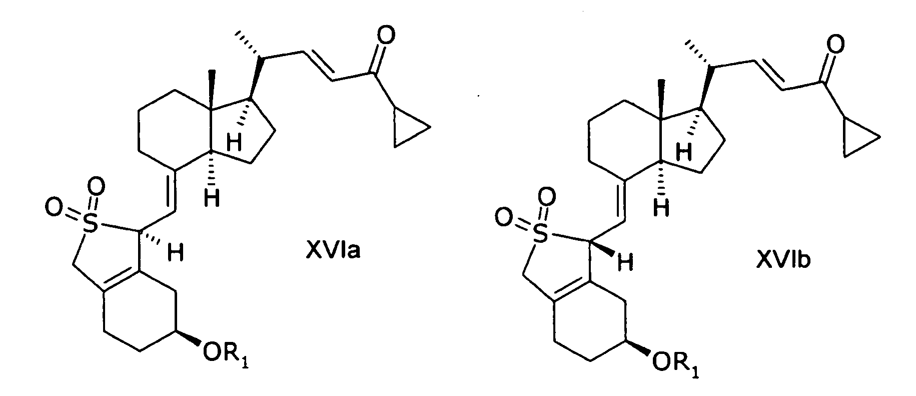 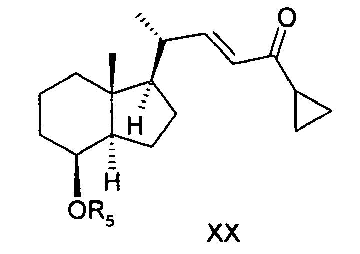 wherein R₁ and R₂ are the same or different and represent hydrogen or a hydroxy protecting group, and wherein R₅ represents hydrogen or a hydroxy protecting group; the method comprising reacting a compound of general structure IIIa, IIIb, VIa, VIb, XIIIa, XIIIb, XVa, or XVb, or IXX respectively, 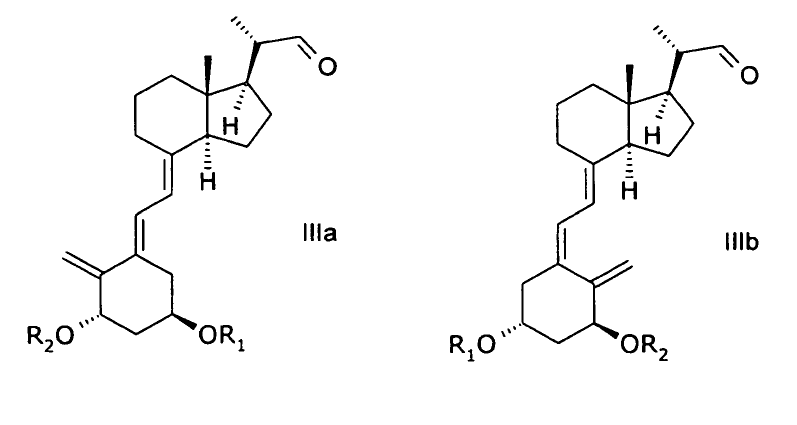 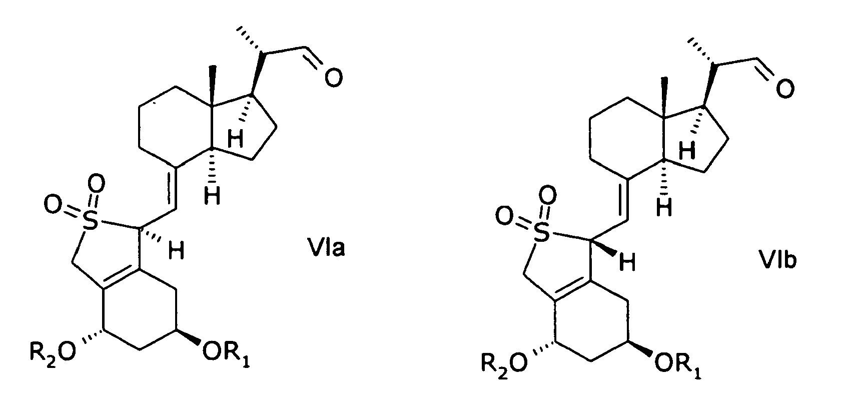 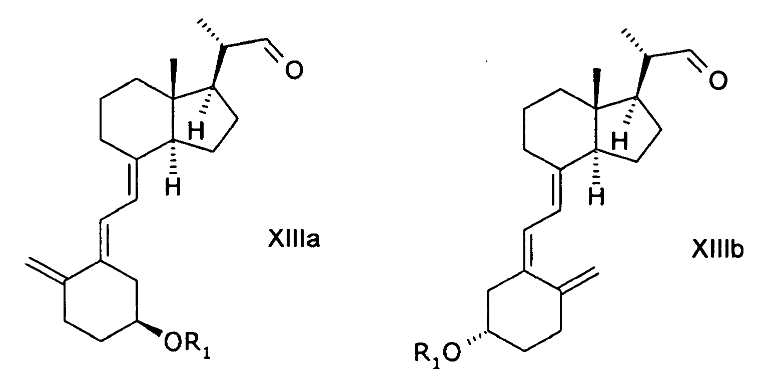 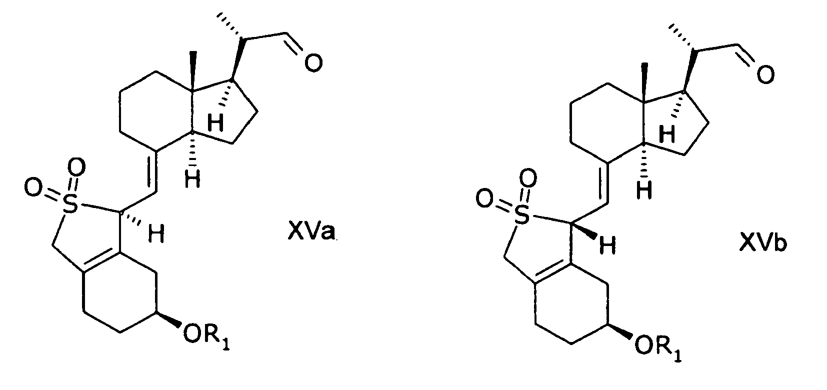 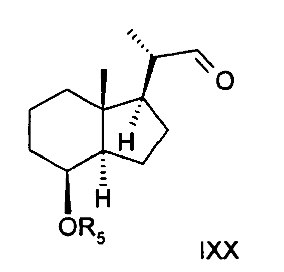 wherein R₁, R₂, and R₅ are as defined above; with a phosphonate of general structure VII, 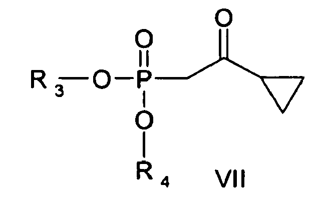 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base;
wherein the term "alkyl" stands for a linear or branched alkyl group, which may be cyclic or acyclic, having one to twenty carbon atoms;
the term "alkenyl" stands for a mono-, di-, tri-, tetra- or pentaunsaturated hydrocarbon radical comprising 2-10 carbon atoms;
the term "alkynyl" stands for a hydrocarbon radical comprising 1-5 triple C-C bonds and 2-20 carbon atoms;
the term "haloalkyl" stands for an alkyl group as defined above substituted with one or more halogen atoms;
the term "hydroxyalkyl" stands for an alkyl group as defined above substituted with one or more hydroxy groups;
the term "alkoxy" stands for a radical of the formula -OR', wherein R' is alkyl as indicated above;
the term "alkoxycarbonyl" stands for a radical of the formula -C(O)-O-R', wherein R' is alkyl as indicated above;
the term "alkylcarbonyloxy" stands for a radical of the formula -O-C(O)-R', wherein R' is alkyl as indicated above;
the term "cycloalkyl" stands for a saturated cycloalkane radical comprising 3-20 carbon atoms;
the term "cycloalkenyl" stands for mono-, di- tri- or tetraunsaturated non-aromatic cyclic hydrocarbon radicals, comprising 3-20 carbon atoms;
the term "aryl" stands for a radical of aromatic, optionally fused carbocyclic rings comprising 6-20 carbon atoms;
the term "aralkyl" stands for an alkyl group as defined above substituted with one or more aryl radicals as defined above;
the term "aralkenyl" stands for an alkenyl group as defined above substituted with one or more aryl radicals as defined above; and
the term "aralkynyl" stands for an alkynyl group as defined above substituted with one or more aryl radicals as defined above.

2. A method according to claim 1 of preparing a compound of general structure Va, 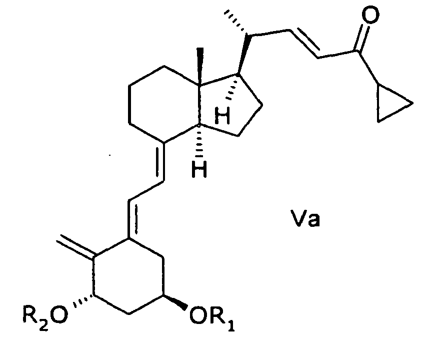 wherein R₁ and R₂ are the same or different and each represent hydrogen or a hydroxy protecting group,
the method comprising reacting a compound of general structure IIIa, 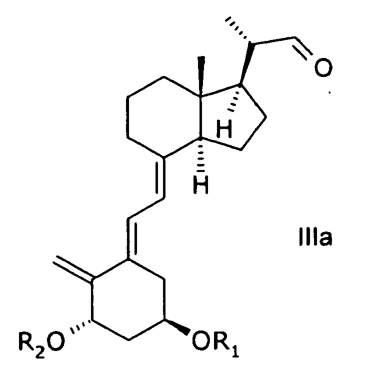 wherein R₁ and R₂ are as defined above,
with a phosphonate of general structure VII, 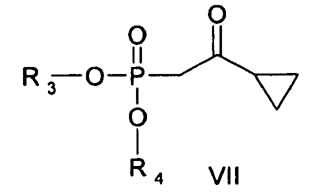 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

3. A method according to claim 1 of preparing a compound of general structure Vb, 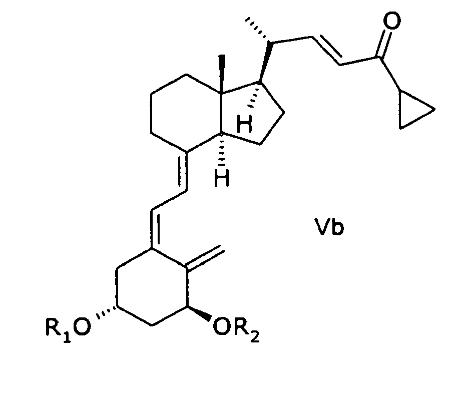 wherein R₁ and R₂ are the same or different and each represent hydrogen or a hydroxy protecting group,
the method comprising reacting a compound of general structure IIIb, 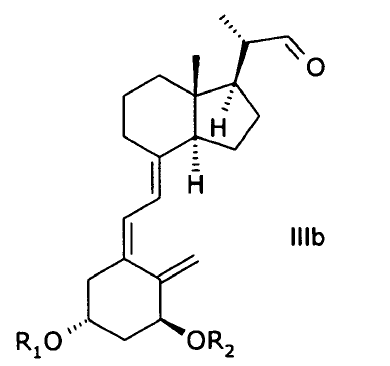 wherein R₁ and R₂ are as defined above,
with a phosphonate of general structure VII, 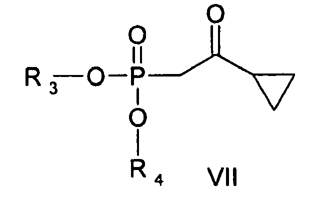 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

4. A method according to claim 1 of preparing a compound of general structure VIIIa or VIIIb respectively, 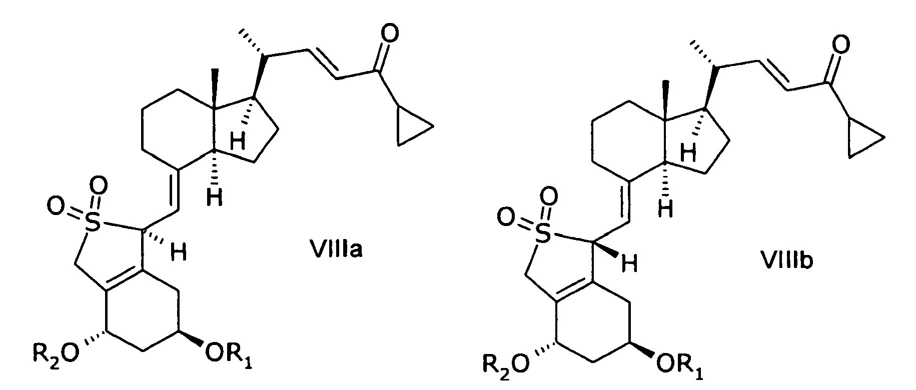 wherein R₁ and R₂ are the same or different and each represent hydrogen or a hydroxy protecting group,
the method comprising reacting a compound of general structure VIa or VIb respectively, 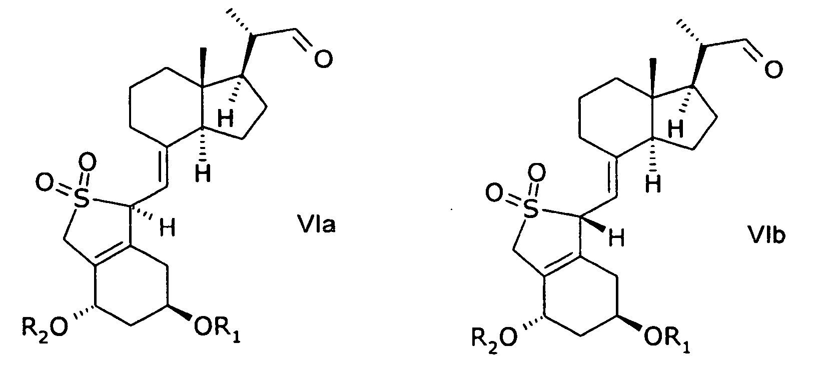 wherein R₁ and R₂ are as defined above,
with a phosphonate of general structure VII, 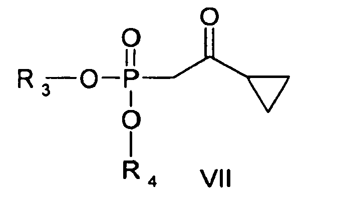 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

5. A method according to claim 1 of preparing a compound of general structure XIVa, 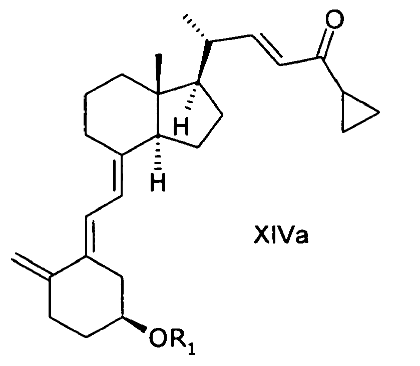 wherein R₁ represents hydrogen or a hydroxy protecting group,
the method comprising reacting a compound of general structure XIIIa, 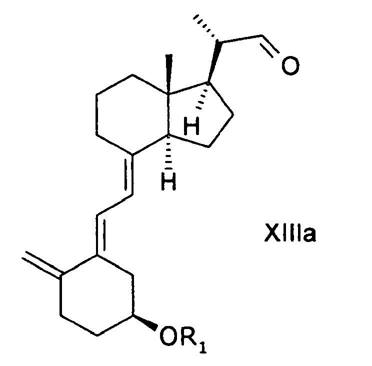 wherein R₁ is as defined above,
with a phosphonate of general structure VII, 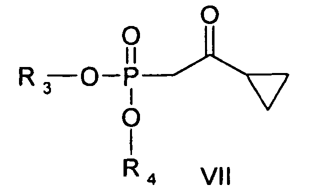 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

6. A method according to claim 1 of preparing a compound of general structure XIVb, 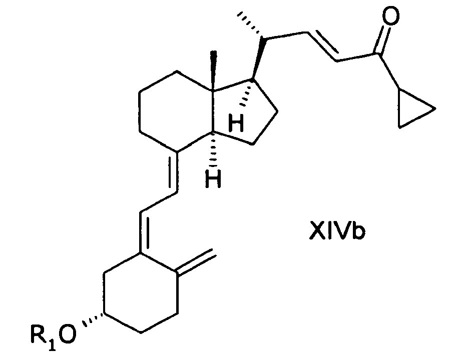 wherein R₁ represents hydrogen or a hydroxy protecting group,
the method comprising reacting a compound of general structure XIIIb, 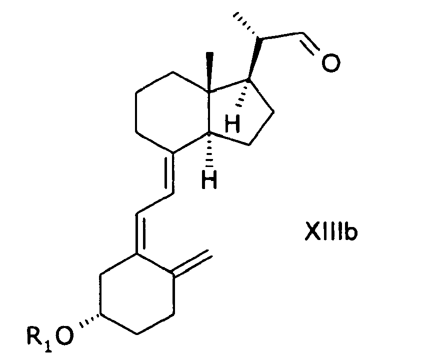 wherein R₁ is as defined above,
with a phosphonate of general structure VII, 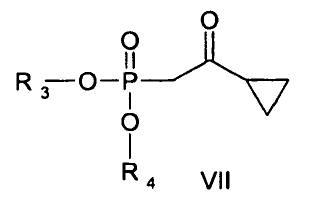 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

7. A method according to claim 1 of preparing a compound of general structure XVIa or XVIb respectively, 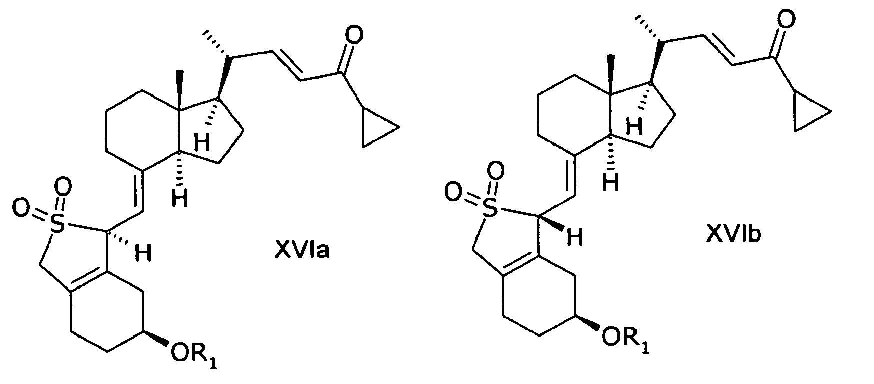 wherein R₁ represents hydrogen or a hydroxy protecting group,
the method comprising reacting a compound of general structure XVa or XVb respectively, 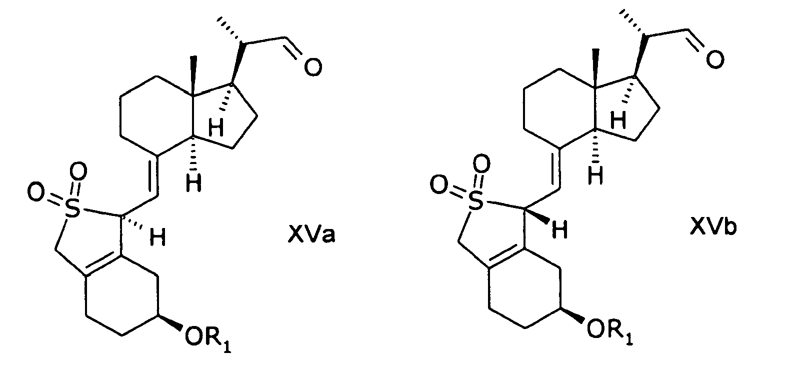 wherein R₁ is as defined above,
with a phosphonate of general structure VII, 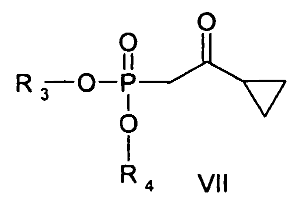 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

8. A method according to claim 1 of preparing a compound of general structure XX, 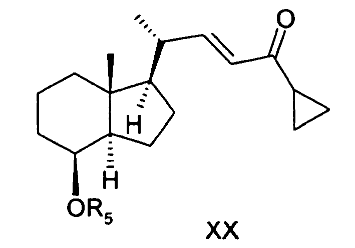 wherein R₅ represents hydrogen or a hydroxy protecting group,
the method comprising reacting a compound of general structure IXX, 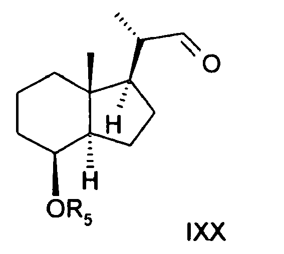 wherein R₅ is as defined above,
with a phosphonate of general structure VII, 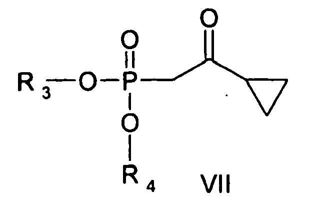 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base.

9. A method of preparing calcipotriol or calcipotriol monohydrate, the method comprising the method according to any one of claims 1, 2, 3, 4, 5, 6, 7, or 8.

10. A method for producing calcipotriol or calcipotriol monohydrate according to claim 9, the method comprising the steps of:
(i) reacting a compound of general structure IIIa, 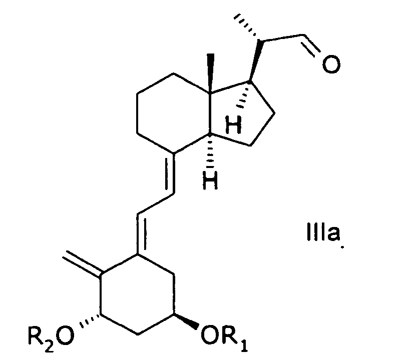 wherein R₁ and R₂ are the same or different and represent hydrogen or a hydroxy protecting group,
with a phosphonate of general structure VII, 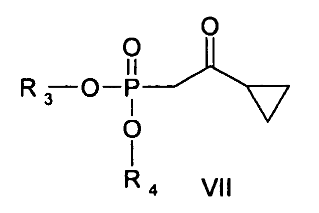 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure Va, 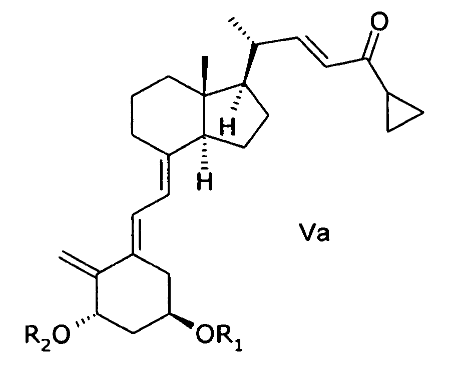 wherein R₁ and R₂ are as defined above;
(ii) reducing the compound of general structure Va with a suitable reducing agent, to give a compound of general structure IXa or a mixture of compounds of general structure IXa and IXb, 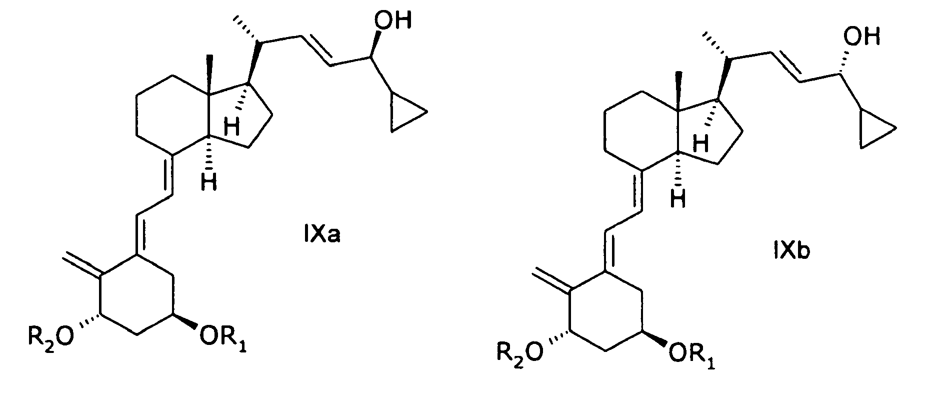 wherein R₁ and R₂ are as defined above;
(iii) optionally separating the compound of general structure IXa from the mixture of compounds of general structure IXa and IXb;
(iv) photoisomerising the compound of general structure IXa to the compound of general structure Xa, 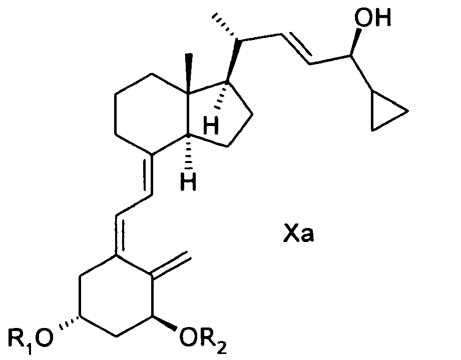 wherein R₁ and R₂ are as defined above;
(v) when R₁ and/or R₂ are not hydrogen, removing the hydroxy protecting group(s) R₁ and/or R₂ of the compound of general structure Xa to generate calcipotriol; and
(vi) optionally crystallising the calcipotriol from a mixture of an organic solvent and water to give calcipotriol monohydrate.

11. A method for producing calcipotriol or calcipotriol monohydrate according to claim 9, the method comprising the steps of:
(i) reacting a compound of general structure IIIb, 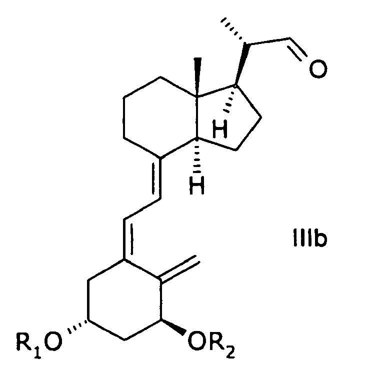 wherein R₁ and R₂ are the same or different and represent hydrogen or a hydroxy protecting group,
with a phosphonate of general structure VII, 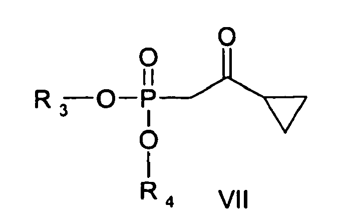 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure Vb, 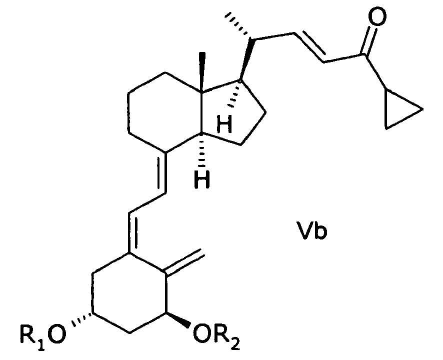 wherein R₁ and R₂ are as defined above;
(ii) reducing the compound of general structure Vb with a suitable reducing agent, to give a compound of general structure Xa or a mixture of compounds of general structure Xa and Xb, 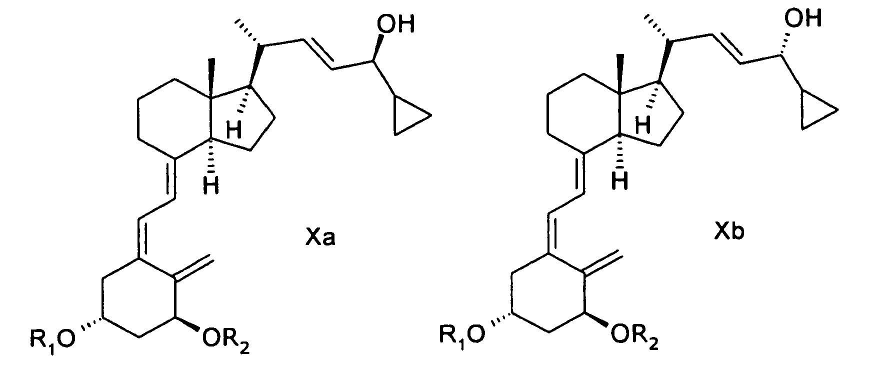 wherein R₁ and R₂ are as defined above;
(iii) optionally separating the compound of general structure Xa from the mixture of compounds of general structure Xa and Xb;
(iv) when R₁ and/or R₂ are not hydrogen, removing the hydroxy protecting group(s) R₁ and/or R₂ of the compound of general structure Xa to generate calcipotriol; and
(v) optionally crystallising the calcipotriol from a mixture of an organic solvent and water to give calcipotriol monohydrate.

12. A method for producing calcipotriol or calcipotriol monohydrate according to claim 9, the method comprising the steps of:
(i) reacting a compound of general structure VIa and/or VIb, 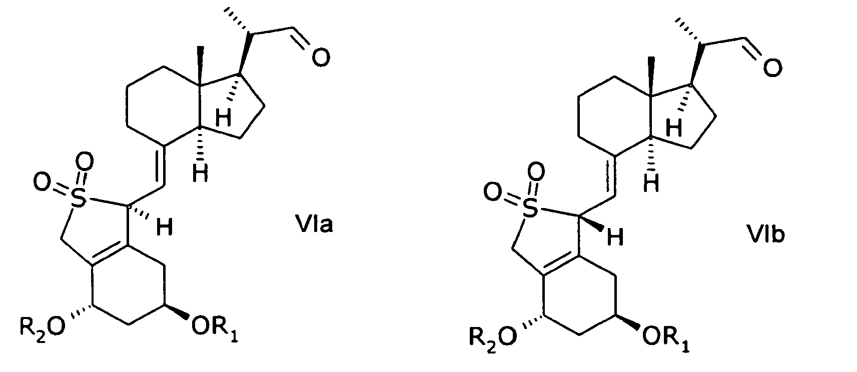 wherein R₁ and R₂ are the same or different and represent hydrogen or a hydroxy protecting group,
with a phosphonate of general structure VII, 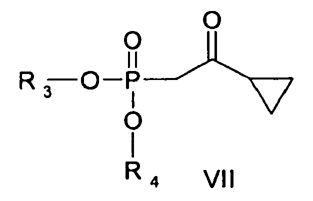 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure VIIIa and/or VIIIb, 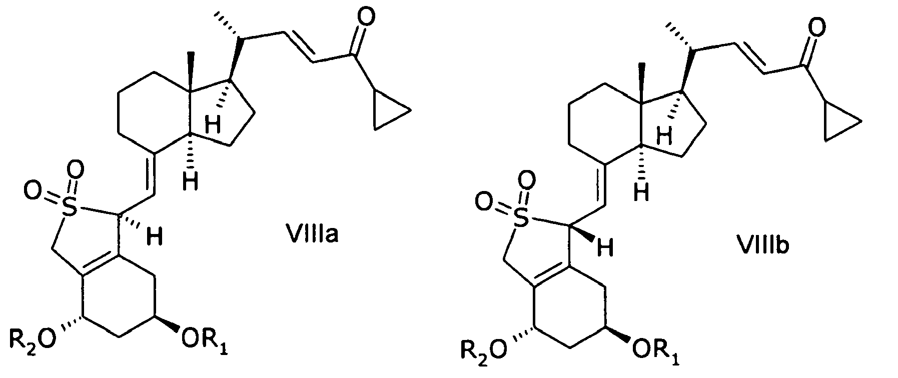 wherein R₁ and R₂ are as defined above;
(ii) heating the compounds of general structure VIIIa and/or VIIIb above 60°C in the presence of a base,
to give a compound of general structure Va, 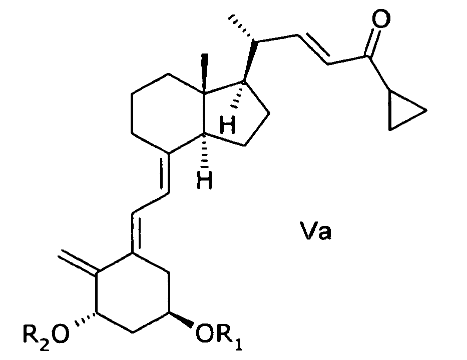 wherein R₁ and R₂ are as defined above;
(iii) reducing the compound of general structure Va with a suitable reducing agent, to give a compound of general structure IXa or a mixture of compounds of general structure IXa and IXb, 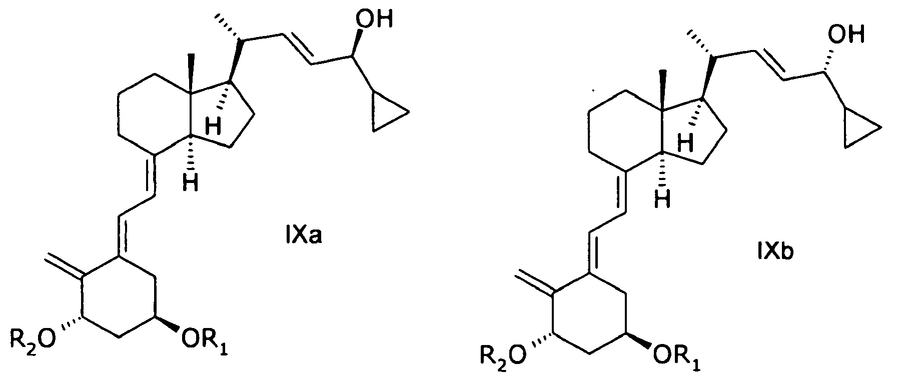 wherein R₁ and R₂ are as defined above;
(iv) optionally separating the compound of general structure IXa from the mixture of compounds of general structure IXa and IXb;
(v) photoisomerising the compound of general structure IXa to the compound of general structure Xa, 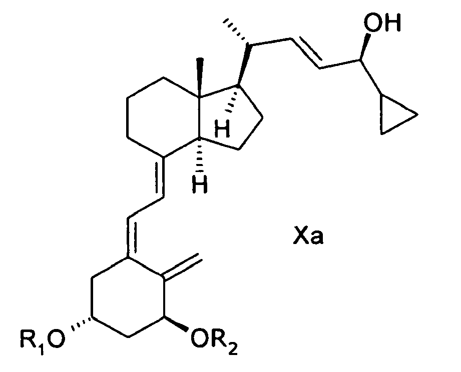 wherein R₁ and R₂ are as defined above;
(vi) when R₁ and/or R₂ are not hydrogen, removing the hydroxy protecting group(s) R₁ and/or R₂ of the compound of general structure Xa to generate calcipotriol; and
(vii) optionally crystallising the calcipotriol from a mixture of an organic solvent and water to give calcipotriol monohydrate.

13. A method for producing calcipotriol or calcipotriol monohydrate according to claim 9, the method comprising the steps of:
(i) reacting a compound of general structure VIa and/or VIb, 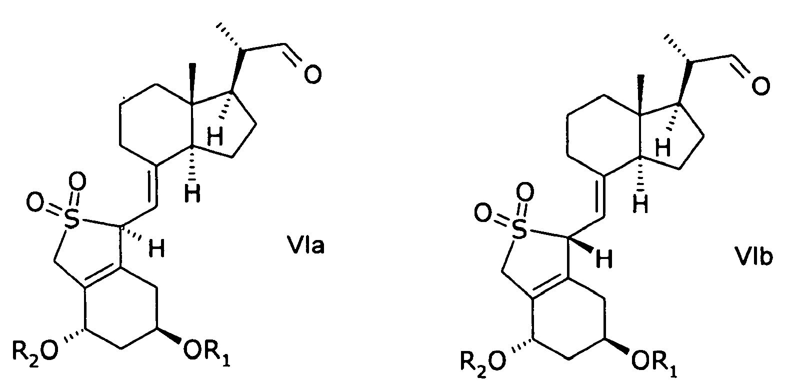 wherein R₁ and R₂ are the same or different and represent hydrogen or a hydroxy protecting group,
with a phosphonate of general structure VII, 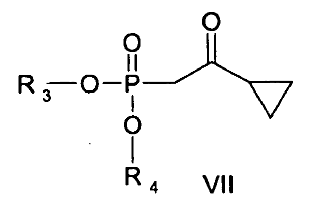 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure VIIIa and/or VIIIb, 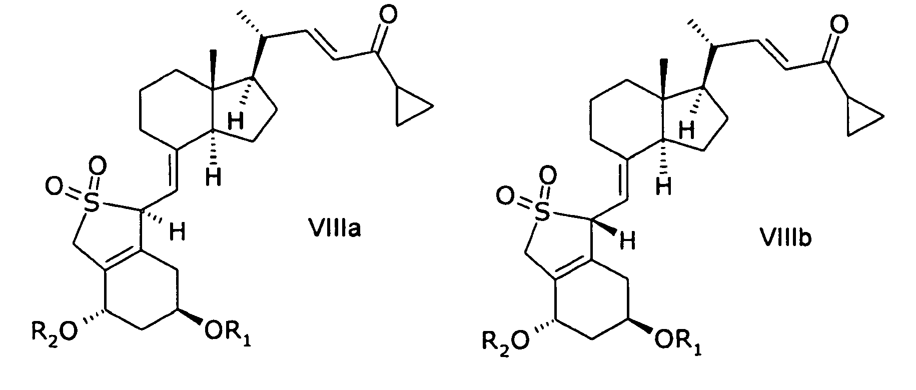 wherein R₁ and R₂ are as defined above;
(ii) reducing the compounds of general structure VIIIa and/or VIIIb, with a suitable reducing agent in an inert solvent,
to give compounds of general structure XIaa and/or XIba, or a mixture of compounds of general structure XIaa and/or XIba and XIab and/or XIbb, 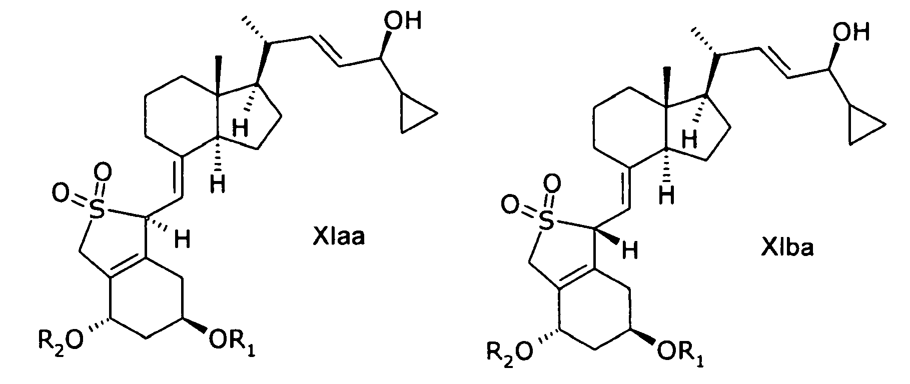 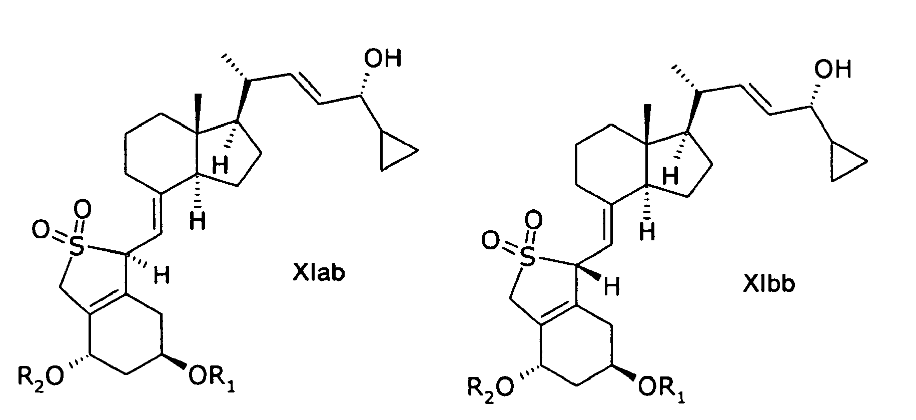 wherein R₁ and R₂ are as defined above;
(iii) optionally separating the compounds of general structure XIaa and/or XIba from the reaction mixture;
(iv) heating the compounds of general structure XIaa and/or XIba above 60°C in the presence of a base,
to give a compound of general structure IXa, 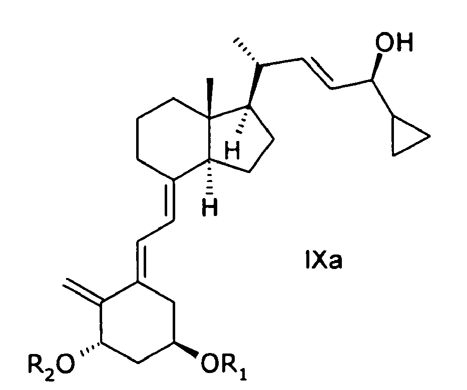 wherein R₁ and R₂ are as defined above;
(v) optionally separating the compound of general IXa from the reaction mixture;
(vi) photoisomerising the compound of general structure IXa to the compound of general structure Xa, 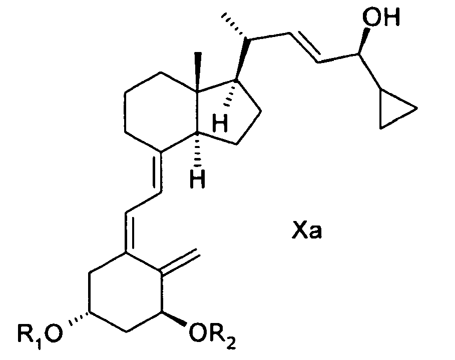 wherein R₁ and R₂ are as defined above;
(vii) when R₁ and/or R₂ are not hydrogen, removing the hydroxy protecting group(s) R₁ and/or R₂ of the compound of general structure Xa to generate calcipotriol; and
(viii) optionally crystallising the calcipotriol from a mixture of an organic solvent and water to give calcipotriol monohydrate;
wherein steps (vi) and (vii) may be in reversed order.

14. A method for producing calcipotriol or calcipotriol monohydrate according to claim 9, the method comprising the steps of:
(i) reacting a compound of general structure XIIIa, 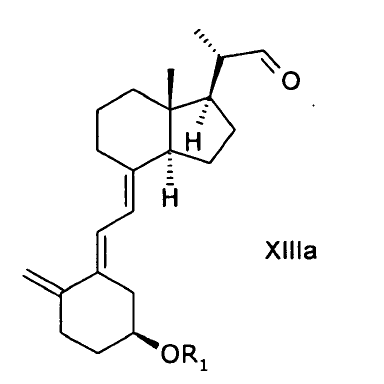 wherein R₁ represents hydrogen or a hydroxy protecting group,
with a phosphonate of general structure VII, 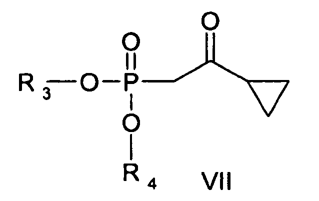 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure XIVa, 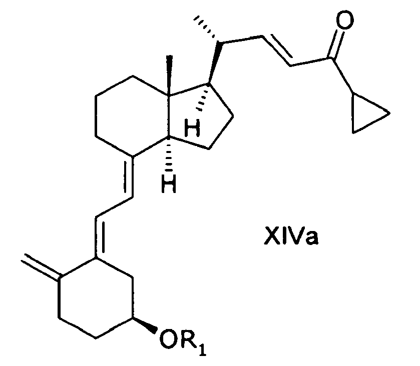 wherein R₁ is as defined above;
(ii) hydroxylating the compound of general structure XIVa with suitable hydroxylating agent,
to give a compound of general structure Va, 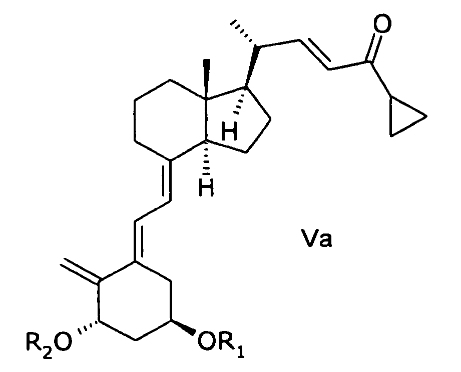 wherein R₁ represents hydrogen or a hydroxy protecting group and R₂ is hydrogen;
(iii) optionally reacting the compound of general structure Va, wherein R₁ represents hydrogen or a hydroxy protecting group and R₂ is hydrogen with a suitable protecting agent,
to give a compound of general structure Va, wherein R₁ and R₂ are the same or different and represent a hydroxy protecting group;
(iv) reducing the compound of general structure Va with a suitable reducing agent,
to give a compound of general structure IXa or a mixture of compounds of general structure IXa and IXb, 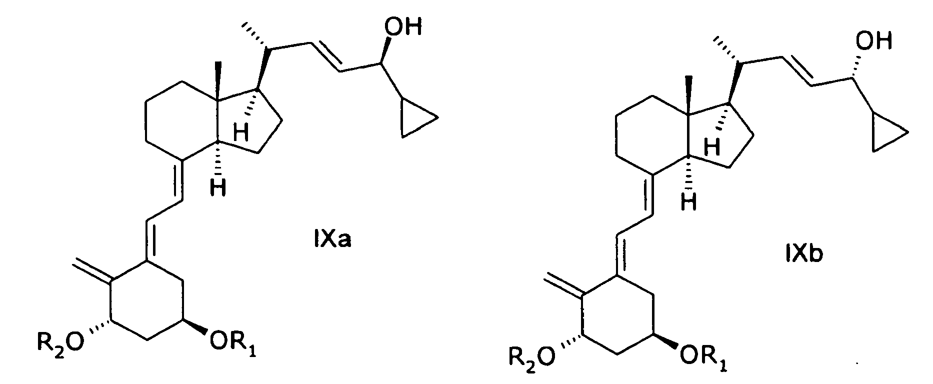 wherein R₁ and R₂ are as defined above;
(v) optionally separating the compound of general structure IXa from the mixture of compounds of general structure IXa and IXb;
(vi) photoisomerising the compound of general structure IXa to a compound of general structure Xa, 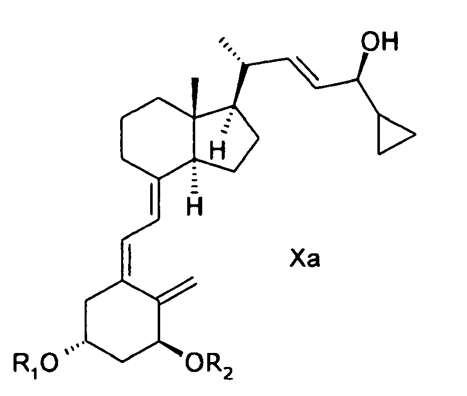 wherein R₁ and R₂ are as defined above;
(vii) when R₁ and/or R₂ are not hydrogen, removing the hydroxy protecting group(s) R₁ and/or R₂ of the compound of general structure Xa to generate calcipotriol; and
(viii) optionally crystallising the calcipotriol from a mixture of an organic solvent and water to give calcipotriol monohydrate.

15. A method for producing calcipotriol or calcipotriol monohydrate according to claim 9, the method comprising the steps of:
(i) reacting a compound of general structure XIIIb, 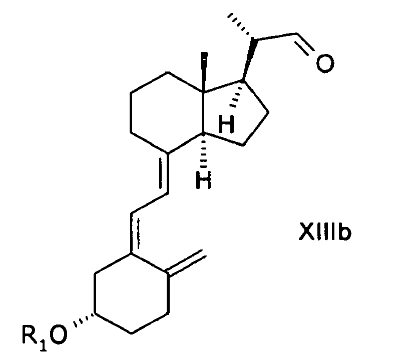 wherein R₁ represents hydrogen or a hydroxy protecting group, with a phosphonate of general structure VII, 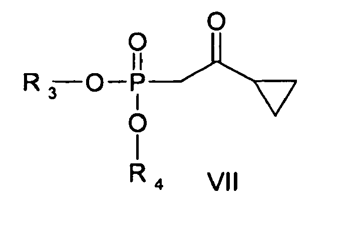 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure XIVb,
wherein R₁ is as defined above;
(ii) photoisomerising the compound of general structure XIVb to a compound of general structure XIVa, 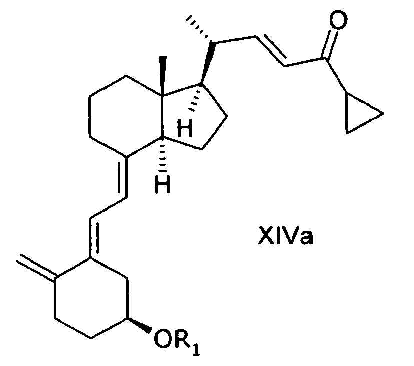 wherein R₁ is as defined above;
(iii) hydroxylating the compound of general structure XIVa with suitable hydroxylating agent,
to give a compound of general structure Va, 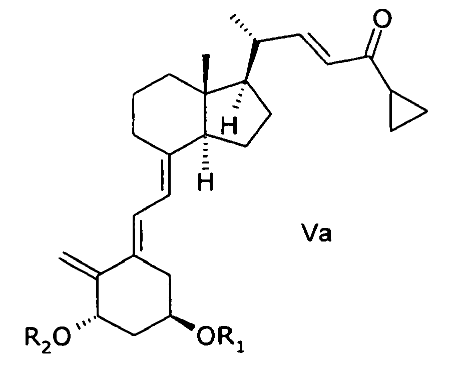 wherein R₁ represents hydrogen or a hydroxy protecting group and R₂ is hydrogen;
(iv) optionally reacting the compound of general structure Va, wherein R₁ represents hydrogen or a hydroxy protecting group and R₂ is hydrogen with a suitable protecting agent to give a compound of general structure Va, wherein R₁ and R₂ are the same or different and represent a hydroxy protecting group;
(v) reducing the compound of general structure Va with a suitable reducing agent, to give a compound of general structure IXa or a mixture of compounds of general structure IXa and IXb, 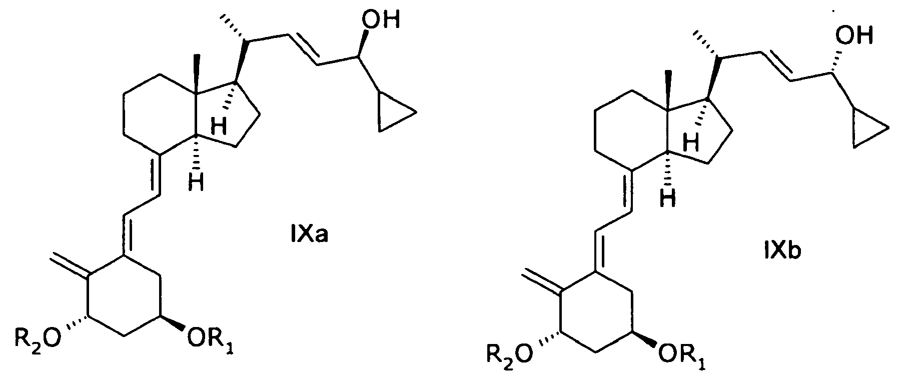 wherein R₁ and R₂ are as defined above;
(vi) optionally separating the compound of general structure IXa from the mixture of compounds of general structure IXa and IXb;
(vii) photoisomerising the compound of general structure IXa to the compound of general structure Xa, 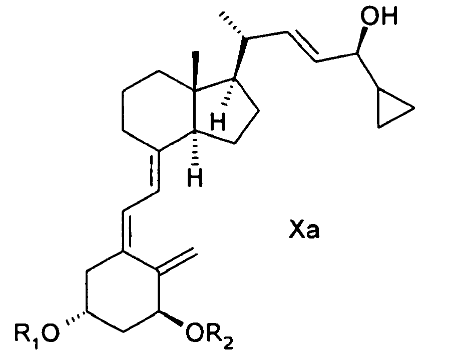 wherein R₁ and R₂ are as defined above;
(viii) when R₁ and/or R₂ are not hydrogen, removing the hydroxy protecting group(s) R₁ and/or R₂ of the compound of general structure Xa to generate calcipotriol; and
(ix) optionally crystallising the calcipotriol from a mixture of an organic solvent and water to give calcipotriol monohydrate.

16. A method for producing calcipotriol or calcipotriol monohydrate according to claim 9, the method comprising the steps of:
(i) reacting a compound of general structure XVa and/or XVb, 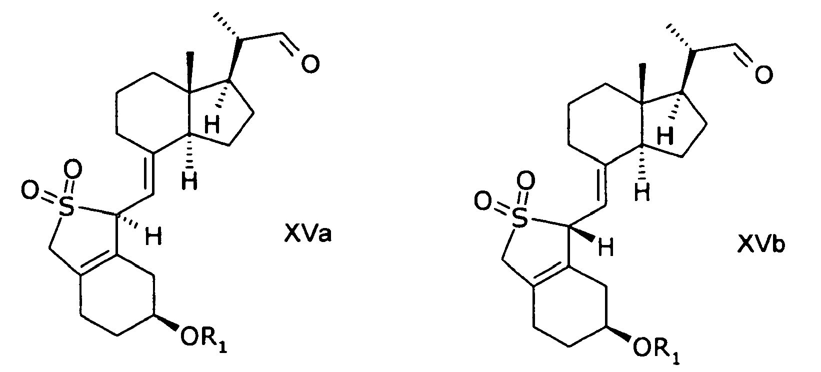 wherein R₁ represents a hydrogen or a hydroxy protecting group, with a phosphonate of general structure VII, 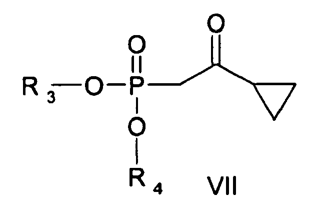 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure XVIa and/or XVIb, 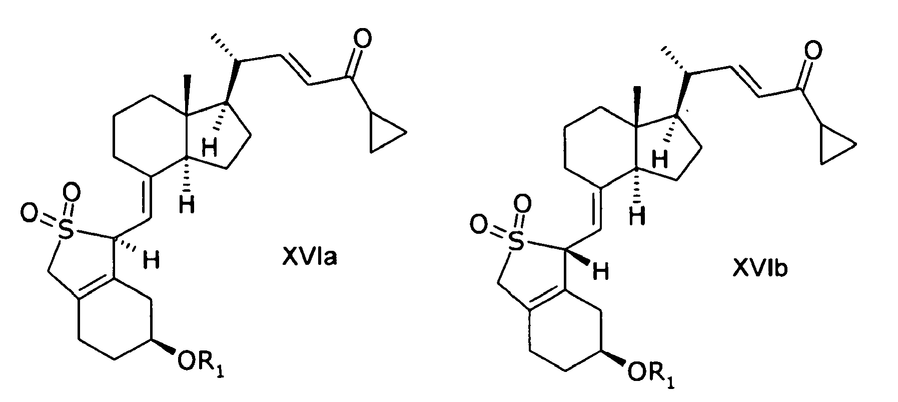 wherein R₁ is as defined above;
(ii) heating the compounds of general structure XVIa and/or XVIb above 60°C in the presence of a base,
to give a compound of general structure XIVa, 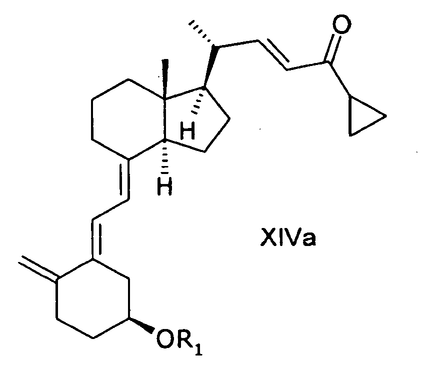 wherein R₁ is as defined above;
(iii) hydroxylating the compound of general structure XIVa with suitable hydroxylating agent,
to give a compound of general structure Va, 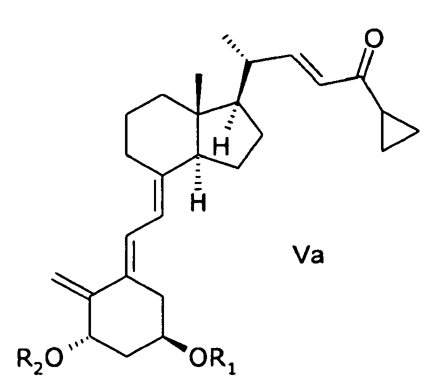 wherein R₁ represents hydrogen or a hydroxy protecting group and R₂ is hydrogen;
(iv) optionally reacting the compound of general structure Va, wherein R₁ represents hydrogen or a hydroxy protecting group and R₂ is hydrogen with a suitable protecting agent,
to give a compound of general structure Va, wherein R₁ and R₂ are the same or different and represent a hydroxy protecting group;
(v) reducing the compound of general structure Va with a suitable reducing agent, to give a compound of general structure IXa or a mixture of compounds of general structure IXa and IXb, 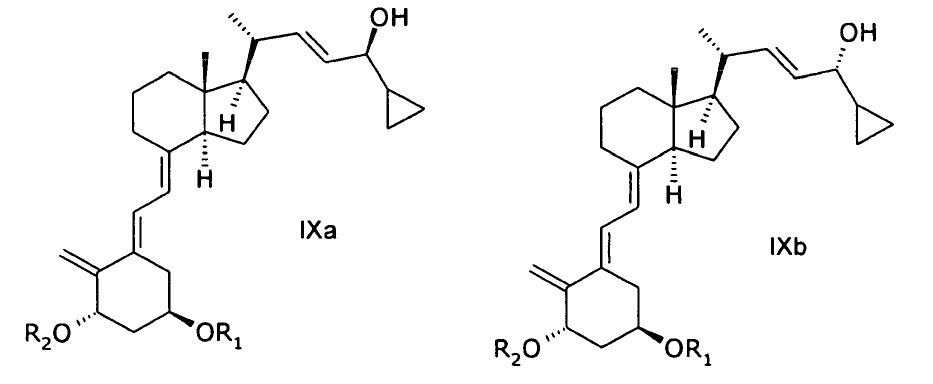 wherein R₁ and R₂ are as defined above;
(vi) optionally separating the compound of general structure IXa from the mixture of compounds of general structure IXa and IXb;
(vii) photoisomerising the compound of general structure IXa to the compound of general structure Xa, 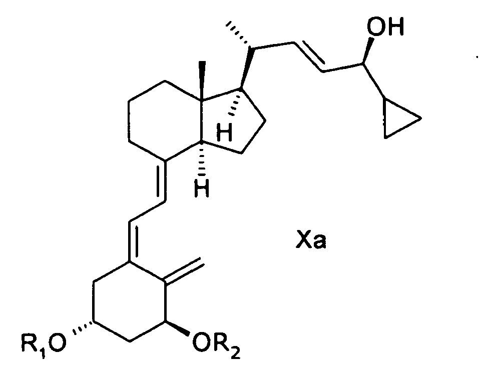 wherein R₁ and R₂ are as defined above;
(viii) when R₁ and/or R₂ are not hydrogen, removing the hydroxy protecting group(s) R₁ and/or R₂ of the compound of general structure Xa to generate calcipotriol; and
(ix) optionally crystallising the calcipotriol from a mixture of an organic solvent and water to give calcipotriol monohydrate.

17. A method for producing calcipotriol or calcipotriol monohydrate according to claim 9, the method comprising the steps of:
(i) reacting a compound of general structure IXX, 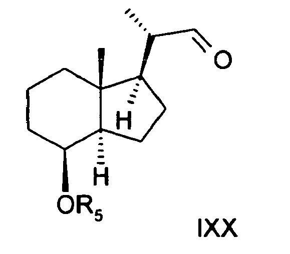 wherein R₅ represents hydrogen or a hydroxy protecting group, with a phosphonate of general structure VII, 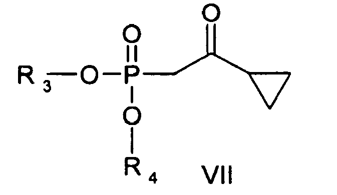 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy,
in the presence of a base,
to give a compound of general structure XX, 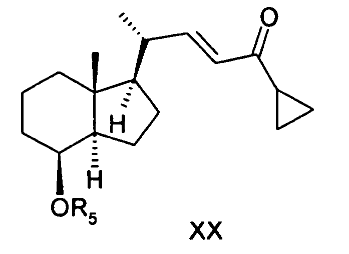 wherein R₅ is as defined above;
(ii) reducing the compound of general structure XX with a suitable reducing agent, to give a compound of general structure XXIa or a mixture of compounds of general structure XXIa and XXIb, 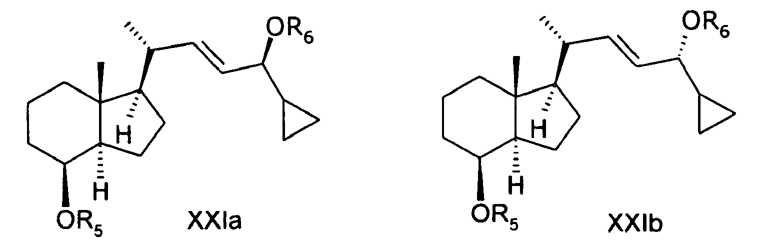 wherein R₅ is as defined above and R₆ is hydrogen;
(iii) optionally separating the compound of general structure XXIa from the mixture of compounds of general structure XXIa and XXIb;
(iv) protecting the allylic hydroxy group of the compound of general structure XXIa with a suitable hydroxy protecting reagent,
to give a compound of general structure XXIa, wherein R₆ is a hydroxy protecting group and R₅ is as defined above;
(v) when R₅ is not hydrogen, removing the hydroxy protecting group R₅ of the compound of general structure XXIa to give a compound of general structure XXIa, wherein R₅ is hydrogen;
(vi) oxidising the hydroxy group of the compound of general structure XXIa with a suitable oxidising agent to give a compound of general structure XXII, 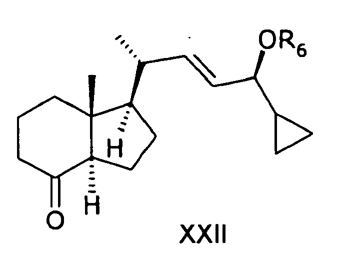 wherein R₆ is as defined above;
(vii) coupling of the compound of general structure XXII with a Wittig reagent XXIIIa or a Wittig Horner reagent XXIIIb, 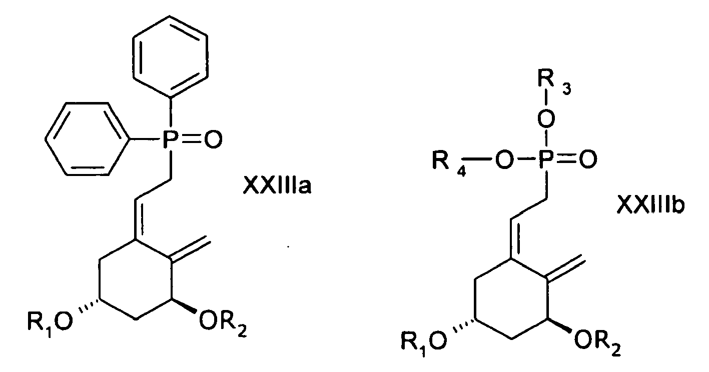 wherein R₁ and R₂ represent a hydrogen or a hydroxy protecting group, and wherein R₃ and R₄ are as defined above;
in the presence of a base,
to give a compound of general structure XXIVa, 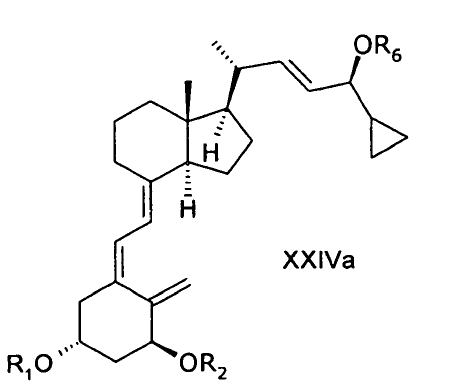 wherein R₁ and R₂ are the same or different and represent hydrogen or a hydroxy protecting group, and wherein R₆ is as defined above;
(viii) when R₆ is not hydrogen, removing the hydroxy protecting group R₆ of the compound of general structure XXIVa;
(ix) optionally separating the compound of general structure XXIVa;
(x) when R₁ and R₂ are not hydrogen, removing the hydroxy protecting group(s) R₁ and R₂ of the compound of general structure XXIVa to generate calcipotriol;
and
(xi) optionally crystallising the calcipotriol from a mixture of an organic solvent and water to give calcipotriol monohydrate.

18. The method according to any one of claims 1-17, wherein R₃ and R₄ are (C₁-C₆)alkyl.

19. The method according to any one of claims 1-17, wherein R₃ and R₄ are methyl or ethyl.

20. The method according to any one of claims 1-19, wherein R₁ and R₂ represent hydrogen or alkylsilyl.

21. The method according to any one of claims 1-19, wherein R₁ and R₂ represent hydrogen or *tert*-butyldimethylsilyl.

22. The method according to claim 8 or 19, wherein R₅ represents triethylsilyl and R₃ and R₄ are methyl or ethyl.

23. The method according to any one of claims 1-22, wherein the reaction with the phosphonate of general structure VII is carried out under phase-transfer conditions.

24. The method according to any one of claims 1-22, wherein the reaction with the phosphonate of general structure VII is carried out under phase-transfer conditions in a mixture of toluene or xylene and water with a tetraalkylammonium halide or a tetraalkylammonium hydrogensulfate as the phase transfer catalyst and with an alkalimetal hydroxide and/or a tetraalkylammoniumhydroxide as the base.

25. The method according to any one of claims 1-24, wherein the reaction with the phosphonate of general structure VII is carried out at a temperature between 10°C-50°C.

26. A compound of general structure VII, 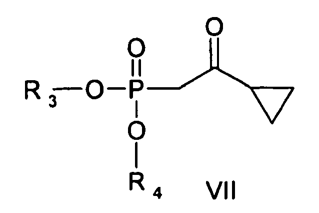 wherein R₃ and R₄ are the same or different and represent alkyl, haloalkyl, hydroxyalkyl, alkenyl, alkynyl, aralkyl, aralkenyl, aralkynyl, or aryl, each being optionally substituted with one or more substituents selected form the group consisting of alkyl, aralkyl, cycloalkyl, cycloalkenyl, haloalkyl, hydroxyalkyl, alkenyl, alkynyl, aralkyl, aralkenyl, aralkynyl, aryl, oxo, alkoxycarbonyl, alkylcarbonyloxy, halogen, alkoxy, carboxy, sulfo or hydroxy, provided that that the compound is not (2-cyclopropyl-2-oxoethyl)phosphonic acid diethyl ester, wherein the terms "alkyl", "alkenyl", "alkynyl", "haloalkyl", hydroxyalkyl", "alkoxy", "alkoxycarbonyl", "alkylcarbonyloxy", "cycloalkyl", "cycloalkenyl", "aryl", "aralkyl" "aralkenyl" and "aralkynyl" are as defined in claim 1.

## Patentansprüche

1. Verfahren zur Herstellung einer Verbindung der allgemeinen Struktur Va, Vb, VIIIa, VIIIb, XIVa, XIVb, XVIa, XVIb bzw. XX, 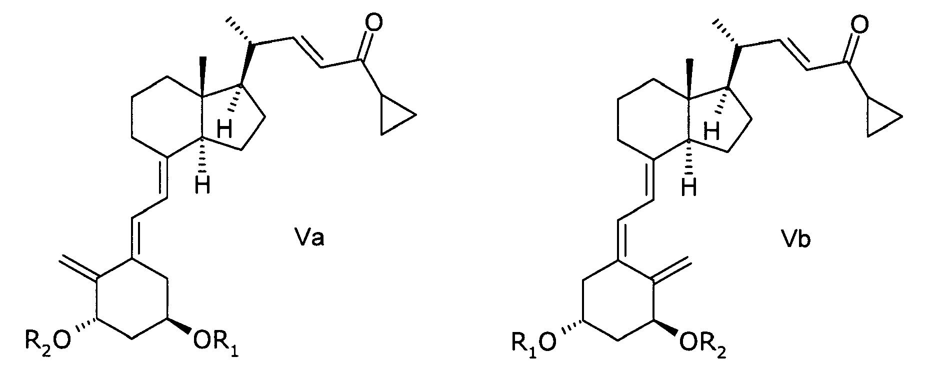 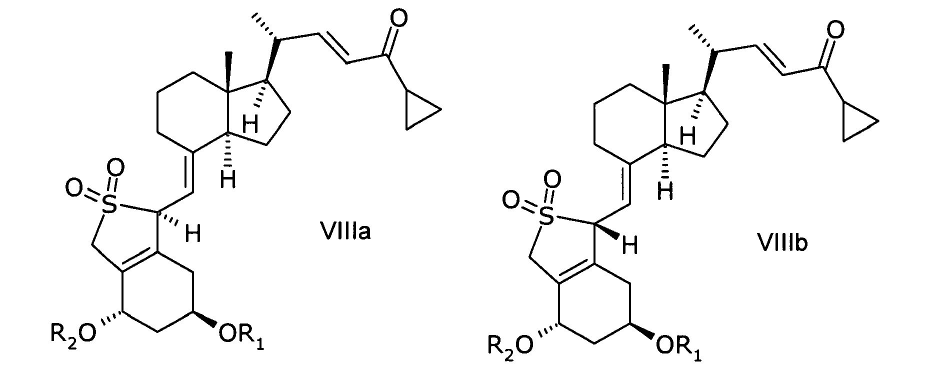 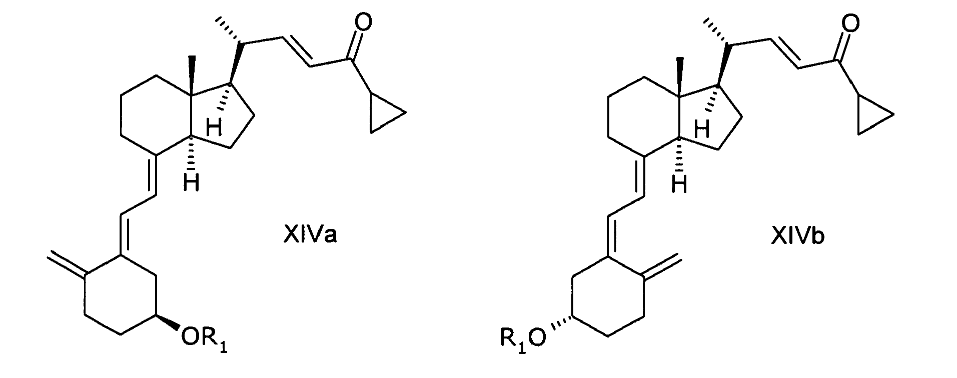 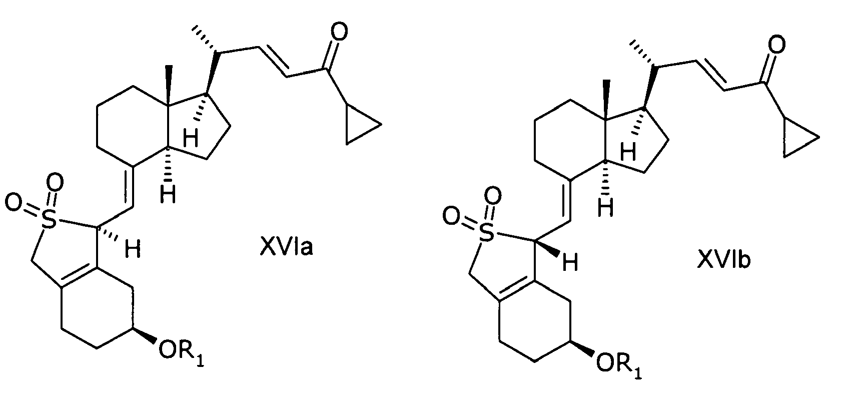 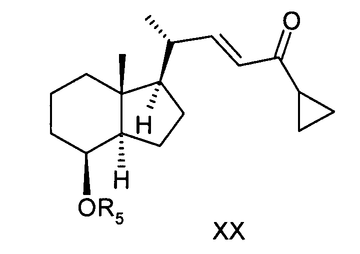 worin R₁ und R₂ gleich oder verschieden sind und jeweils für Wasserstoff oder eine Hydroxyschutzgruppe stehen, und worin R₅ für Wasserstoff oder eine Hydroxyschutzgruppe steht;
wobei das Verfahren es umfasst, dass man eine Verbindung der allgemeinen Struktur IIIa, IIIb, VIa, VIb, XIIIa, XIIIb, XVa, XVb bzw. IXX 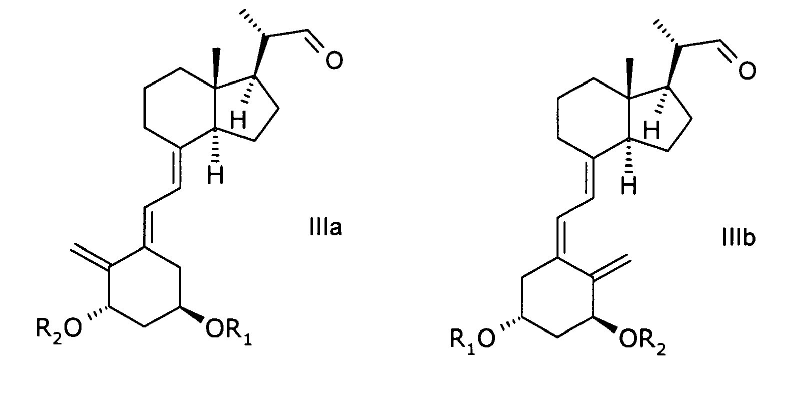 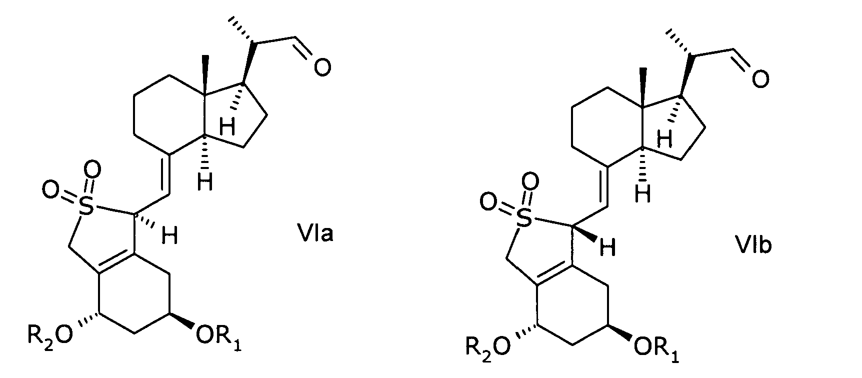 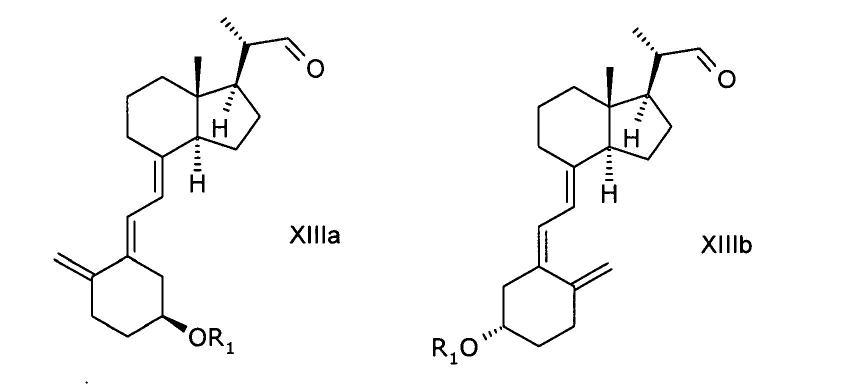 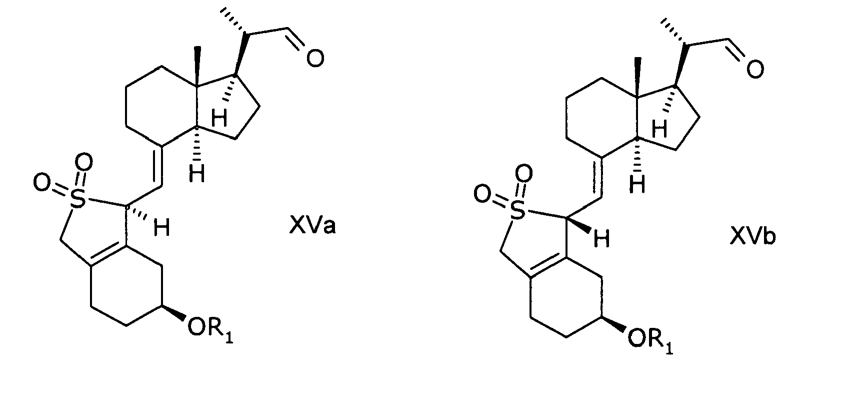 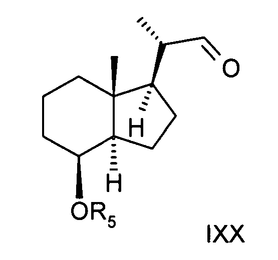 worin R_{1,} R₂ und R₅ wie oben definiert sind;
mit einem Phosphonat der allgemeinen Struktur VII, 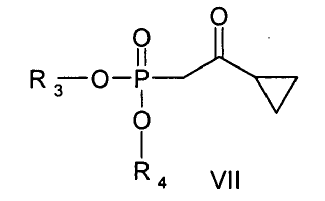 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt;
wobei der Begriff "Alkyl" für eine lineare oder verzweigte Alkylgruppe steht, die cyclisch oder acyclisch sein kann und ein bis zwanzig Kohlenstoffatome besitzt;
der Begriff "Alkenyl" für einen mono-, di-, tri-, tetra- oder penta-ungesättigten Kohlenwasserstoffrest steht, der 2-10 Kohlenstoffatome umfasst;
der Begriff "Alkinyl" für einen Kohlenwasserstoffrest steht, der 1-5 C-C-Dreifachbindungen und 2-20 Kohlenstoffatome umfasst;
der Begriff "Haloalkyl" für eine wie oben definierte Alkylgruppe steht, die mit einem oder mehreren Halogenatomen substituiert ist;
der Begriff "Hydroxyalkyl" für eine wie oben definierte Alkylgruppe steht, die mit einer oder mehreren Hydroxygruppen substituiert ist;
der Begriff "Alkoxy" für einen Rest der Formel -OR' steht, worin R' wie oben angegebenes Alkyl ist;
der Begriff "Alkoxycarbonyl" für einen Rest der Formel -C(O)-O-R' steht, worin R' wie oben angegebenes Alkyl ist;
der Begriff "Alkylcarbonyloxy" für einen Rest der Formel -O-C(O)-R' steht, worin R' wie oben angegebenes Alkyl ist;
der Begriff "Cycloalkyl" für einen gesättigten Cycloalkanrest steht, der 3-20 Kohlenstoffatome umfasst;
der Begriff "Cycloalkenyl" für mono-, di- tri- oder tetra-ungesättigte nichtaromatische cyclische Kohlenwasserstoffreste steht, die 3-20 Kohlenstoffatome umfassen;
der Begriff "Aryl" für einen Rest eines aromatischen, gegebenenfalls kondensierten carbocyclischen Rings steht, der 6-20 Kohlenstoffatome umfasst;
der Begriff "Aralkyl" für eine wie oben definierte Alkylgruppe steht, die mit einem oder mehreren wie oben definierten Arylresten substituiert ist;
der Begriff "Aralkenyl" für eine wie oben definierte Alkenylgruppe steht, die mit einem oder mehreren wie oben definierten Arylradikalen substituiert ist; und
der Begriff "Aralkinyl" für eine wie oben definierte Alkinylgruppe steht, die mit einem oder mehreren wie oben definierten Arylradikalen substituiert ist.

2. Verfahren nach Anspruch 1 zur Herstellung einer Verbindung der allgemeinen Struktur
Va, 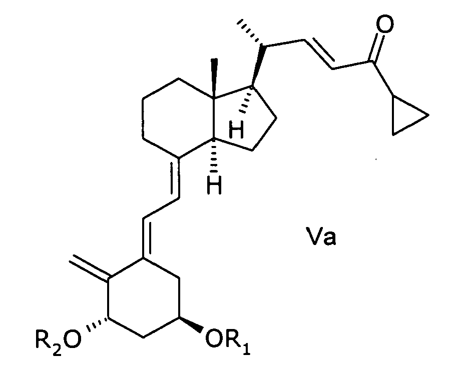 worin R₁ und R₂ gleich oder verschieden sind und jeweils für Wasserstoff oder eine Hydroxyschutzgruppe stehen,
wobei das Verfahren es umfasst, dass man eine Verbindung der allgemeinen Struktur IIIa 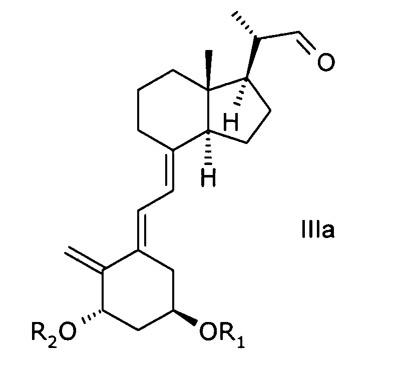 worin R₁ und R₂ wie oben definiert sind,
mit einem Phosphonat der allgemeinen Struktur VII, 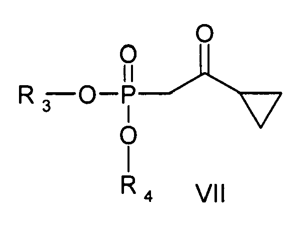 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

3. Verfahren nach Anspruch 1 zur Herstellung einer Verbindung der allgemeinen Struktur Vb, 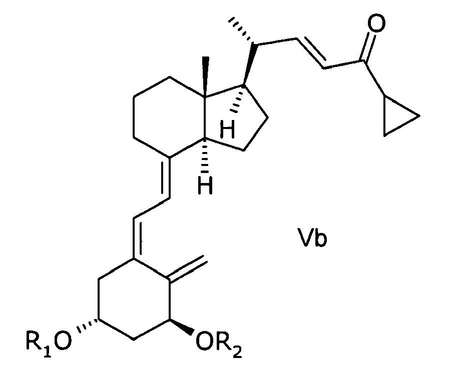 worin R₁ und R₂ gleich oder verschieden sind und jeweils für Wasserstoff oder eine Hydroxyschutzgruppe stehen,
wobei das Verfahren es umfasst, dass man eine Verbindung der allgemeinen Struktur IIIb, 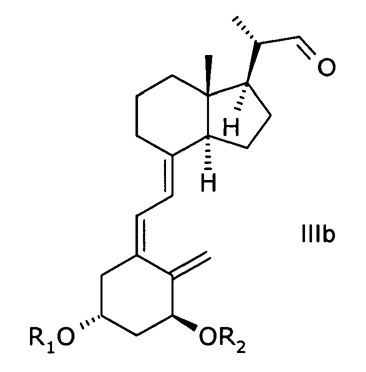 worin R₁ und R₂ wie oben definiert sind,
mit einem Phosphonat der allgemeinen Struktur VII, 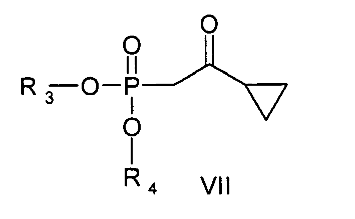 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

4. Verfahren nach Anspruch 1 zur Herstellung einer Verbindung der allgemeinen Formel
VIIIa bzw. Vlllb, 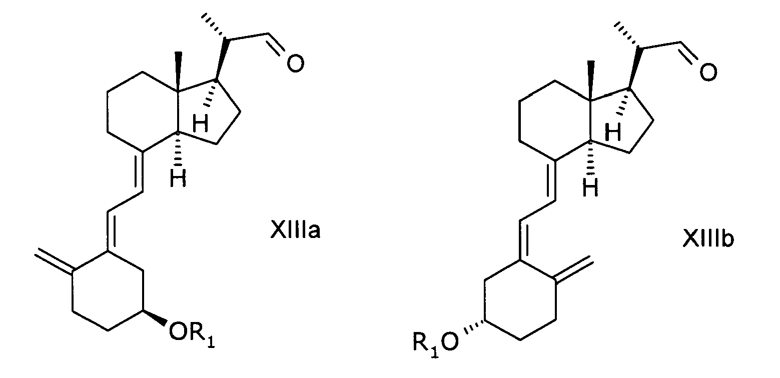 worin R₁ und R₂ gleich oder verschieden sind und jeweils für Wasserstoff oder eine Hydroxyschutzgruppe stehen,
wobei das Verfahren es umfasst, dass man eine Verbindung der allgemeinen Struktur VIa bzw. VIb, 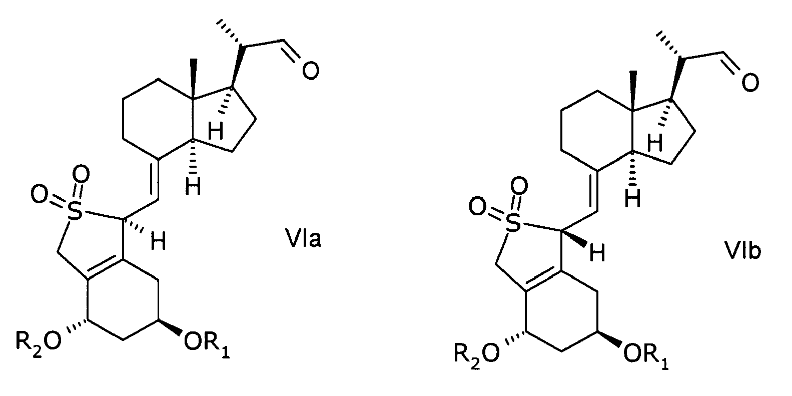 worin R₁ und R₂ wie oben definiert sind,
mit einem Phosphonat der allgemeinen Struktur VII, 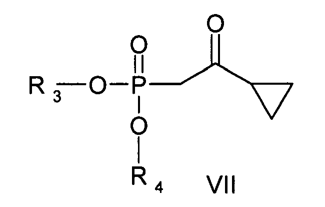 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

5. Verfahren nach Anspruch 1 zur Herstellung einer Verbindung der allgemeinen Struktur XIVa, 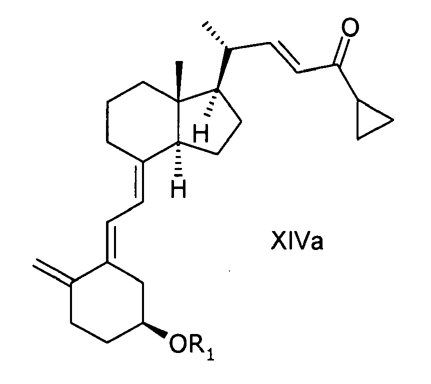 worin R₁ für Wasserstoff oder eine Hydroxyschutzgruppe steht,
wobei das Verfahren es umfasst, dass man eine Verbindung der allgemeinen Struktur XIIIa, 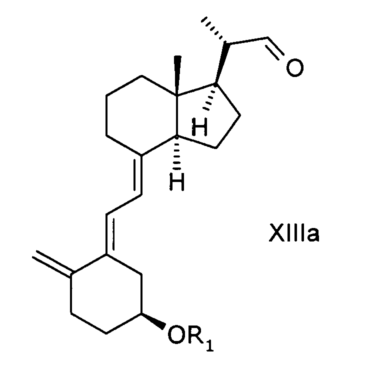 worin R₁ wie oben definiert ist,
mit einem Phosphonat der allgemeinen Formel VII, 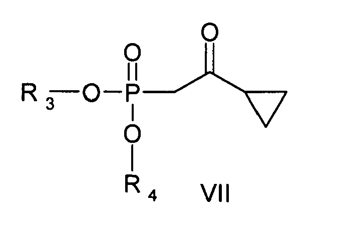 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

6. Verfahren nach Anspruch 1 zur Herstellung einer Verbindung der allgemeinen Struktur XIVB, 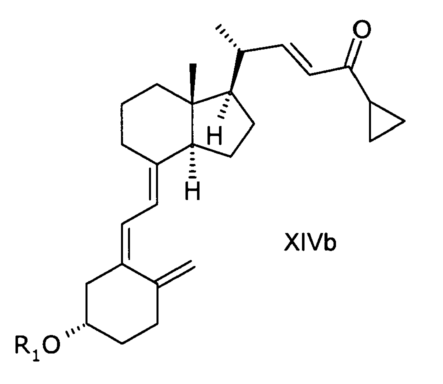 worin R₁ für Wasserstoff oder eine Hydroxyschutzgruppe steht,
wobei das Verfahren es umfasst, dass man eine Verbindung der allgemeinen Struktur Xlllb, 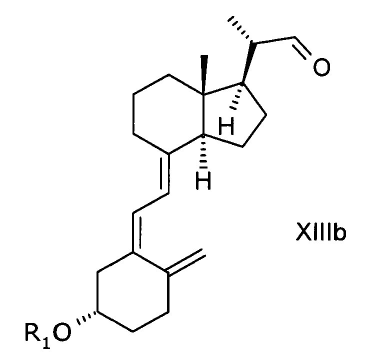 worin R₁ wie oben definiert ist,
mit einem Phosphonat der allgemeinen Formel VII, 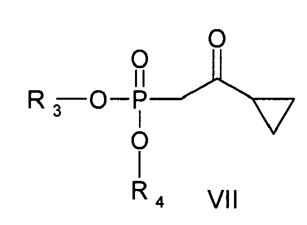 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

7. Verfahren nach Anspruch 1 zur Herstellung einer Verbindung der allgemeinen Struktur XVIa bzw. XVIb, 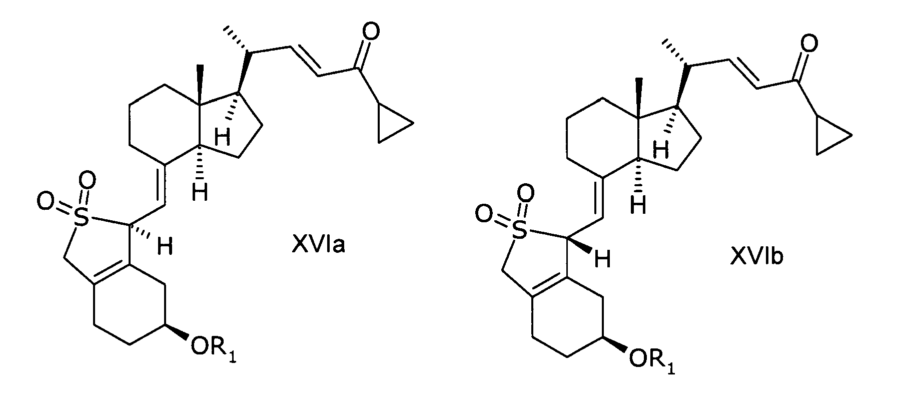 worin R₁ für Wasserstoff oder eine Hydroxyschutzgruppe steht,
wobei das Verfahren es umfasst, dass man eine Verbindung der allgemeinen Struktur XVa bzw. XVb, 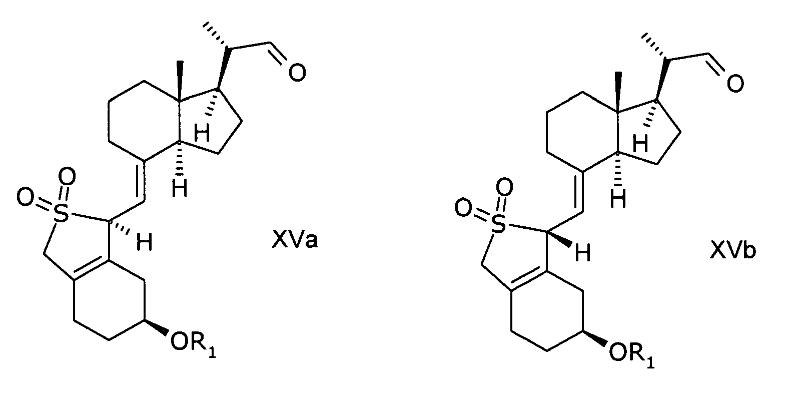 worin R₁ wie oben definiert ist,
mit einem Phosphonat der allgemeinen Struktur VII, 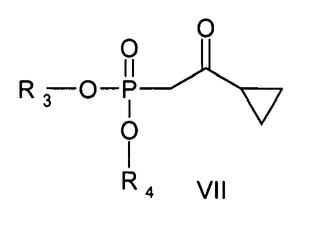 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

8. Verfahren nach Anspruch 1 zur Herstellung einer Verbindung der allgemeinen Struktur XX, 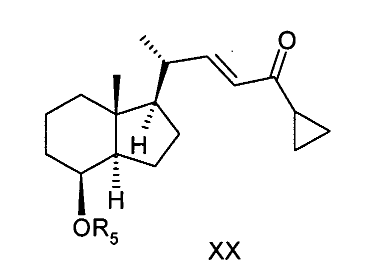 worin R₅ für Wasserstoff oder eine Hydroxyschutzgruppe steht,
wobei das Verfahren es umfasst, dass man eine Verbindung der allgemeinen Struktur IXX, 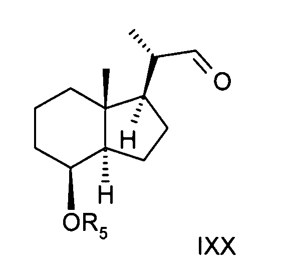 worin R₅ wie oben definiert ist,
mit einem Phosphonat der allgemeinen Struktur VII, 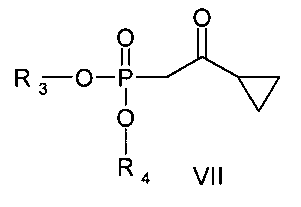 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base umsetzt.

9. Verfahren zur Herstellung von Calcipotriol oder Calcipotriol-Monohydrat, wobei das Verfahren das Verfahren nach einem der Ansprüche 1, 2, 3, 4, 5, 6, 7 oder 8 umfasst.

10. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur IIIa, 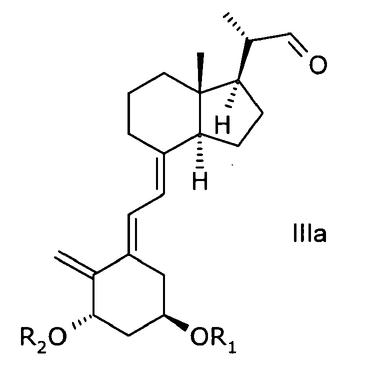 worin R₁ und R₂ gleich oder verschieden sind und für Wasserstoff oder eine Hydroxyschutzgruppe stehen,
mit einem Phosphonat der allgemeinen Struktur VII, 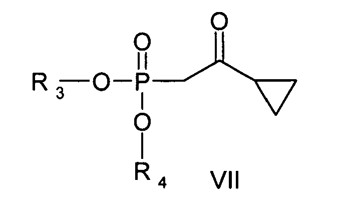 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur Va, 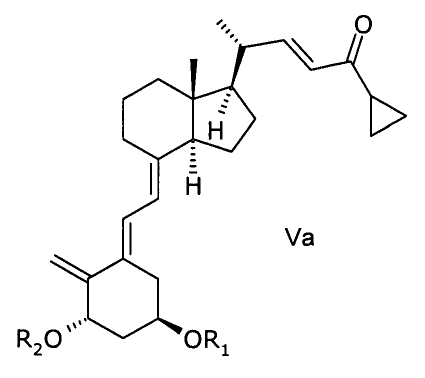 worin R₁ und R₂ wie oben definiert sind, ergibt;
(ii) Reduzieren der Verbindung der allgemeinen Struktur Va mit einem geeigneten Reduktionsmittel, was eine Verbindung der allgemeinen Struktur IXa oder ein Gemisch der Verbindungen der allgemeinen Struktur IXa und IXb, 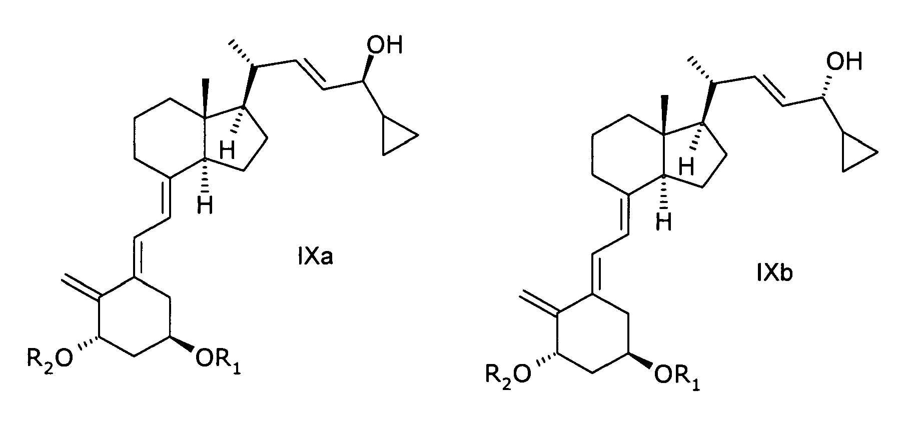 worin R₁ und R₂ wie oben definiert sind, ergibt;
(iii) gegebenenfalls Abtrennen der Verbindung der allgemeinen Struktur IXa aus dem Gemisch der Verbindungen der allgemeinen Struktur IXa und IXb;
(iv) Photoisomerisieren der Verbindung der allgemeinen Struktur IXa zur Verbindung der allgemeinen Struktur Xa, 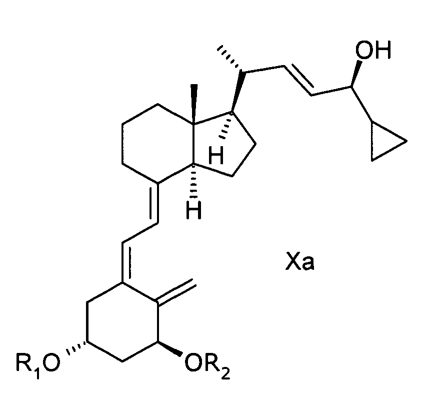 worin R₁ und R₂ wie oben definiert sind;
(v) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(vi) gegebenenfalls Kristallisieren des Calcipotriols aus einem Gemisch eines organischen Lösungsmittels und Wasser, was Calcipotriol-Monohydrat ergibt.

11. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur IIIb 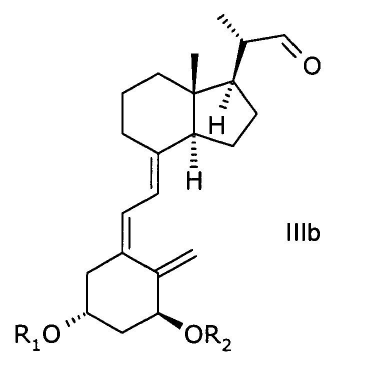 worin R₁ und R₂ gleich oder verschieden sind und für Wasserstoff oder eine Hydroxyschutzgruppe stehen,
mit einem Phosphonat der allgemeinen Struktur VII, 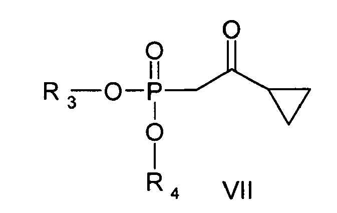 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur Vb, 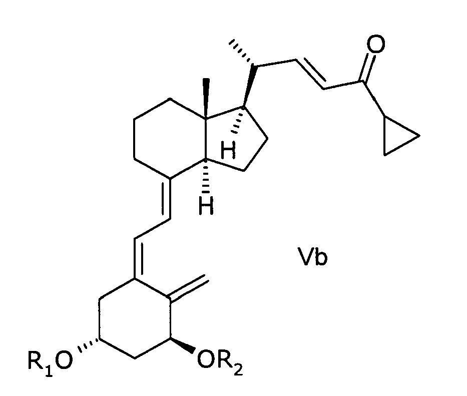 worin R₁ und R₂ wie oben definiert sind, ergibt;
(ii) Reduzieren der Verbindung der allgemeinen Struktur Vb mit einem geeigneten Reduktionsmittel, was eine Verbindung der allgemeinen Struktur Xa oder ein Gemisch der Verbindungen der allgemeinen Struktur Xa und Xb, 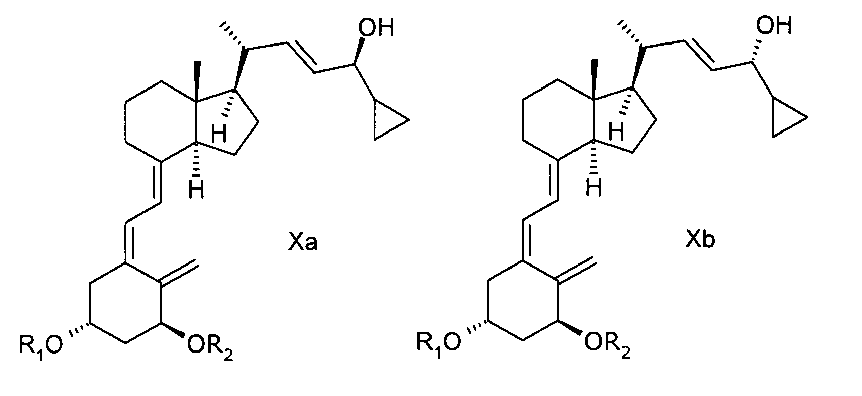 worin R₁ und R₂ wie oben definiert sind, ergibt;
(iii) gegebenenfalls Abtrennen der Verbindung der allgemeinen Struktur Xa aus dem Gemisch der Verbindungen der allgemeinen Struktur Xa und Xb;
(iv) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(v) gegebenenfalls Kristallisieren des Calcipotriols aus einem Gemisch eines organischen Lösungsmittels und Wasser, was Calcipotriol-Monohydrat ergibt.

12. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur VIa und/oder VIb, 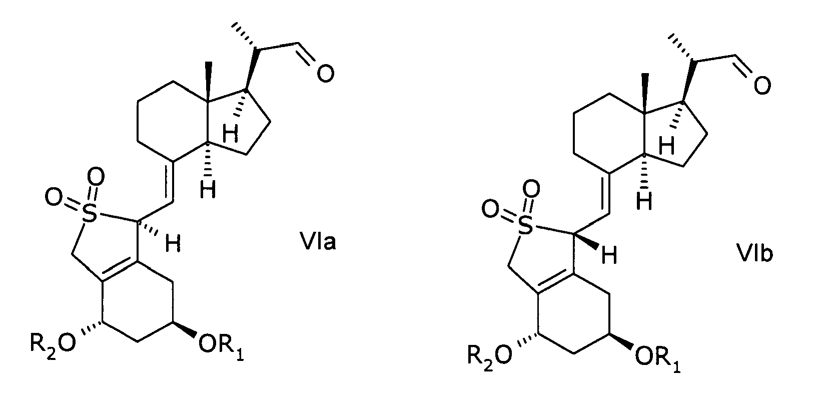 worin R₁ und R₂ gleich oder verschieden sind und für Wasserstoff oder eine Hydroxyschutzgruppe stehen,
mit einem Phosphonat der allgemeinen Struktur VII, 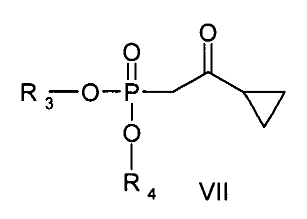 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur VIIIa und/oder Vlllb, 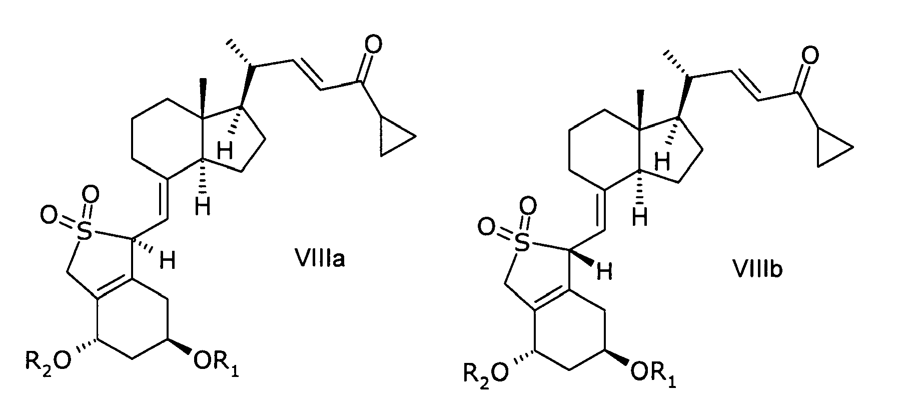 worin R₁ und R₂ wie oben definiert sind, ergibt;
(ii) Erwärmen der Verbindungen der allgemeinen Struktur VIIIa und/oder VIIIb oberhalb von 60 °C in Gegenwart einer Base,
was eine Verbindung der allgemeinen Struktur Va, 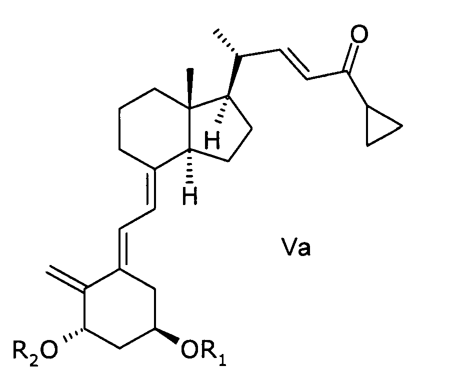 worin R₁ und R₂ wie oben definiert sind, ergibt;
(iii) Reduzieren der Verbindung der allgemeinen Struktur Va mit einem geeigneten Reduktionsmittel, was eine Verbindung der allgemeinen Struktur IXa oder ein Gemisch der Verbindungen der allgemeinen Struktur IXa und IXb, 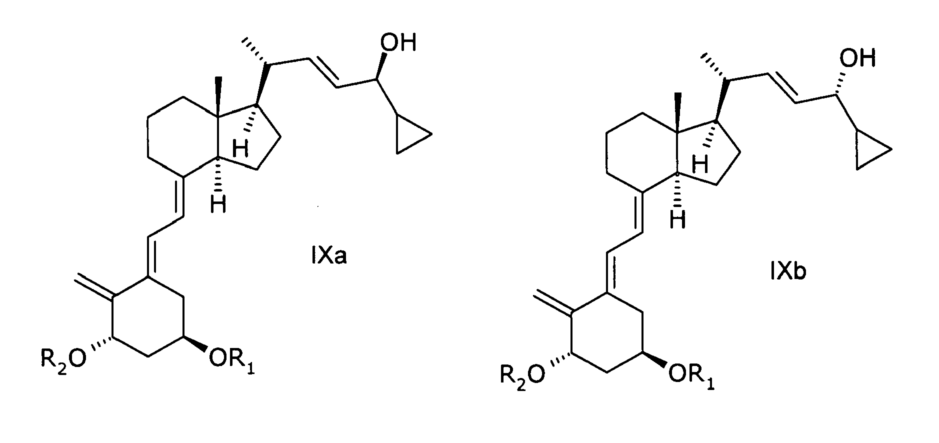 worin R₁ und R₂ wie oben definiert sind;
(iv) gegebenenfalls Abtrennen der Verbindung der allgemeinen Struktur IXa aus dem Gemisch der Verbindungen der allgemeinen Struktur IXa und IXb;
(v) Photoisomerisieren der Verbindung der allgemeinen Struktur IXa zur Verbindung der allgemeinen Struktur Xa, 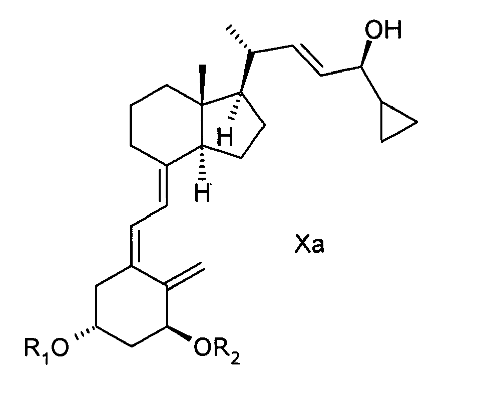 worin R₁ und R₂ wie oben definiert sind;
(vi) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(vii) gegebenenfalls Kristallisieren des Calcipotriols aus einem Gemisch eines organischen Lösungsmittels und Wasser, was Calcipotriol-Monohydrat ergibt.

13. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur VIa und/oder Vlb, 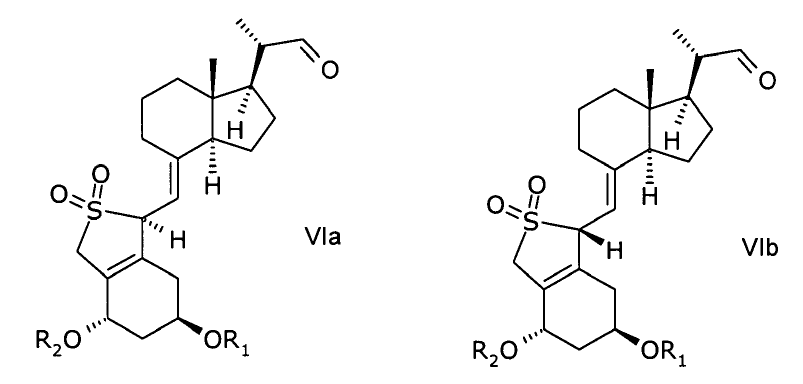 worin R₁ und R₂ gleich oder verschieden sind und für Wasserstoff oder eine Hydroxyschutzgruppe stehen,
mit einem Phosphonat der allgemeinen Struktur VII, 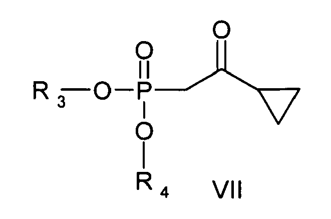 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur VIIIa und/oder VIIIb, 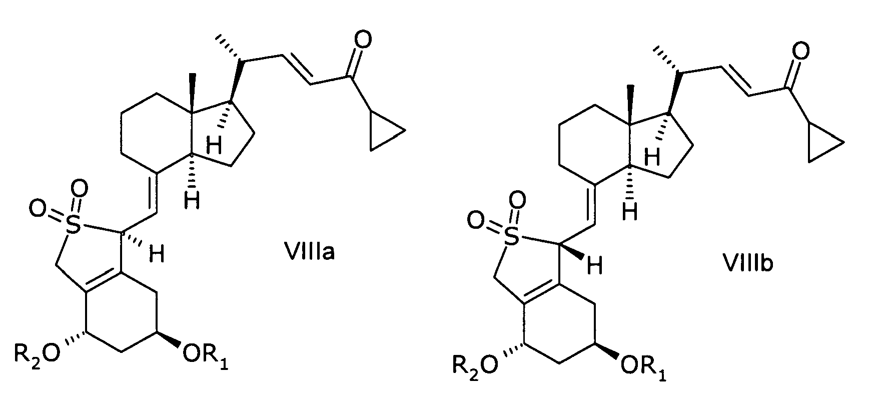 worin R₁ und R₂ wie oben definiert sind, ergibt;
(ii) Reduzieren der Verbindungen der allgemeinen Struktur VIIIa und/oder VIIIb mit einem geeigneten Reduktionsmittel in einem inerten Lösungsmittel, was eine Verbindung der allgemeinen Struktur Xlaa und/oder Xlba oder ein Gemisch der Verbindungen der allgemeinen Struktur Xlaa und/oder Xlba und XIab und/oder Xlbb, 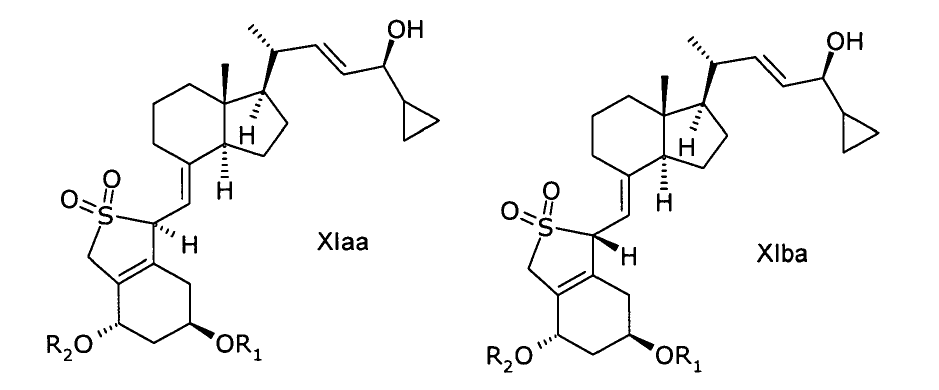 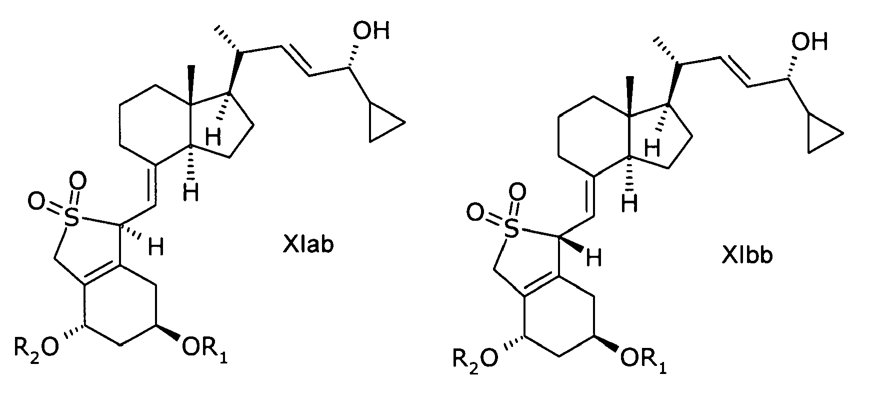 worin R₁ und R₂ wie oben definiert sind, ergibt;
(iii) gegebenenfalls Abtrennen der Verbindungen der allgemeinen Struktur Xlaa und/oder Xlba aus dem Reaktionsgemisch;
(iv) Erwärmen der Verbindungen der allgemeinen Struktur XIaa und/oder Xlba oberhalb von 60°C in Gegenwart einer Base,
was eine Verbindung der allgemeinen Struktur IXa, 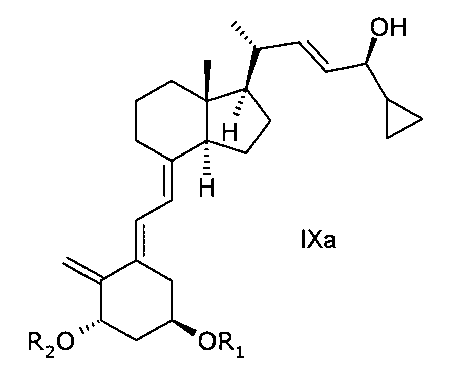 worin R₁ und R₂ wie oben definiert sind, ergibt;
(v) gegebenenfalls Abtrennen der Verbindung der allgemeinen Formel IXa aus dem Reaktionsgemisch;
(vi) Photoisomerisieren der Verbindung der allgemeinen Struktur IXa zur Verbindung der allgemeinen Struktur Xa, 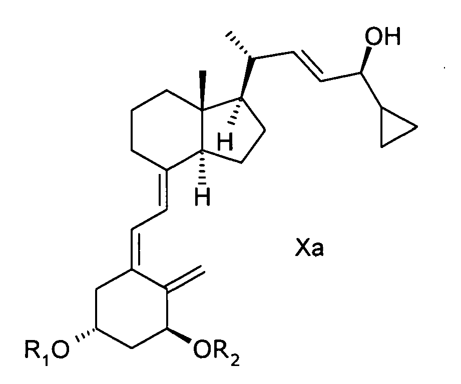 worin R₁ und R₂ wie oben definiert sind;
(vii) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(viii) gegebenenfalls Kristallisieren des Calcipotriols aus einem Gemisch eines organischen Lösungsmittels und Wasser, was Calcipotriol-Monohydrat ergibt,
wobei die Schritte (vi) und (vii) in umgekehrter Reihenfolge durchgeführt werden können.

14. Verfahren zur Herstellung von Calcipotriol oder Calcipotriol-Monohydrat, wobei das Verfahren die folgenden Schritte umfasst:
(i) Umsetzen einer Verbindung der allgemeinen Struktur Xllla, 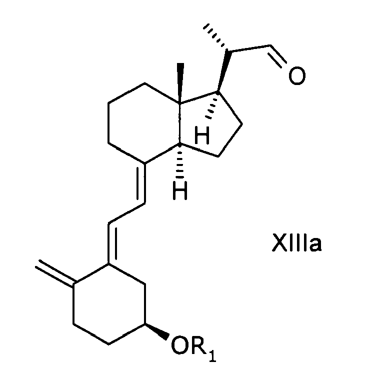 worin R₁ für Wasserstoff oder eine Hydroxyschutzgruppe steht,
mit einem Phosphonat der allgemeinen Struktur VII, 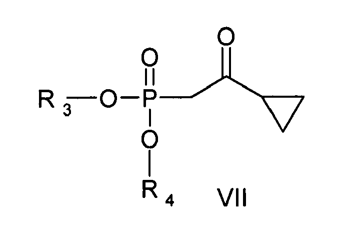 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur XIVa, 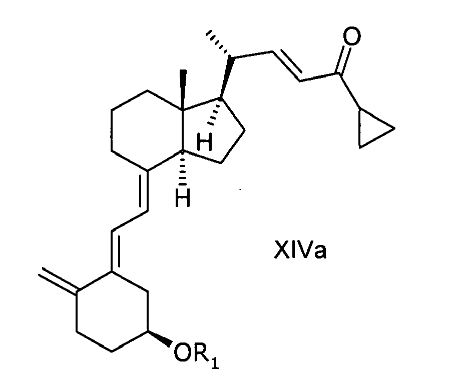 worin R₁ wie oben definiert ist, ergibt;
(ii) Hydroxylieren der Verbindung der allgemeinen Struktur XIVa mit einem geeigneten Hydroxylierungsagens,
was eine Verbindung der allgemeinen Struktur Va, 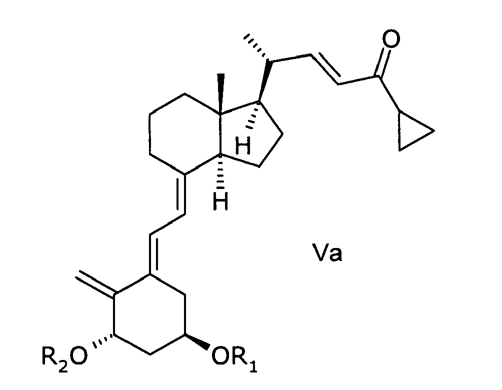 worin R₁ für Wasserstoff oder eine Hydroxyschutzgruppe und R₂ für Wasserstoff steht, ergibt;
(iii) gegebenenfalls Umsetzen der Verbindung der allgemeinen Struktur Va, worin R₁ für Wasserstoff oder eine Hydroxyschutzgruppe steht und R₂ für Wasserstoff steht, mit einem geeigneten Schutzagens,
was eine Verbindung der allgemeinen Struktur Va, worin R₁ und R₂ gleich oder verschieden sind und für eine Hydroxyschutzgruppe stehen, ergibt;
(iv) Reduzieren der Verbindung der allgemeinen Struktur Va mit einem geeigneten Reduktionsmittel,
was eine Verbindung der allgemeinen Struktur IXa oder ein Gemisch der Verbindungen der allgemeinen Struktur IXa und IXb, 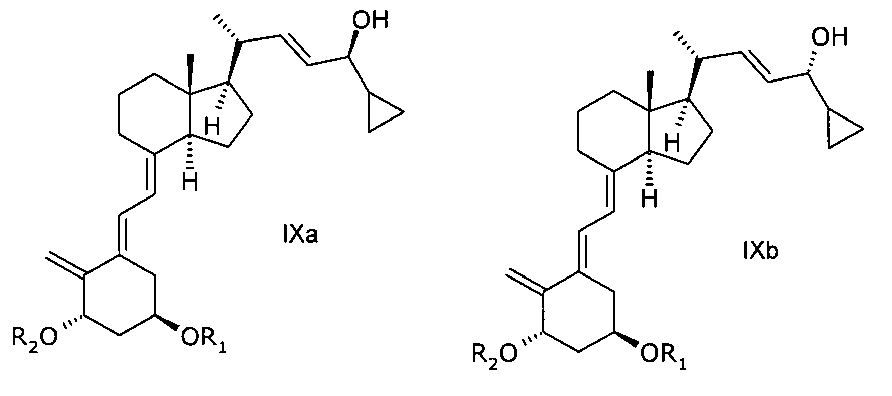 worin R₁ und R₂ wie oben definiert sind, ergibt;
(v) gegebenenfalls Abtrennen der Verbindung der allgemeinen Struktur IXa aus dem Gemisch der Verbindungen der allgemeinen Struktur IXa und IXb;
(vi) Photoisomerisieren der Verbindung der allgemeinen Struktur IXa zur Verbindung der allgemeinen Struktur Xa, 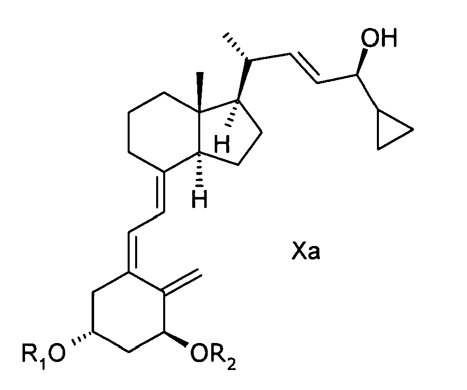 worin R₁ und R₂ wie oben definiert sind;
(vii) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(viii) gegebenenfalls Kristallisieren des Calcipotriols aus einem Gemisch eines organischen Lösungsmittels und Wasser, was Calcipotriol-Monohydrat ergibt.

15. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur Xlllb, 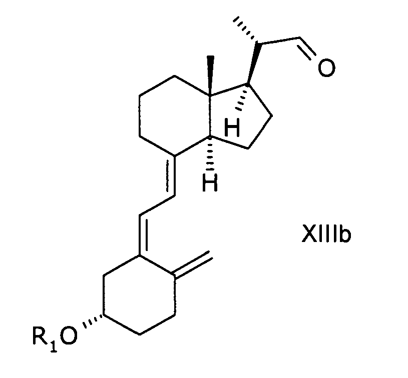 worin R₁ für Wasserstoff oder eine Hydroxyschutzgruppe steht, mit einem Phosphonat der allgemeinen Struktur VII, 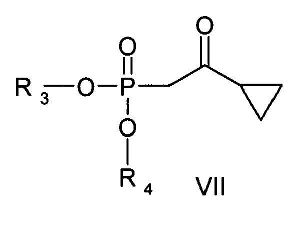 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur XIVb, worin R₁ wie oben definiert ist, ergibt;
(ii) Photoisomerisieren der Verbindung der allgemeinen Struktur XIVb zur Verbindung der allgemeinen Struktur XIVa, 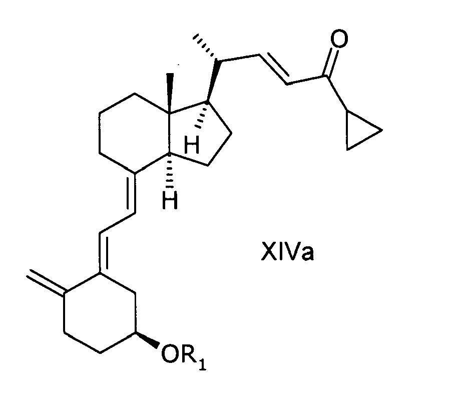 worin R₁ wie oben definiert ist;
(iii) Hydroxylieren der Verbindung der allgemeinen Struktur XIVa mit einem geeigneten Hydroxylierungsmittel,
was eine Verbindung der allgemeinen Struktur Va, 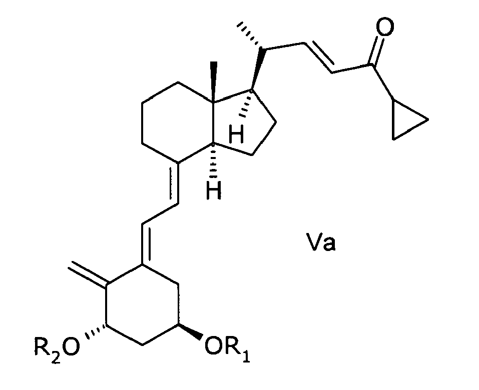 worin R₁ für Wasserstoff oder eine Hydroxyschutzgruppe und R₂ für Wasserstoff steht, ergibt;
(iv) gegebenenfalls Umsetzen der Verbindung der allgemeinen Struktur Va, worin R₁ für Wasserstoff oder eine Hydroxyschutzgruppe steht und R₂ Wasserstoff ist, mit einem geeigneten Schutzagens, was eine Verbindung der allgemeinen Formel Va ergibt, worin R₁ und R₂ gleich oder verschieden sind und für eine Hydroxyschutzgruppe stehen;
(v) Reduzieren der Verbindung der allgemeinen Struktur Va mit einem geeigneten Reduktionsmittel,
was eine Verbindung der allgemeinen Struktur IXa oder ein Gemisch der Verbindungen der allgemeinen Struktur IXa und IXb ergibt, 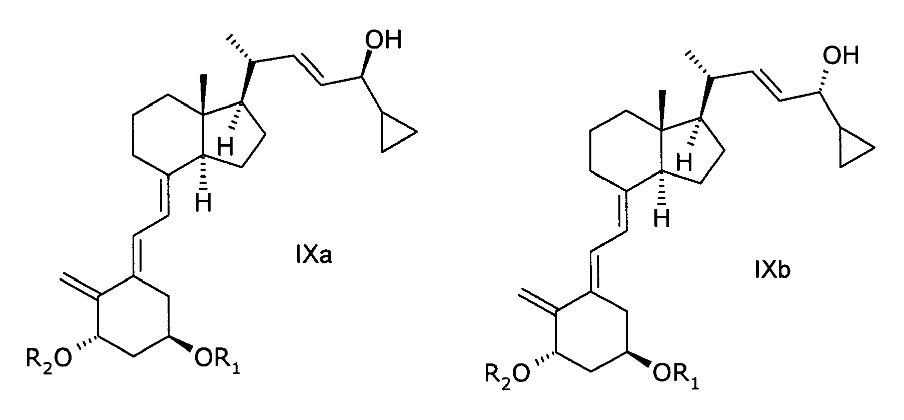 worin R₁ und R₂ wie oben definiert sind;
(vi) gegebenenfalls Abtrennen der Verbindung der allgemeinen Struktur IXa aus dem Gemisch der Verbindungen der allgemeinen Struktur IXa und IXb;
(vii) Photoisomerisieren der Verbindung der allgemeinen Struktur IXa zur Verbindung der allgemeinen Struktur Xa, 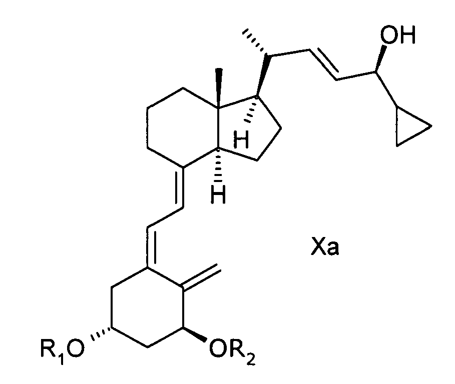 worin R₁ und R₂ wie oben definiert sind,
(viii) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(ix) gegebenenfalls Kristallisieren des Calcipotriols aus einem Gemisch eines organischen Lösungsmittels und Wasser, was Calcipotriol-Monohydrat ergibt.

16. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur XVa und/oder XVb, 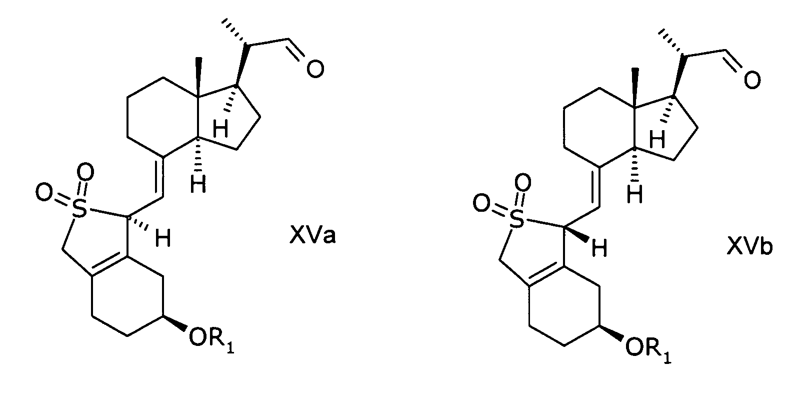 worin R₁ für Wasserstoff oder eine Hydroxyschutzgruppe steht,
mit einem Phosphonat der allgemeinen Struktur VII, 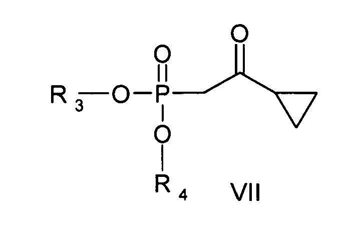 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur XVIa und/oder XVIb, 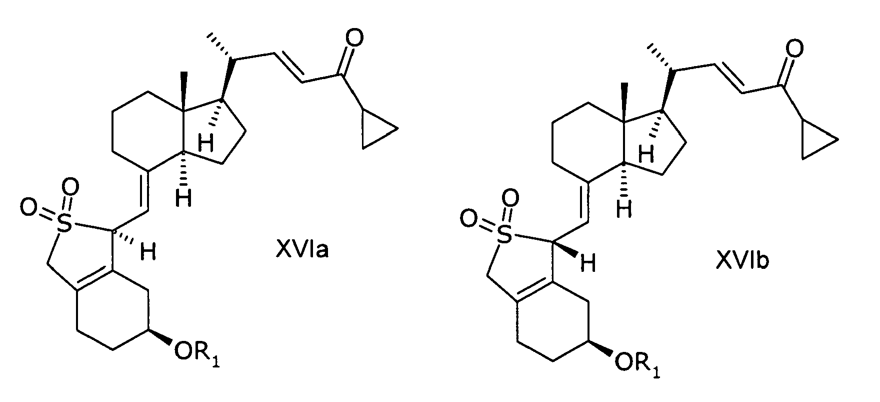 worin R₁ wie oben definiert ist, ergibt;
(ii) Erwärmen der Verbindungen der allgemeinen Struktur XVIa und/oder XVIb oberhalb von 60 °C in Gegenwart einer Base,
was eine Verbindung der allgemeinen Struktur XIVa, 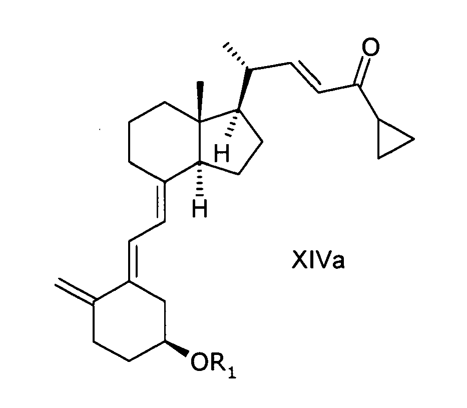 worin R₁ wie oben definiert ist, ergibt;
(iii) Hydroxylieren der Verbindung der allgemeinen Struktur XIVa mit einem geeigneten Hydroxylierungsagens,
was eine Verbindung der allgemeinen Struktur Va, 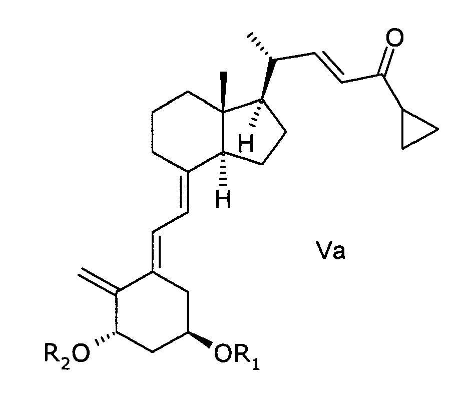 worin R₁ für Wasserstoff oder eine Hydroxyschutzgruppe steht und R₂ Wasserstoff ist, ergibt;
(iv) gegebenenfalls Umsetzen der Verbindung der allgemeinen Struktur Va, worin R₁ für Wasserstoff oder eine Hydroxyschutzgruppe steht und R₂ Wasserstoff ist, mit einem geeigneten Schutzagens,
was eine Verbindung der allgemeinen Struktur Va, worin R₁ und R₂ gleich oder verschieden sind und für eine Hydroxyschutzgruppe stehen, ergibt;
(v) Reduzieren der Verbindung der allgemeinen Struktur Va mit einem geeigneten Reduktionsmittel,
was eine Verbindung der allgemeinen Struktur IXa oder ein Gemisch der Verbindungen der allgemeinen Struktur IXa und IXb ergibt, 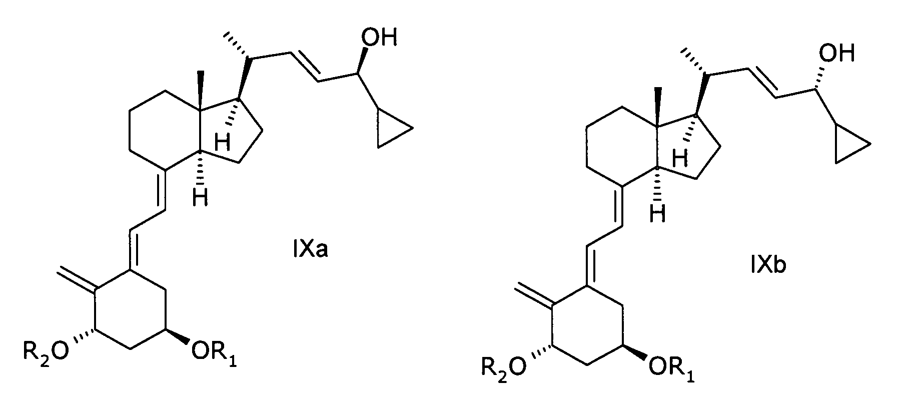 worin R₁ und R₂ wie oben definiert sind;
(vi) gegebenenfalls Abtrennen der Verbindung der allgemeinen Struktur IXa aus dem Gemisch der Verbindungen der allgemeinen Struktur IXa und IXb;
(vii) Photoisomerisieren der Verbindung der allgemeinen Struktur IXa zur Verbindung der allgemeinen Struktur Xa, 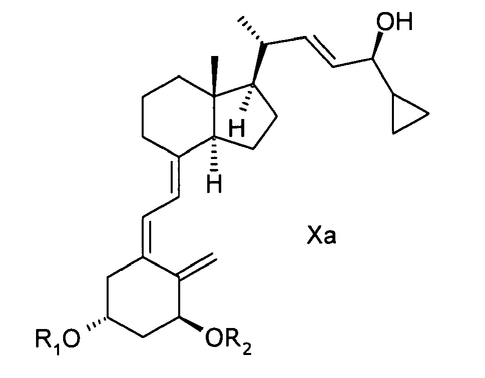 worin R₁ und R₂ wie oben definiert sind;
(vii) falls R₁ und/oder R₂ nicht Wasserstoff sind, Entfernen der Hydroxyschutzgruppe(n) R₁ und/oder R₂ der Verbindung der allgemeinen Struktur Xa, um Calcipotriol zu bilden; und
(viii) wahlweises Kristallisieren des Calcipotriols aus einem Gemisch eines organischen Lösungsmittels und Wasser, was Calcipotriol-Monohydrat ergibt.

17. Verfahren zur Herstellung von Calcipotriol oder Calcipotriol-Monohydrat nach Anspruch 9, wobei das Verfahren die folgenden Schritte umfasst:
(i) Umsetzen einer Verbindung der allgemeinen Struktur IXX, 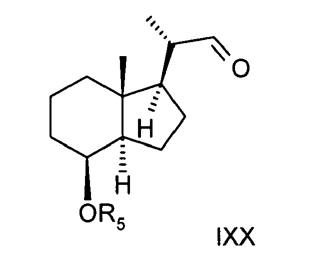 worin R₅ für Wasserstoff oder eine Hydroxyschutzgruppe steht, mit einem Phosphonat der allgemeinen Struktur VII, 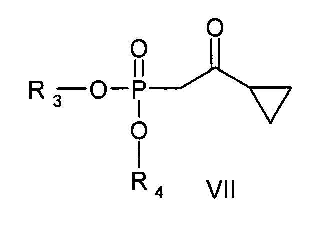 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy,
in Gegenwart einer Base,
was eine Verbindung der allgemeinen Struktur XX, 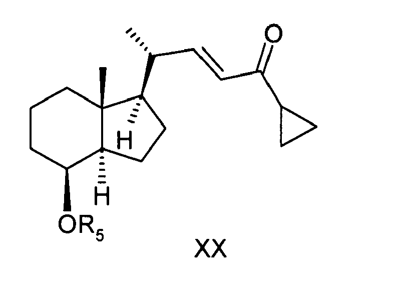 worin R₅ wie oben definiert ist, ergibt;
(ii) Reduzieren der Verbindung der allgemeinen Struktur XX mit einem geeigneten Reduktionsmittel,
was eine Verbindung der allgemeinen Struktur XXIa oder ein Gemisch der Verbindungen der allgemeinen Struktur XXIa und XXIb, 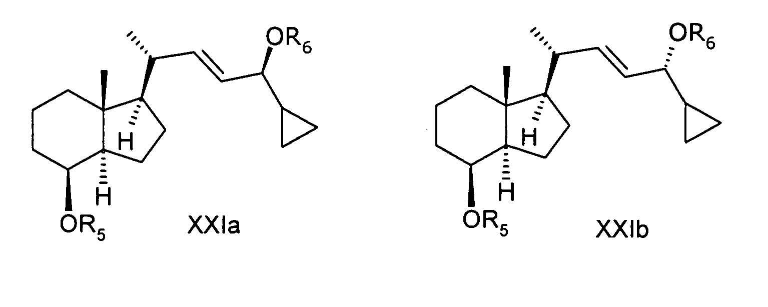 worin R₅ wie oben definiert ist und R₆ Wasserstoff ist, ergibt;
(iii) gegebenenfalls Abtrennen der Verbindung der allgemeinen Struktur XXIa aus dem Gemisch der Verbindungen der allgemeinen Struktur XXIa und XXIb;
(iv) Schützen der Allylhydroxygruppe der Verbindung der allgemeinen Struktur XXIa mit einem geeigneten Hydroxyschutzreagens,
was eine Verbindung der allgemeinen Struktur XXIa, worin R₆ für eine Hydroxyschutzgruppe steht und R₅ wie oben definiert ist, ergibt;
(v) falls R₅ nicht Wasserstoff ist, Abtrennen der Hydroxyschutzgruppe R₅ der Verbindung der allgemeinen Struktur XXIa, was eine Verbindung der allgemeinen Struktur XXIa, worin R₅ Wasserstoff ist, ergibt;
(vi) Oxidieren der Hydroxygruppe der Verbindung der allgemeinen Struktur XXIa mit einem geeigneten Oxidationsmittel, was eine Verbindung der allgemeinen Struktur XXII, 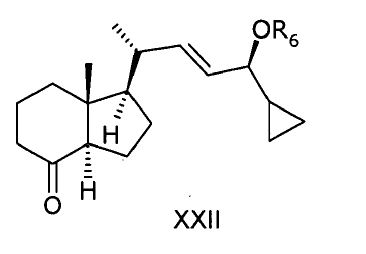 worin R₆ wie oben definiert ist, ergibt;
(vii) Kopplung der Verbindung der allgemeinen Struktur XXII mit einem Wittig-Reagens XXIIIa oder einem Wittig-Horner-Reagens XXIIIb, 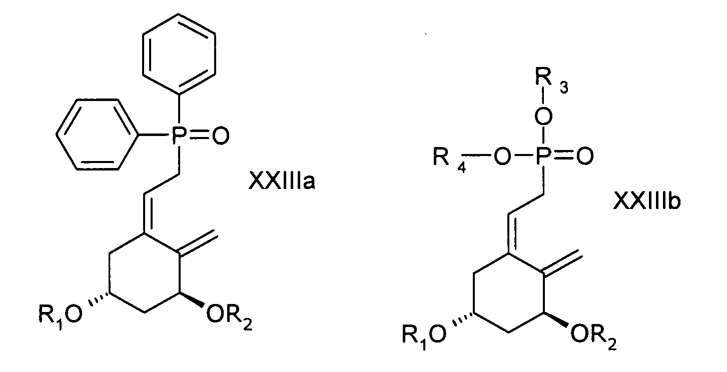 worin R₁ und R₂ für Wasserstoff oder eine Hydroxyschutzgruppe stehen und R₃ und R₄ wie oben definiert sind;
in Gegenwart einer Base,
was eine Verbindung der allgemeinen Struktur XXIVa, 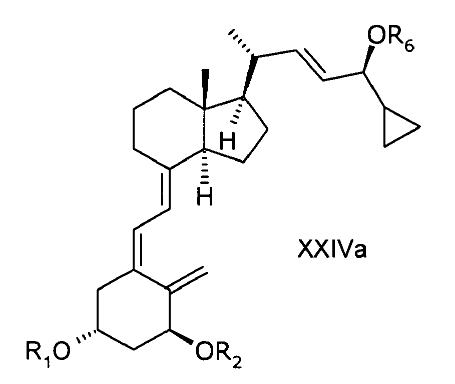 worin R₁ und R₂ gleich oder verschieden sind und für Wasserstoff oder eine Hydroxyschutzgruppe stehen und R₆ wie oben definiert ist, ergibt;
(viii) falls R₆ nicht Wasserstoff ist, Entfernen der Hydroxyschutzgruppe R₆ der Verbindung der allgemeinen Struktur XXIVa;
(ix) gegebenenfalls Abtrennen der Verbindung der allgemeinen Struktur XXIVa;
(x) falls R₁ und R₂ nicht Wasserstoff sind, Entfernen der Hydroxyschutzgruppe(n) R₁ und R₂ der Verbindung der allgemeinen Struktur XXIVa, um Calcipotriol zu bilden; und
(xi) gegebenenfalls Kristallisieren des Calcipotriols aus einem Gemisch eines organischen Lösungsmittels und Wasser, was Calcipotriol-Monohydrat ergibt.

18. Verfahren nach einem der Ansprüche 1 bis 17, wobei R₃ und R₄ (C₁-C₆)-Alkyl sind.

19. Verfahren nach einem der Ansprüche 1 bis 17, wobei R₃ und R₄ Methyl oder Ethyl sind.

20. Verfahren nach einem der Ansprüche 1 bis 19, wobei R₁ und R₂ Wasserstoff oder Alkylsilyl sind.

21. Verfahren nach einem der Ansprüche 1 bis 19, wobei R₁ und R₂ Wasserstoff oder *tert-*Butyldimetylsilyl sind.

22. Verfahren nach einem der Ansprüche 8 oder 19, worin R₅ Triethylsilyl ist und R₃ und R₄ Methyl oder Ethyl sind.

23. Verfahren nach einem der Ansprüche 1 bis 22, wobei die Umsetzung mit dem Phosphonat der allgemeinen Struktur VII unter Phasentransferbedingungen durchgeführt wird.

24. Verfahren nach einem der Ansprüche 1 bis 22, wobei die Umsetzung mit dem Phosphonat der allgemeinen Struktur VII unter Phasentransferbedingungen in einem Gemisch aus Toluol oder Xylol und Wasser mit einem Tetraalkylammoniumhalogenid oder einem Tetraalkylammoniumhydrogensulfat als Phasentransferkatalysator und mit einem Alkalimetallhydroxid und/oder einem Tetraalkylammoniumhydroxid als Base durchgeführt wird.

25. Verfahren nach einem der Ansprüche 1 bis 24, wobei die Umsetzung mit dem Phosphonat der allgemeinen Struktur VII bei einer Temperatur von 10 °C bis 50 °C durchgeführt wird.

26. Verbindung der allgemeinen Struktur VII, 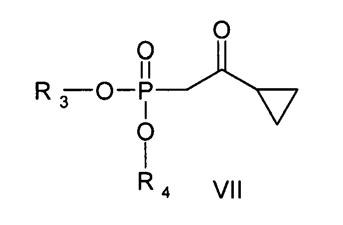 worin R₃ und R₄ gleich und verschieden sind und für Alkyl, Haloalkyl, Hydroxyalkyl, Alkenyl, Alkinyl, Aralkyl, Aralkenyl, Aralkinyl oder Aryl stehen, wobei jeder Rest gegebenenfalls mit einem oder mehreren Substituenten substituiert ist, die ausgewählt sind unter Alkyl, Aralkyl, Cycloalkyl, Cycloalkenyl, Haloalkyl, Hydroxyalkyl, Alkenyl, Alkinyl, Aralkyl, Aralkenyl, Aralkinyl, Aryl, Oxo, Alkoxycarbonyl, Alkylcarbonyloxy, Halogen, Alkoxy, Carboxy, Sulfo oder Hydroxy, mit der Maßgabe, dass die Verbindung nicht (2-Cyclopropyl-2-oxoethyl)-phosphonsäurediethylester ist,
wobei die Begriffe Alkyl, Alkenyl, Alkinyl, Haloalkyl, Hydroxyalkyl, Alkoxy, Alkoxycarbonyl, Alkylcarbonyloxy, Cycloalkyl, Cycloalkenyl, Aryl, Aralkyl, Aralkenyl und Aralkinyl wie in Anspruch 1 definiert sind0.

## Revendications

1. Procédé de préparation d'un composé de la structure générale Va, Vb, VIIIa, VIIIb, XIVa, XIVb, XVIa, XVIb ou XX respectivement, 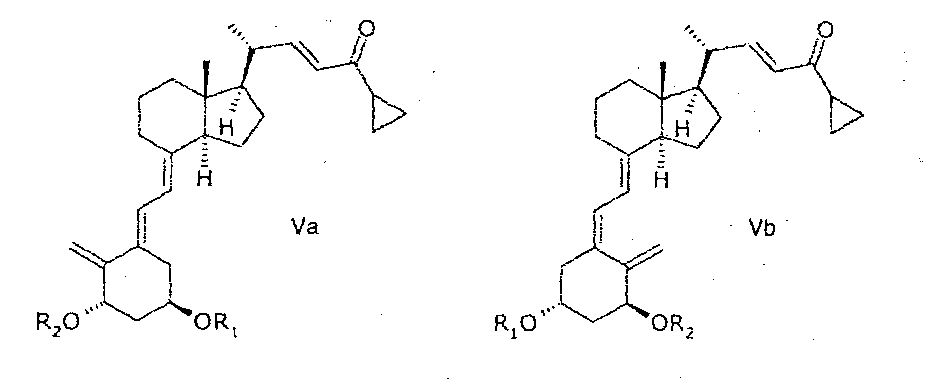 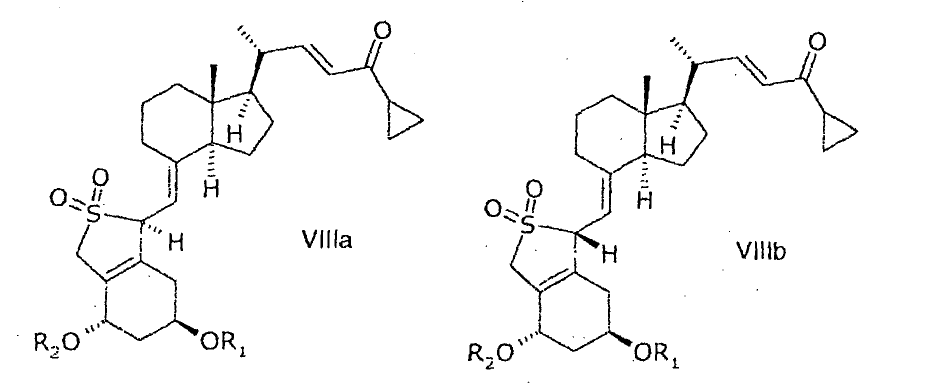 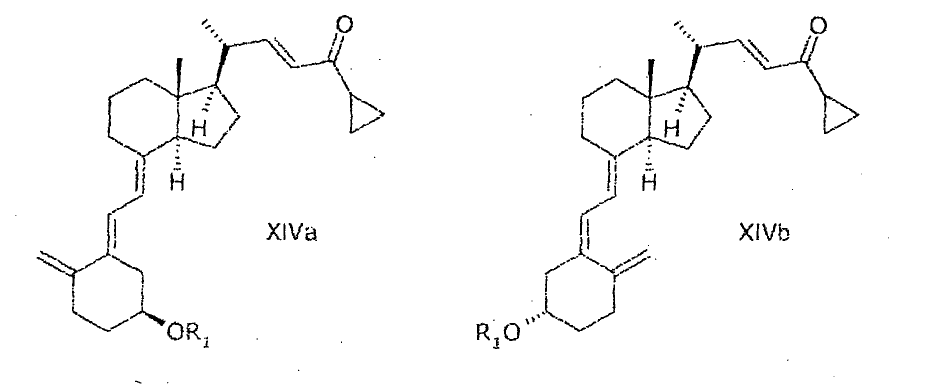 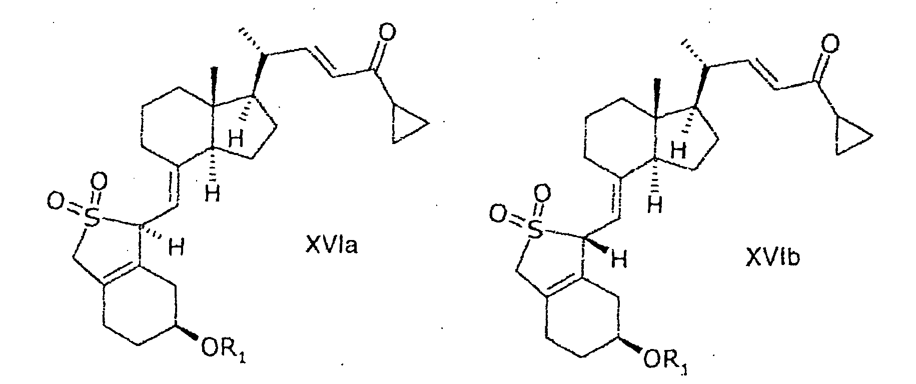 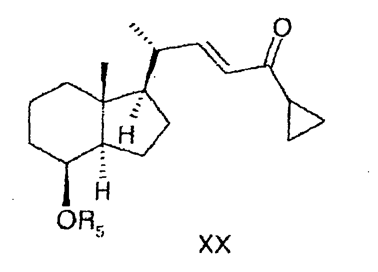 dans lesquelles R₁ et R₂ sont identiques ou différents et représentent un atome d'hydrogène ou un groupe hydroxy protecteur, et dans lesquelles R₅ représente un atome d'hydrogène ou un groupe hydroxy protecteur ;
le procédé comprenant la réaction d'un composé de la structure générale IIIa, IIIb, VIa, VIb, XIIIa, XIIIb, XVa, ou XVb, ou IXX respectivement, 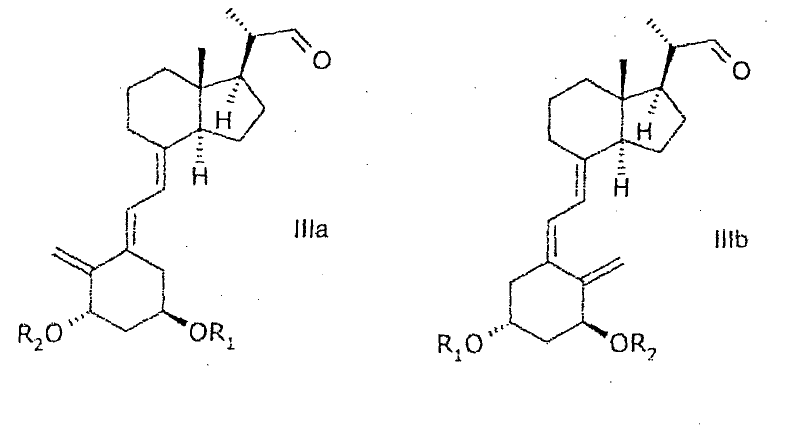 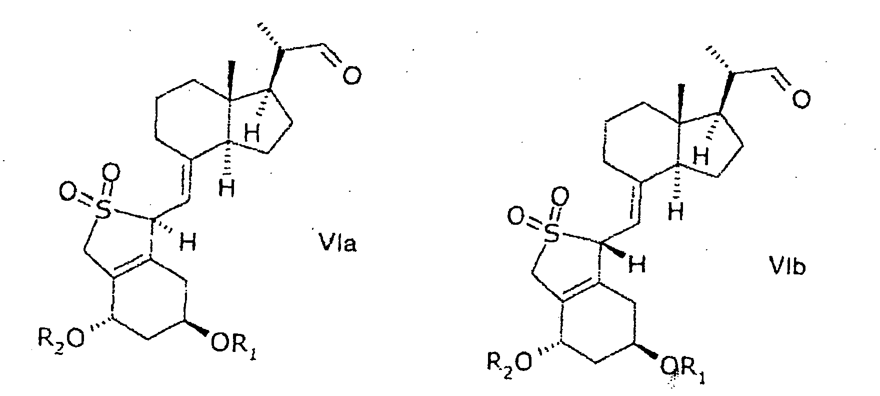 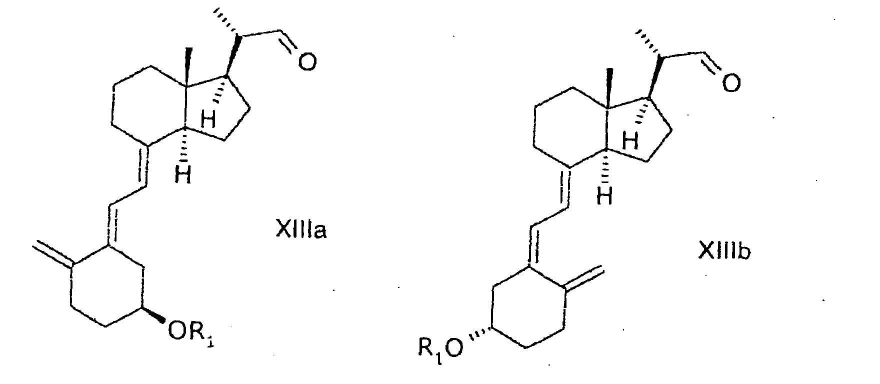 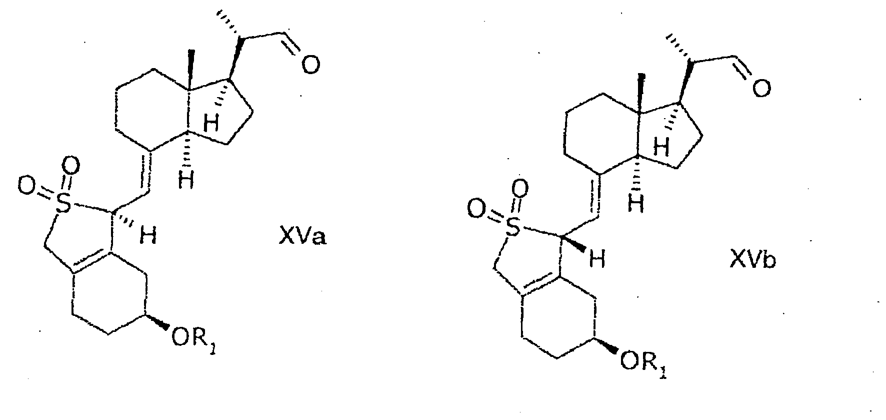 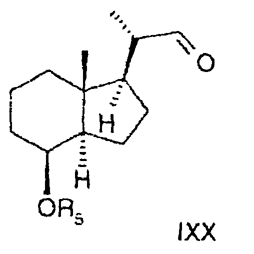 dans lesquelles R₁, R₂ et R₅ sont comme définis ci-dessus ; avec un phosphonate de la structure générale VII, 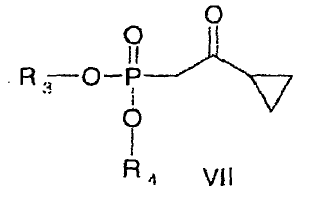 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base ;
dans lequel le terme "alkyle" représente un groupe alkyle linéaire ou ramifié qui peut être cyclique ou acyclique ayant de 1 à 20 atomes de carbone ;
le terme "alcényle" représente un radical hydrocarboné mono-, di-, tri-, tétra- ou pentainsaturé comprenant 2-10 atomes de carbone ;
le terme "alcynyle" représente un radical hydrocarboné comprenant 1-5 triples liaisons C-C et 2-20 atomes de carbone ;
le terme "haloalkyle" représente un groupe alkyle comme défini ci-dessus substitué par un ou plusieurs atomes d'halogène ;
le terme "hydroxyalkyle," représente un groupe alkyle comme défini ci-dessus substitué par un ou plusieurs groupes hydroxy ;
le terme "alcoxy" représente un radical de la formule -OR', dans laquelle R' est un groupe alkyle comme indiqué ci-dessus ;
le terme "alcoxycarbonyle" représente un radical de la formule -C(O)-O-R', dans laquelle R' est un groupe alkyle comme indiqué ci-dessus ;
le terme "alkylcarbonyloxy" représente un radical de la formule -C(O)-O-R', dans laquelle R' est un groupe alkyle comme indiqué ci-dessus ;
le terme "cycloalkyle" représente un radical cycloalcane saturé comprenant 3-20 atomes de carbone ;
le terme "cycloalcényle" représente des radicaux hydrocarbonés cycliques mono-, di-, tri- ou tétrainsaturés non-aromatiques comprenant 3-20 atomes de carbone ;
le terme "aryle" représente un radical de cycles aromatiques, carbocycliques éventuellement condensés comprenant 6-20 atomes de carbone ;
le terme "aralkyle" représente un groupe alkyle comme défini ci-dessus substitué par un ou plusieurs radicaux aryle comme défini ci-dessus ;
le terme "aralcényle" représente un groupe alcényle comme défini ci-dessus substitué par un ou plusieurs radicaux aryle comme définis ci-dessus ; et
le terme "aralcynyle" représente un groupe alcynyle comme défini ci-dessus substitué par un ou plusieurs radicaux aryle comme définis ci-dessus.

2. Procédé selon la revendication 1 pour la préparation d'un composé de la structure générale Va, 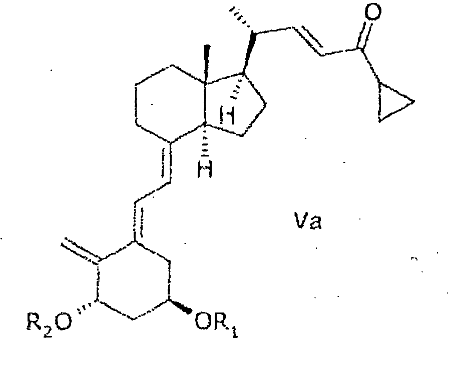 dans laquelle R₁ et R₂ sont identiques ou différents et représentent chacun un atome d'hydrogène ou un groupe hydroxy protecteur,
le procédé comprenant la réaction d'un composé de la structure générale IIIa, 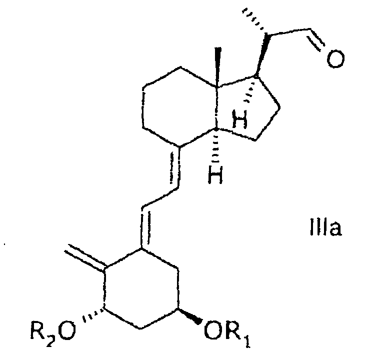 dans laquelle R₁ et R₂ sont comme définis ci-dessus, avec un phosphonate de la structure générale VII, 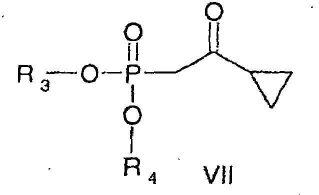 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

3. Procédé selon la revendication 1 pour la préparation d'un composé de la structure générale Vb, 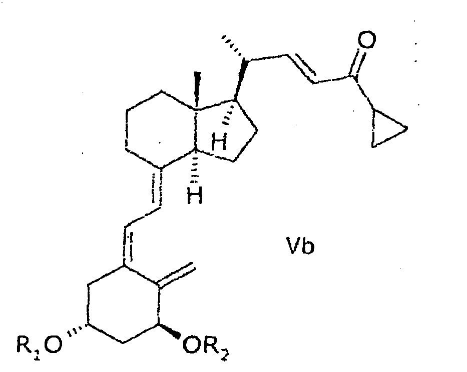 dans laquelle R₁ et R₂ sont identiques ou différents et représentent chacun un atome d'hydrogène ou un groupe hydroxy protecteur,
le procédé comprenant la réaction d'un composé de la structure générale IIIb, 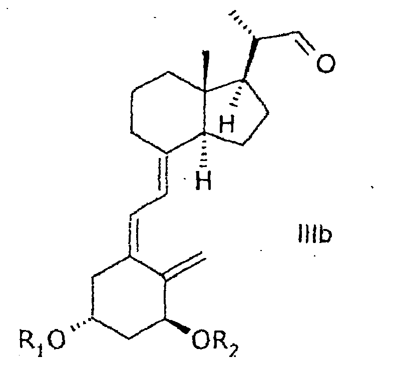 dans laquelle R₁ et R₂ sont comme définis ci-dessus, avec un phosphonate de la structure générale VII, 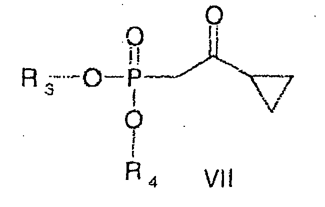 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par
un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

4. Procédé selon la revendication 1 pour la préparation d'un composé de la structure générale VIIIa ou VIIIb respectivement, 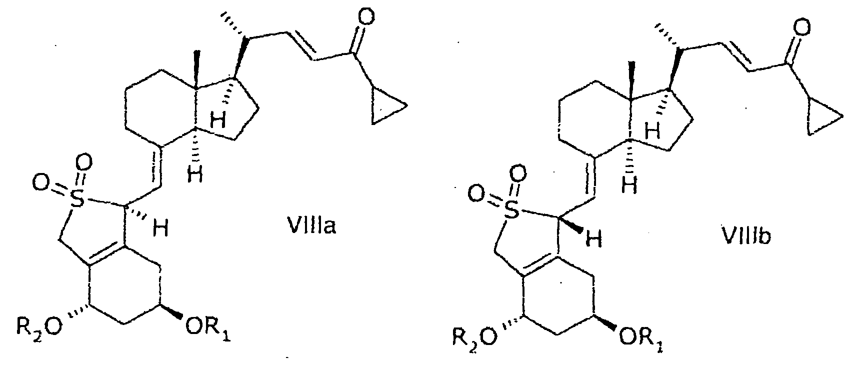 dans lesquelles R₁ et R₂ sont identiques ou différents et représentent chacun un atome d'hydrogène ou un groupe hydroxy protecteur le procédé comprenant la réaction d'un composé de la structure générale VIa ou VIb respectivement, 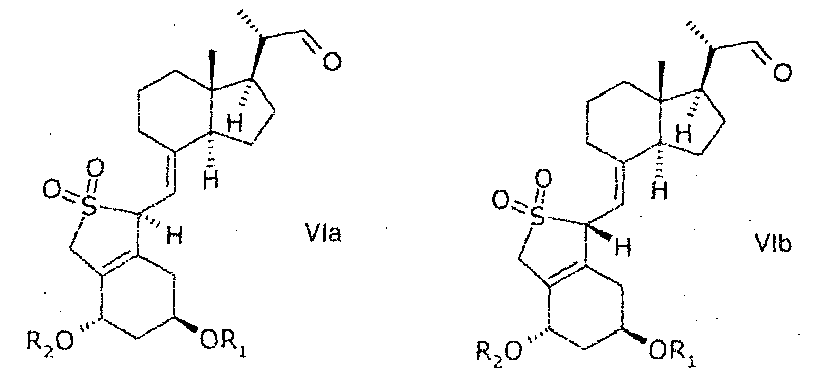 dans lesquelles R₁ et R₂ sont comme définis ci-dessus,
avec un phosphonate de la structure générale VII, 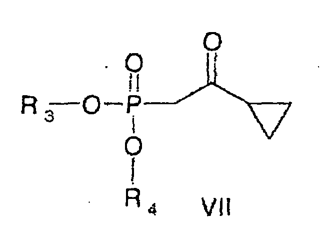 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

5. Procédé selon la revendication 1 pour la préparation d'un
composé de la structure générale XIVa, 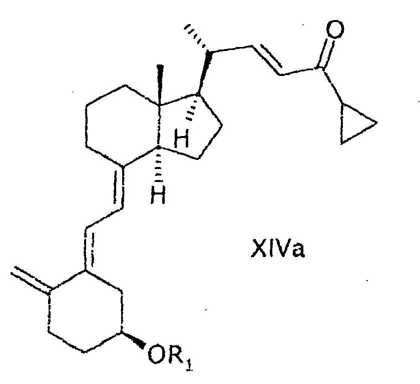 dans laquelle R₁ représente un atome d'hydrogène ou un groupe hydroxy protecteur,
le procédé comprenant la réaction d'un composé de la structure générale XIIIa, 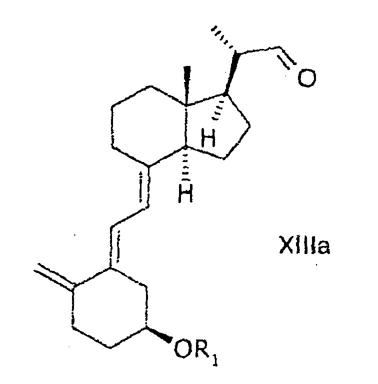 dans laquelle R₁ est comme défini ci-dessus,
avec un phosphonate de la structure générale VII, 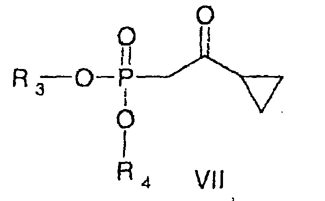 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

6. Procédé selon la revendication 1 pour la préparation d'un composé de la structure générale XIVb, 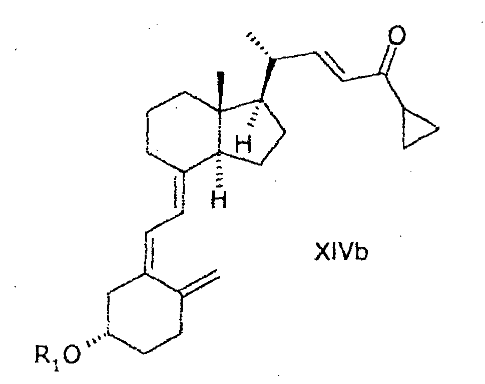 dans laquelle R₁ représente un atome d'hydrogène ou un groupe hydroxy protecteur,
le procédé comprenant la réaction d'un composé de la structure générale XIIIb, 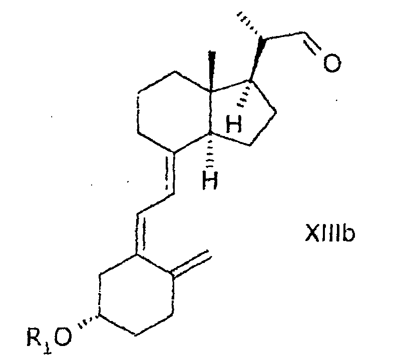 dans laquelle R₁ est comme défini ci-dessus,
avec un phosphonate de la structure générale VII, 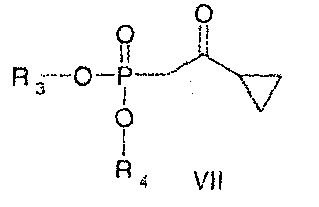 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

7. Procédé selon la revendication 1 pour la préparation d'un
composé de la structure générale XVIa ou XVIb respectivement, 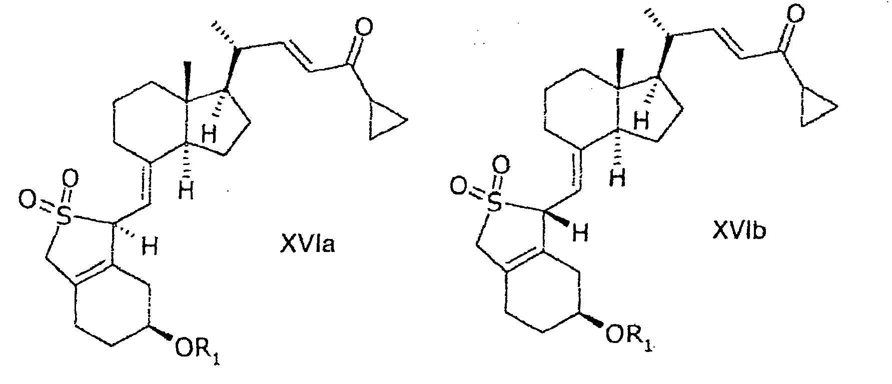 dans lesquelles R₁ représente un atome d'hydrogène ou un groupe hydroxy protecteur,
le procédé comprenant la réaction d'un composé de la structure générale XVa ou XVb respectivement, 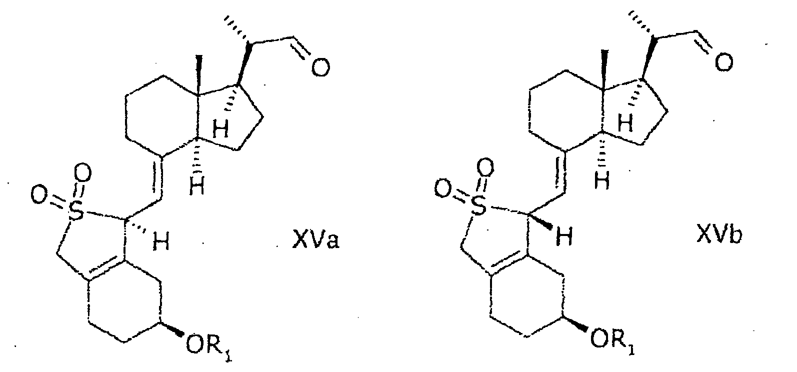 dans laquelle R₁ est comme défini ci-dessus,
avec un phosphonate de la structure générale VII, 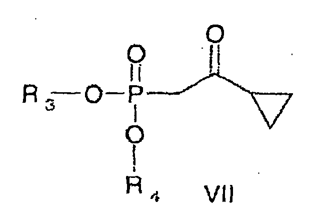 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

8. Procédé selon la revendication 1 pour la préparation d'un
composé de la structure générale XX, 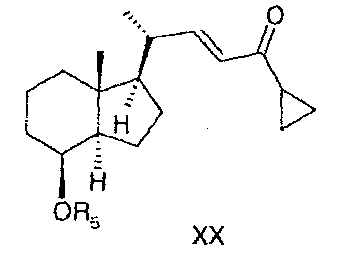 dans laquelle R₅ représente un atome d'hydrogène ou un groupe hydroxy protecteur,
le procédé comprenant la réaction d'un composé de la structure générale IXX, 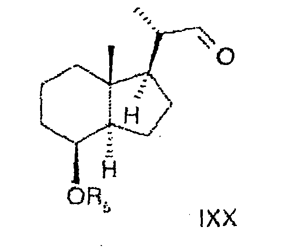 dans laquelle, R₅, est comme défini ci-dessus,
avec un phosphonate de la structure générale VII, 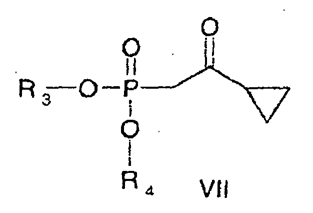 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base.

9. Procédé de préparation de calcipotriol ou de monohydrate de calcipotriol, le procédé comprenant le procédé selon l'une quelconque des revendications 1, 2, 3, 4, 5, 6, 7 ou 8.

10. Procédé de production de calcipotriol ou de monohydrate de calcipotriol selon la revendication 9, le procédé comprenant les étapes consistant :
(i) à faire réagir un composé de la structure générale IIIa, 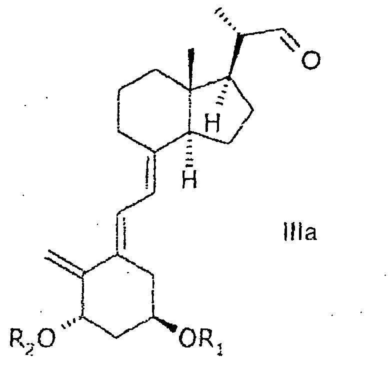 dans laquelle R₁ et R₂ sont identiques ou différents et représentent un atome d'hydrogène ou un groupe hydroxy protecteur,
avec un phosphonate de la structure générale VII, 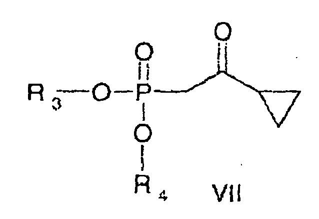 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale Va, 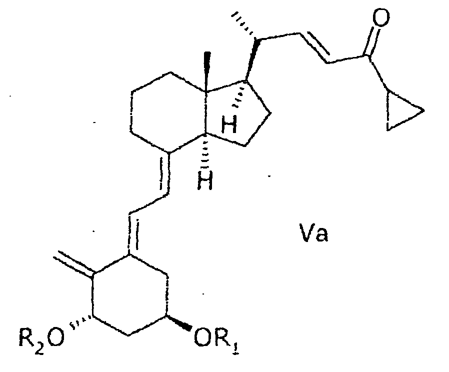 dans laquelle R₁ et R₂ sont comme définis ci-dessus;
(ii) à réduire le composé de la structure générale Va avec un agent réducteur approprié, pour fournir un composé de la structure générale IXa ou un mélange de composés de la structure IXa et IXb, 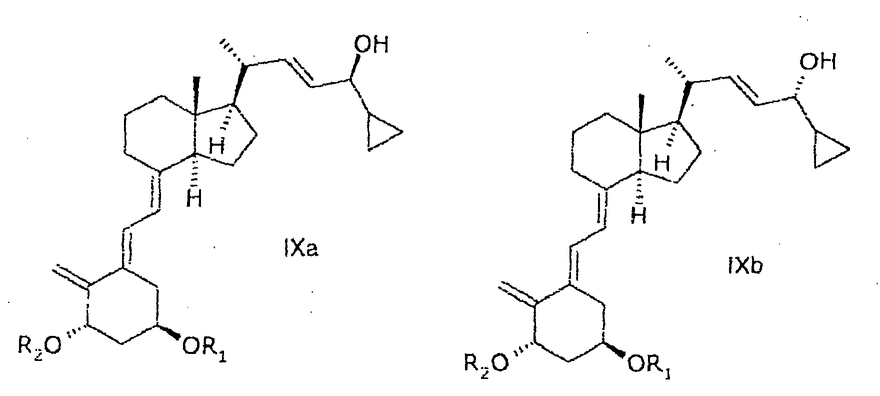 dans lesquelles R₁ et R₂ sont comme définis ci-dessus ;
(iii) à séparer éventuellement le composé de la structure générale IXa du mélange des composés de la structure IXa et IXb ;
(iv) à réaliser une photoisomérisation du composé de la structure générale IXa en le composé de la structure générale Xa, 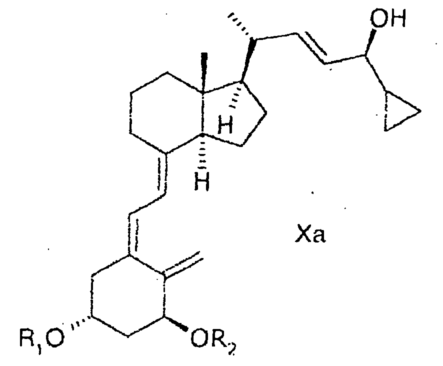 dans laquelle R₁ et R₂ sont comme définis ci-dessus ;
(v) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(vi) à faire cristalliser éventuellement le calcipotriol à partir d'un mélange d'un solvant organique et d'eau pour fournir du monohydrate de calcipotriol.

11. Procédé de production de calcipotriol ou de monohydrate de calcipotriol selon la revendication 9, le procédé comprenant les étapes consistant :
(i) à faire réagir un composé de la structure générale IIIb, 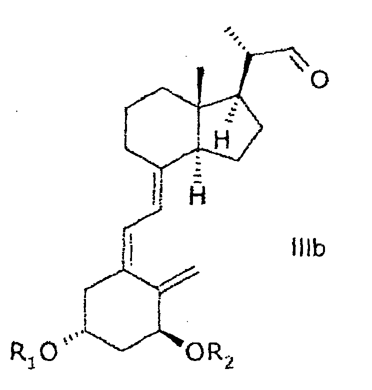 dans laquelle R₁ et R₂ sont identiques ou différents et représentent un atome d'hydrogène ou un groupe hydroxy protecteur,
avec un phosphonate de la structure générale VII, 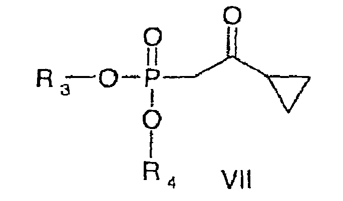 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale Vb, 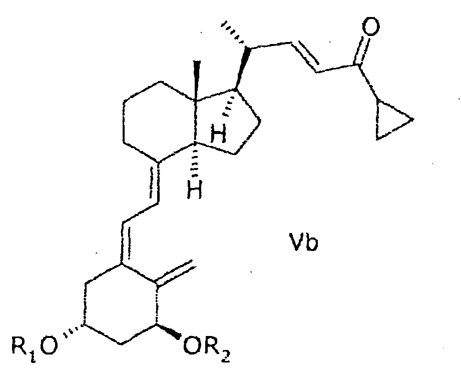 dans laquelle R₁ et R₂ sont comme définis ci-dessus ;
(ii) à réduire le composé de la structure générale Vb avec un agent réducteur approprié pour fournir un composé de la structure générale Xa ou un mélange de composés de la structure générale Xa et Xb, 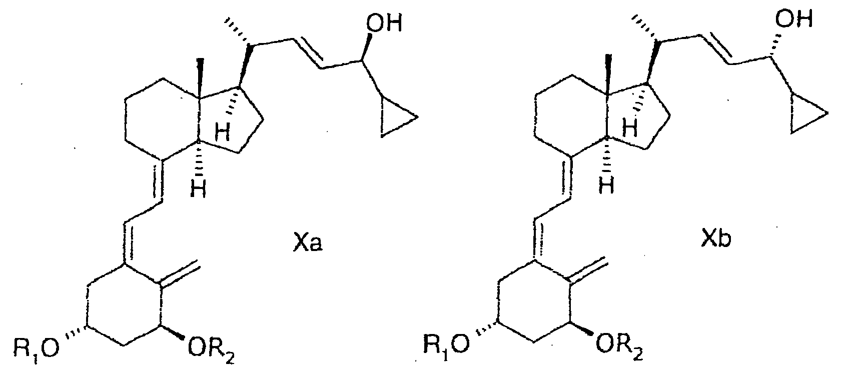 dans lesquelles R₁ et R₂ sont comme définis ci-dessus ;
(iii) à séparer éventuellement le composé de la structure générale Xa du mélange de composés de la structure générale Xa et Xb ;
(iv) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(v) à faire éventuellement cristalliser le calcipotriol à partir d'un mélange d'un solvant organique et d'eau pour produire du monohydrate de calcipotriol.

12. Procédé de production de calcipotriol ou de monohydrate de calcipotriol selon la revendication 9, le procédé comprenant les étapes consistant :
(i) à faire réagir un composé de la structure générale VIa et/ou VIb, 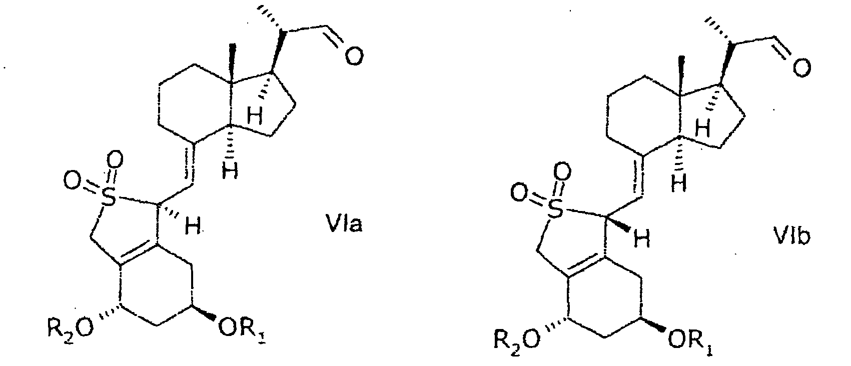 dans lesquelles R₁ et R₂ sont identiques ou différents et représentent un atome d'hydrogène ou un groupe hydroxy protecteur,
avec un phosphonate de la structure générale VII, 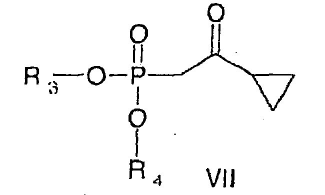 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale VIIIa et/ou VIIIb, 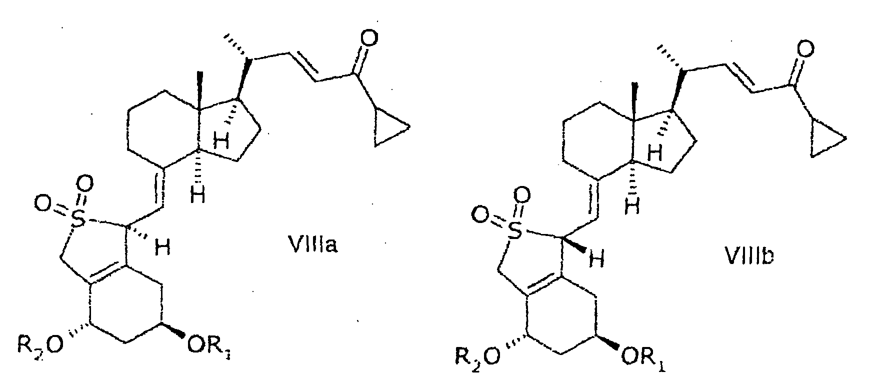 dans lesquelles R₁ et R₂ sont comme définis ci-dessus ;
(ii) à chauffer les composés de la structure générale VIIIa et/ou VIIIb au-delà de 60°C en présence d'une base,
pour fournir un composé de la structure générale Va, 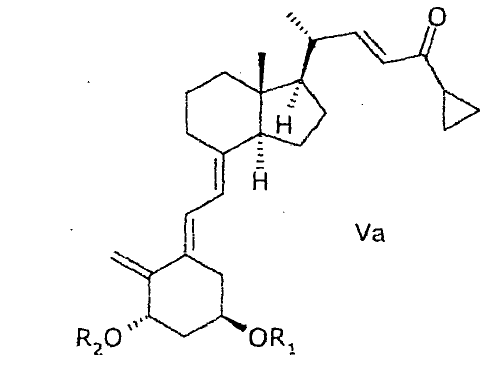 dans laquelle R₁ et R₂ sont comme définis ci-dessus ;
(iii) à réduire le composé de la structure générale Va avec un agent réducteur approprié, pour fournir un composé de la structure générale IXa ou un mélange de composés de la structure générale IXa et IXb, 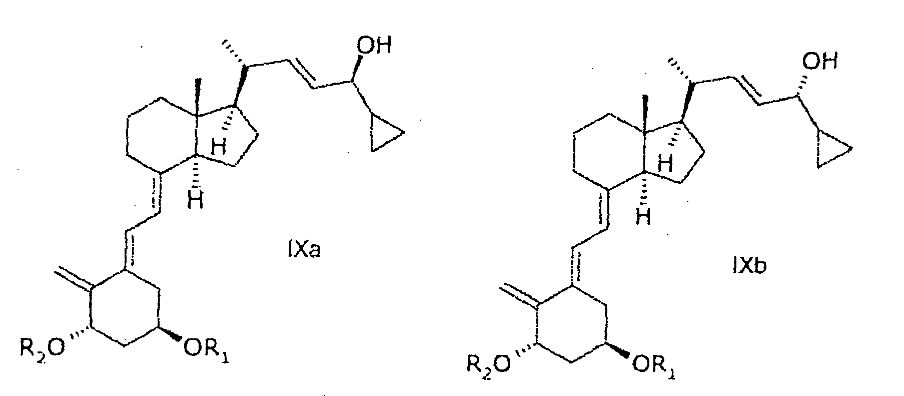 dans lesquelles R₁ et R₂ sont comme définis ci-dessus ;
(iv) à séparer éventuellement le composé de la structure générale IXa du mélange de composés de la structure générale IXa et IXb ;
(v) à réaliser une photoisomérisation du composé de la structure générale IXa en le composé de la structure générale Xa, 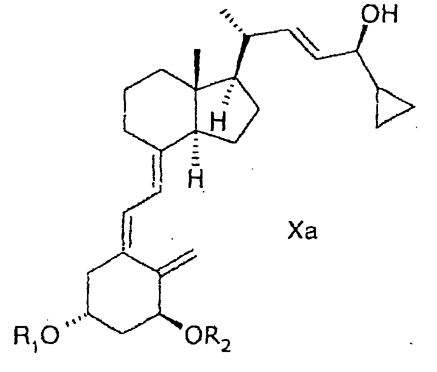 dans laquelle R₁ et R₂ sont comme définis ci-dessus ;
(vi) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(vii) à faire éventuellement cristalliser le calcipotriol à partir d'un mélange d'un solvant organique et d'eau pour fournir du monohydrate de calcipotriol.

13. Procédé de production de calcipotriol ou de monohydrate de calcipotriol selon la revendication 9, le procédé comprenant les étapes consistant :
(i) à faire réagir un composé de la structure générale VIa et/ou VIb, 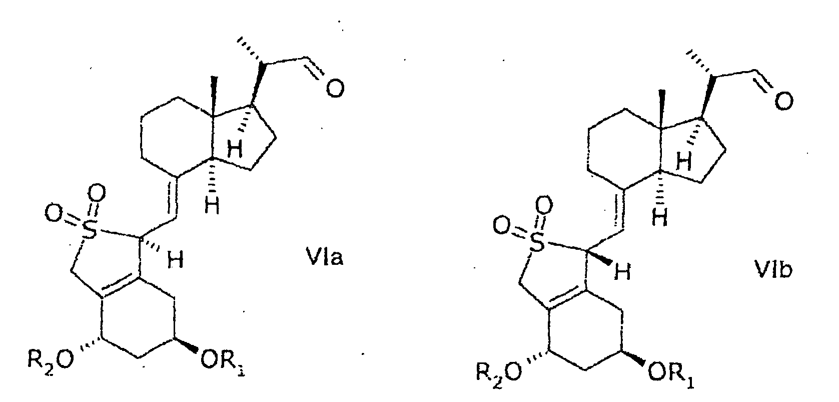 dans lesquelles R₁ et R₂ sont identiques ou différents et représentent un atome d'hydrogène ou un groupe hydroxy protecteur,
avec un phosphonate de la structure générale VII, 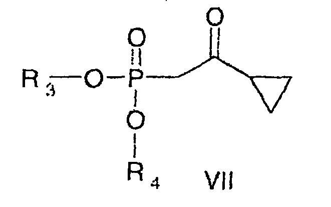 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale VIIIa et/ou VIIIb, 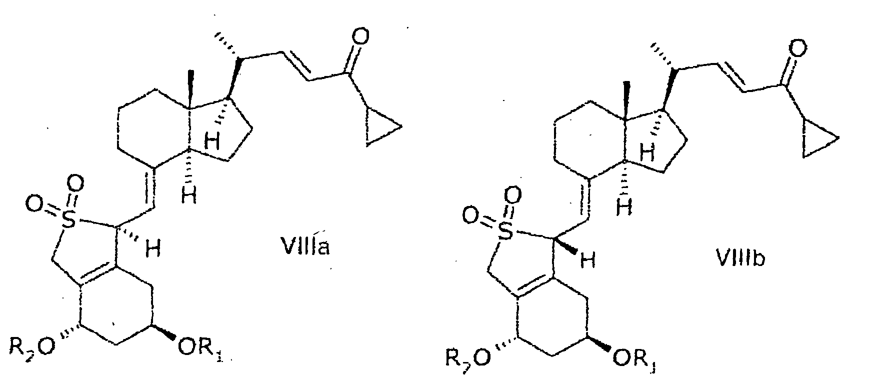 dans lesquelles R₁ et R₂ sont comme définis ci-dessus ;
(ii) à réduire les composés de la structure générale VIIIa et/ou VIIIb, avec un agent réducteur approprié dans un solvant inerte,
pour fournir les composés de la structure générale XIaa et/ou XIba, ou un mélange de composés de la structure générale XIaa et/ou XIba et XIab et/ou XIbb, 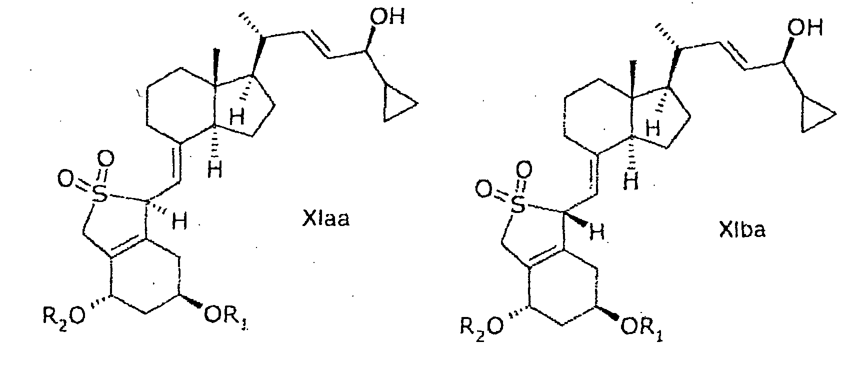 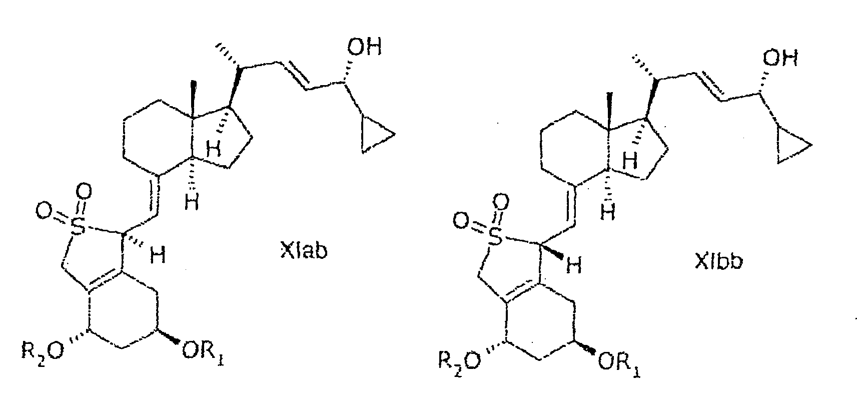 dans lesquelles R₁ et R₂ sont comme définis ci-dessus ;
(iii) à séparer éventuellement les composés de la structure générale XIaa et/ou XIba du mélange réactionnel ;
(iv) à chauffer les composés de la structure générale XIaa et/ou XIba au-delà de 60°C en présence d'une base,
pour fournir un composé de la structure générale IXa, 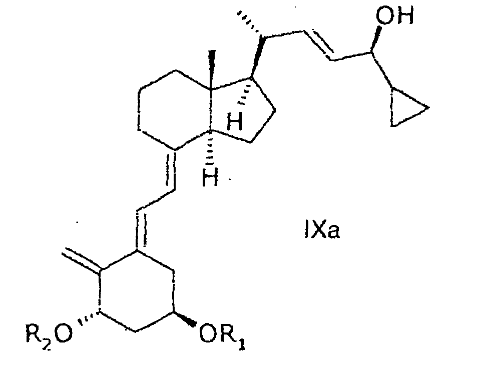 dans laquelle R₁ et R₂ sont comme définis ci-dessus ;
(v) à séparer éventuellement le composé de la structure générale IXa du mélange réactionnel ;
(vi) à réaliser une photoisomérisation du composé de la structure générale IXa en le composé de la structure générale Xa, 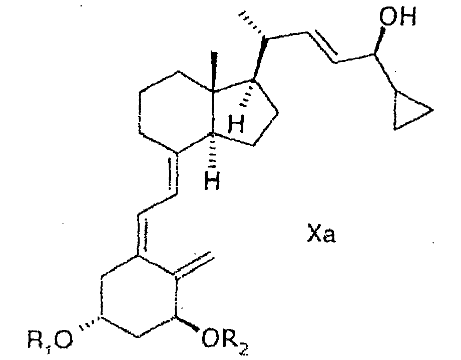 dans laquelle R₁ et R₂ sont comme définis ci-dessus ;
(vii) lorsque R₁ et/ou R₂ ne sont pas un atome d'hydrogène, à éliminer le(les) groupe(s) hydroxy, protecteur(s) R₁ et/ou R₂ du composé de la structure générale Xa pour produire du calcipotriol ; et
(viii) à faire éventuellement cristalliser le calcipotriol à partir d'un mélange d'un solvant organique et d'eau pour fournir du monohydrate de calcipotriol ;
dans lequel les étapes (vi) et (vii) peuvent être réalisées dans l'ordre inverse.

14. Procédé de production de calcipotriol ou de monohydrate de calcipotriol selon la revendication 9, le procédé comprenant les étapes consistant :
(i) à faire réagir un composé de la structure générale XIIIa, 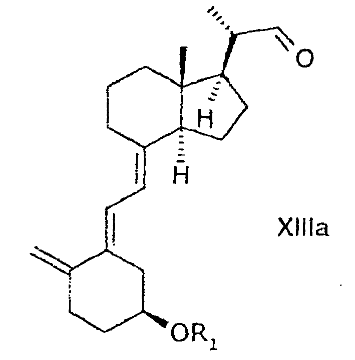 dans laquelle R₁ représente un atome d'hydrogène ou un groupe hydroxy protecteur,
avec un phosphonate de la structure générale VII, 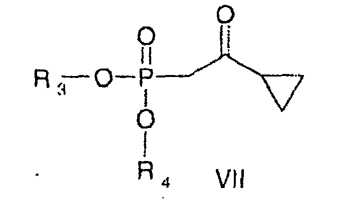 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale XIVa, 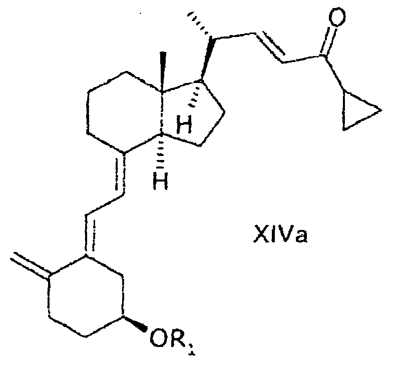 dans laquelle R₁ est comme défini ci-dessus ;
(ii) à hydroxyler le composé de la structure générale XIVa avec un agent d'hydroxylation approprié,
pour fournir un composé de la structure générale Va, 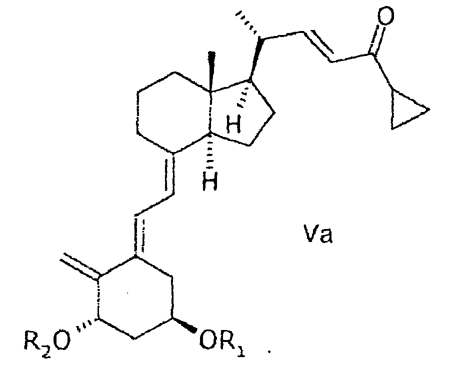 dans laquelle R₁ représente un atome d'hydrogène ou un groupe hydroxy protecteur et R₂ est un atome d'hydrogène ;
(iii) à faire éventuellement réagir le composé de la structure générale Va, dans laquelle R₁ représente un atome d'hydrogène ou un groupe hydroxy protecteur et R₂ est un atome d'hydrogène avec un agent protecteur approprié,
pour fournir un composé de la structure générale Va, dans laquelle R₁ et R₂ sont identiques ou différents et représentent un groupe hydroxy protecteur ;
(iv) à réduire le composé de la structure générale Va avec un agent réducteur approprié pour fournir un composé de la structure générale IXa ou un mélange de composés de la structure générale IXa et IXb, 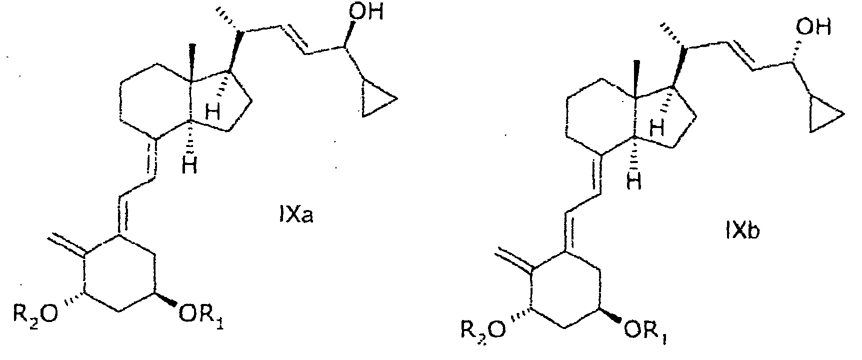 dans lesquelles R₁ et R₂ sont comme définis ci-dessus ;
(v) à séparer éventuellement le composé de la structure générale IXa du mélange de composés de la structure générale IXa et IXb ;
(vi) à réaliser une photoisomérisation du composé de la structure générale IXa en un composé de la structure générale Xa, 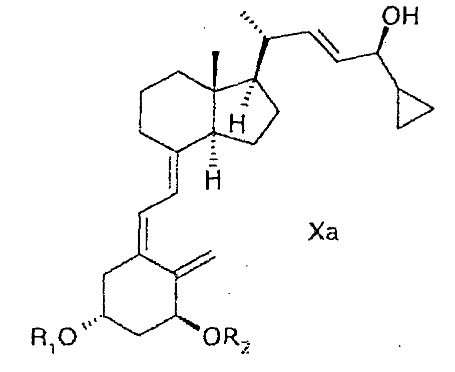 dans laquelle R₁ et R₂ sont comme définis ci-dessus ;
(vii) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(viii) à faire éventuellement cristalliser le calcipotriol à partir d'un mélange d'un solvant organique et d'eau pour fournir du monohydrate de calcipotriol.

15. Procédé de production de calcipotriol ou de monohydrate de calcipotriol selon la revendication 9, le procédé comprenant les étapes consistant :
(i) à faire réagir un composé de la structure générale XIIIb, 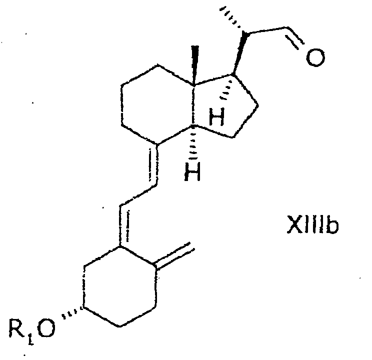 dans laquelle R₁ représente un atome d'hydrogène ou un groupe hydroxy protecteur,
avec un phosphonate de la structure générale VII, 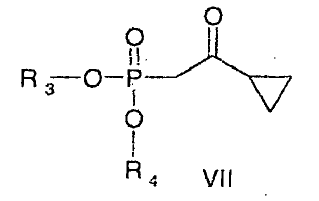 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale XIVb,
dans laquelle R₁ est comme défini ci-dessus ;
(ii) à réaliser une photoisomérisation du composé de la structure générale XIVb en un composé de la structure général XlVa, 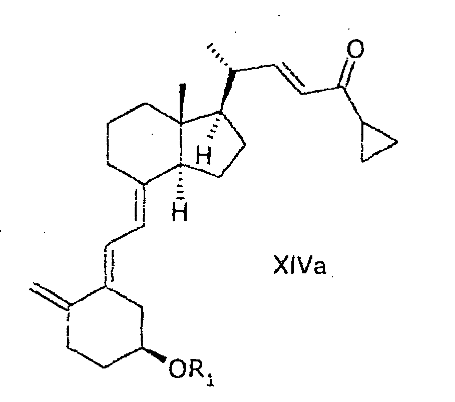 dans laquelle R₁ est comme défini ci-dessus ;
(iii) à hydroxyler le composé de la structure générale XIVa avec un agent d'hydroxylation approprié,
pour fournir un composé de la structure générale Va, 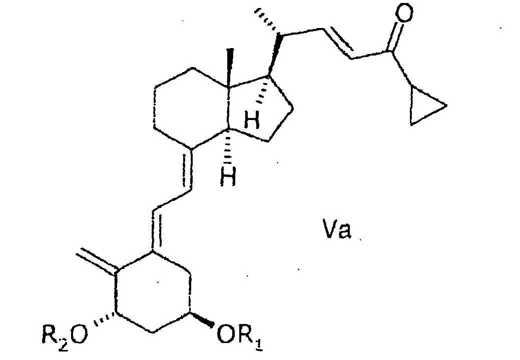 dans laquelle R₁ représente un atome d'hydrogène ou un groupe hydroxy protecteur et R₂ est un atome d'hydrogène ;
(iv) à faire réagir éventuellement le composé de la structure générale Va, dans laquelle R₁ représente un atome d'hydrogène ou un groupe hydroxy protecteur et R₂ est un atome d'hydrogène avec un agent protecteur approprié pour fournir un composé de la structure générale Va, dans laquelle R₁ et R₂ sont identiques ou différents et représentent un groupe hydroxy protecteur ;
(v) à réduire le composé de la structure générale Va avec un agent réducteur approprié, pour fournir un composé de la structure générale IXa ou un mélange de composés de la structure générale IXa et IXb, 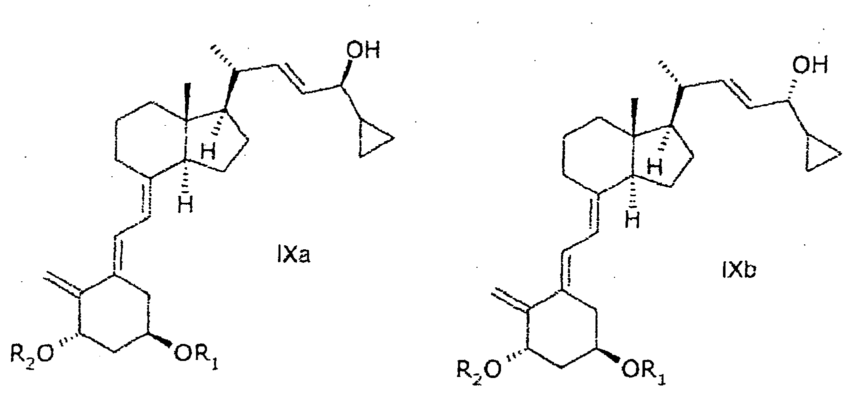 dans lesquelles R₁ et R₂ sont comme définis ci-dessus ;
(vi) à séparer éventuellement le composé de la structure générale IXa du mélange de composés de la structure générale IXa et IXb ;
(vii) à réaliser une photoisomérisation du composé de la structure générale IXa en le composé de la structure générale Xa, 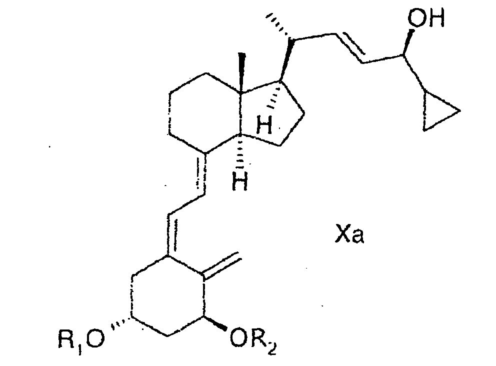 dans laquelle R₁ et R₂ sont comme définis ci-dessus ;
(viii) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(ix) à faire éventuellement cristalliser le calcipotriol à partir d'un mélange d'un solvant organique et d'eau pour fournir du monohydrate de calcipatriol.

16. Procédé de production de calcipotriol ou de monohydrate de calcipotriol selon la revendication 9, le procédé comprenant les étapes consistant :
(i) à faire réagir une composé de la structure générale XVa et/ou XVb, 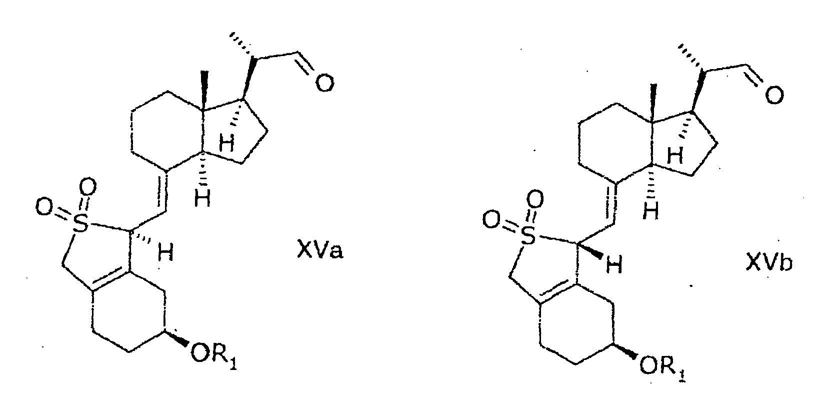 dans lesquelles R₁ représente un atome d'hydrogène ou un groupe hydroxy protecteur,
avec un phosphonate de la structure générale VII, 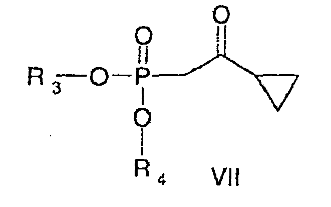 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale XVIa et/ou XVIb, 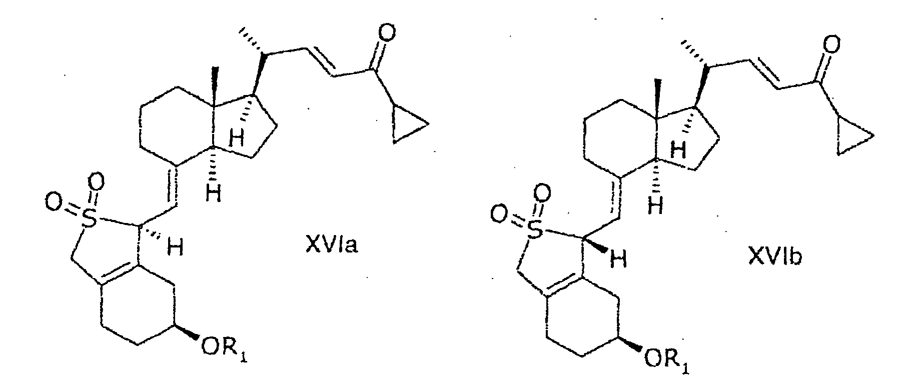 dans lesquelles R₁ est comme défini ci-dessus ;
(ii) à chauffer les composés de la structure générale XVIa et/ou XVIb au-delà de 60°C en présence d'une base,
pour fournir un composé de la structure générale XIVa, 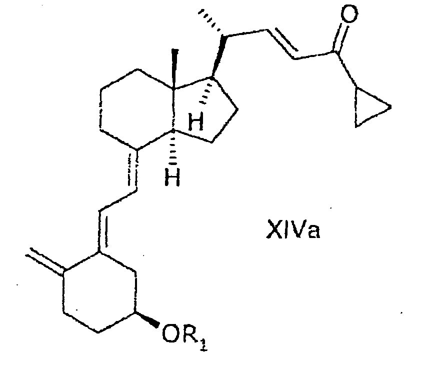 dans laquelle R₁ est comme défini ci-dessus ;
(iii) à hydroxyler le composé de la structure générale XIVa avec un agent d'hydroxylation approprié,
pour fournir un composé de la structure générale Va, 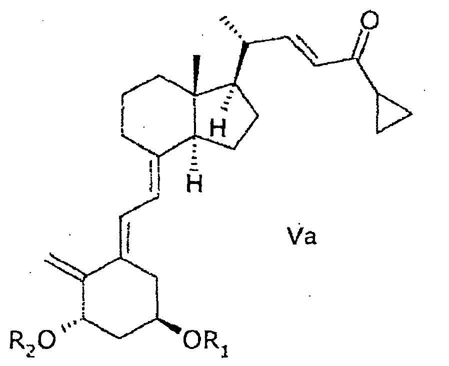 dans laquelle R₁ représente un atome d'hydrogène ou un groupe hydroxy protecteur et R₂ est un atome d'hydrogène ;
(iv) à faire éventuellement réagir le composé de la structure générale Va, dans laquelle R₁ représente un atome d'hydrogène ou un groupe hydroxy protecteur et R₂ est un atome d'hydrogène avec un agent protecteur approprié,
pour fournir un composé de la structure générale Va, dans laquelle R₁ et R₂ sont identiques ou différents et représentent un groupe hydroxy protecteur ;
(v) à réduire le composé de la structure générale Va avec un agent réducteur approprié, pour fournir un composé de la structure générale IXa ou un mélange de composés de la structure générale IXa et IXb, 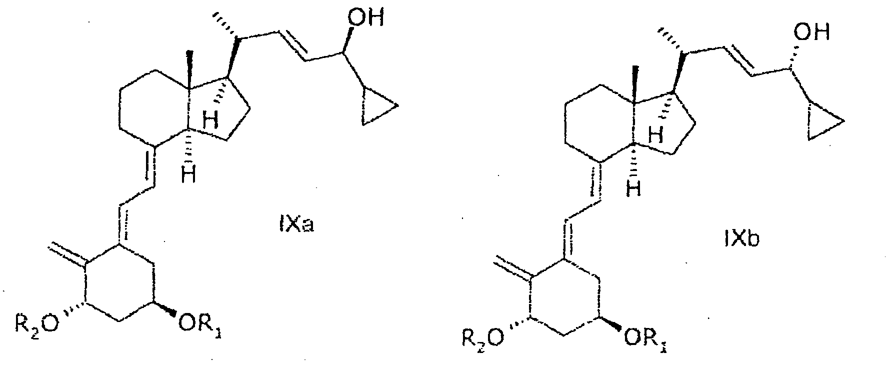 dans lesquelles R₁ et R₄ sont comme définis ci-dessus ;
(vi) à séparer éventuellement le composé de la structure générale IXa du mélange de composés de la structure générale IXa et IXb ;
(vii) à réaliser une photoisomérisation du composé de la structure générale IXa en le composé de la structure générale Xa, 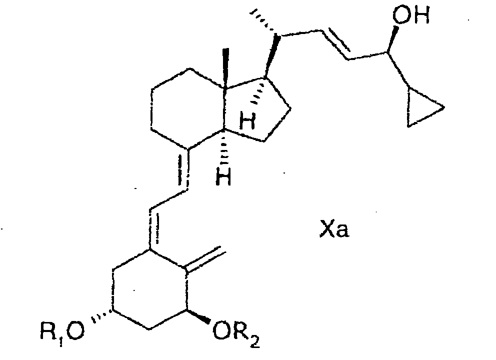 dans laquelle R₁ et R₇ sont comme définis ci-dessus ;
(viii) lorsque R₁ et/ou R₂ ne sont pas un atome d'hydrogène, à éliminer le(les) groupe(s) hydroxy protecteur(s) R₁ et/ou R₂ du composé de la structure générale Xa pour produire du calcipotriol ; et
(ix) à faire éventuellement cristalliser le calcipotriol à partir d'un mélange d'un solvant organique et d'eau pour fournir du monohydrate de calcipotriol.

17. Procédé de production de calcipotriol ou de monohydrate de calcipotriol selon la revendication 9, le procédé comprenant les étapes consistant :
(i) à faire réagir un composé de la structure générale IXX, 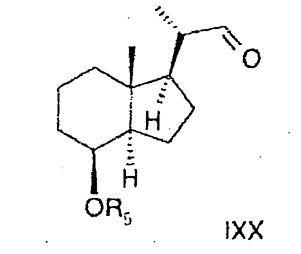 dans laquelle R₅ représente un atome d'hydrogène ou un groupe hydroxy protecteur,
avec un phosphonate de la structure générale VII, 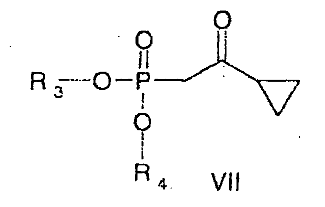 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy,
en présence d'une base,
pour fournir un composé de la structure générale XX, 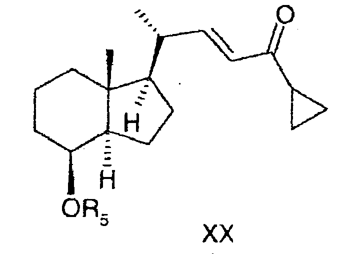 dans laquelle R₅ est comme défini ci-dessus ;
(ii) à réduire le composé de la structure générale XX avec un agent réducteur approprié, pour fournir un composé de la structure générale XXIa ou un mélange de composés de la structure générale XXIa et XXIb, 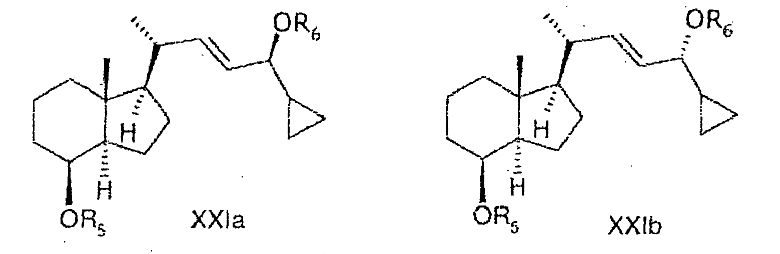 dans lesquelles R₅ est comme défini ci-dessus et R₆ est un atome d'hydrogène ;
(iii) à séparer éventuellement le composé de la structure générale XXIa du mélange de composés de la structure générale XXIa et XXIb ;
(iv) à protéger le groupe hydroxy allylique du composé de la structure générale XXIa avec un agent hydroxy protecteur approprié,
pour fournir un composé de la structure générale XXIa, dans laquelle R₆ est un groupe hydroxy protecteur et R₅ est comme défini ci-dessus ;
(v) lorsque R₅ n'est pas un atome d'hydrogène, à éliminer le groupe hydroxy protecteur R₅ du composé de la structure générale XXIa pour fournir un composé de la structure générale XXIa, dans laquelle R₅ est l'atome d'hydrogène ;
(vi) à oxyder le groupe hydroxy du composé de la structure générale XXIa avec un agent oxydant approprié pour fournir un composé de la structure générale XXII, 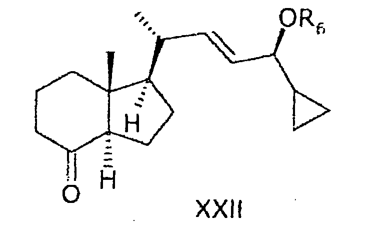 dans laquelle R₆ est comme défini ci-dessus ;
(vii) à réaliser le couplage du composé de la structure générale XXII avec un réactif de Wittig XXIIIa ou un réactif de Wittig Horner XXIIIb, 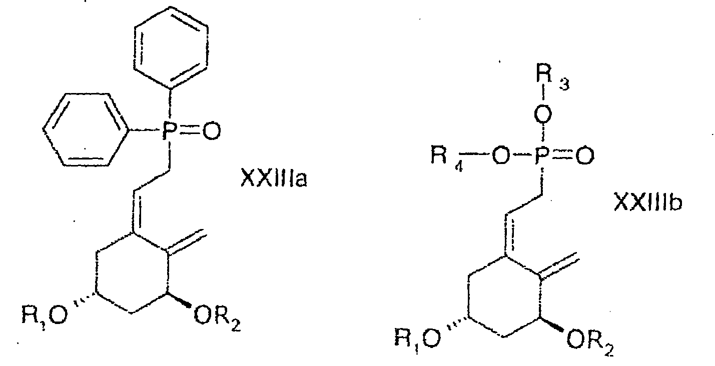 dans lesquelles R₁ et R₂ représentent un atome d'hydrogène ou un groupe hydroxy protecteur, et dans desquelles R₃ et R₄ sont comme définis ci-dessus ;
en présence d'une base,
pour fournir un composé de la structure générale XXIVa, 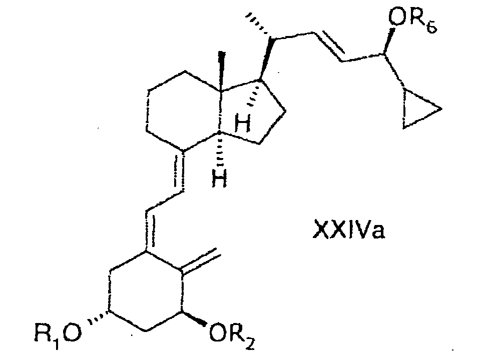 dans laquelle R₁ et R₂ sont identiques ou différents et représentent un atome d'hydrogène ou un groupe hydroxy protecteur, et dans laquelle R₆ est comme défini ci-dessus ;
(viii) lorsque R₆ n'est pas un atome d'hydrogène, à éliminer le groupe hydroxy protecteur R₆ du composé de la structure générale XXIVa ;
(ix) à séparer éventuellement le composé de la structure générale XXIVa ;
(x) lorsque R₁ et R₂ ne sont pas un atome d'hydrogène, à éliminer le(les) groupe(s) hydroxy, protecteur(s) R₁ et/ou R₂ du composé de la structure générale XXIVa pour produire du calcipotriol;
et
(xi) à faire cristalliser éventuellement le calcipotriol à partir d'un mélange d'un solvant organique et d'eau pour fournir du monohydrate de calcipotriol.

18. Procédé selon l'une quelconque des revendications 1-17, dans lequel R₃ et R₄ sont un groupe alkyle en C₁-C₆.

19. Procédé selon l'une quelconque des revendications 1-17, dans lequel R₃ et R₄ sont le groupe méthyle ou éthyle.

20. Procédé selon l'une quelconque des revendications 1-19, dans lequel R₁ et R₂ représentent un atome d'hydrogène ou un groupe alkylsilyle.

21. Procédé selon l'une quelconque des revendications 1-19, dans lequel R₁ et R₂ représentent l'atome d'hydrogène ou le groupe tert-butyldiméthylsilyle.

22. Procédé selon la revendication 8 ou 19, dans lequel R₅ représente le groupe triéthylsilyle et R₃ et R₄ sont le groupe méthyle ou éthyle.

23. Procédé selon l'une quelconque des revendications 1-22, dans lequel la réaction avec le phosphonate de la structure générale VII est réalisée dans des conditions de transfert de phase.

24. Procédé selon l'une quelconque des revendications 1-22, dans lequel la réaction avec le phosphonate de la structure générale VII est réalisée dans des conditions de transfert de phase dans un mélange de toluène ou de xylène et d'eau avec un halogénure de tétraalkylammonium ou un hydrogénosulfate de tétraalkylammonium comme catalyseur de transfert de phase et avec un hydroxyde de métal alcalin et/ou un hydroxyde de tétraalkylammonium comme base.

25. Procédé selon l'une quelconque des revendications 1-24, dans lequel la réaction avec le phosphonate de la structure générale VII est réalisée à une température entre 10°C et 50°C.

26. Composé de la structure générale VII, 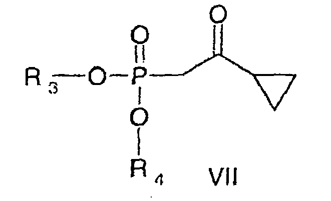 dans laquelle R₃ et R₄ sont identiques ou différents et représentent un groupe alkyle, haloalkyle, hydroxyalkyle, alcényle, alcynyle, aralkyle, aralcényle, aralcynyle ou aryle, chacun étant éventuellement substitué par un ou plusieurs substituants choisis dans le groupe constitué des groupes alkyle, aralkyle, cycloalkyle, cycloalcényle, haloalkyle, hydroxyalkyle, alcényle, alcynyle, aralkyle, aralcényle, aralcynyle, aryle, oxo, alcoxycarbonyle, alkylcarbonyloxy, d'atomes d'halogène, des groupes alcoxy, carboxy, sulfo ou hydroxy, à condition que le composé ne soit pas le (2-cyclopropyl-2-oxoéthyl)phosphonate de diéthyle, dans lequel les termes "alkyle", "alcényle", "alcynyle", "haloalkyle", "hydroxyalkyle", "alcoxy", "alcoxycarbonyle", "alkylcarbonyloxy", "cycloalkyle", "cycloalcényle", "aryle", "arylalkyle", "aralcényle" et "aralcynyle" sont comme définis dans la revendication 1.
